# EUROPEAN PATENT APPLICATION

(11) **EP 3 045 448 A1**
(43) Date of publication of application: **20.07.2016**
(21) Application number: 14843405.3
(22) Date of filing: 12.09.2014
(51) Int. Cl.: C07D 207/16, A61K 31/40, A61K 31/445, A61K 31/453, A61K 31/4535, A61K 31/4545, A61P 1/04, A61P 9/10, A61P 9/14, A61P 11/06, A61P 13/08, A61P 13/12, A61P 17/00, A61P 17/06, A61P 19/02, A61P 25/00, A61P 25/04, A61P 25/08, A61P 25/24, A61P 25/28, A61P 27/02

(54) **NITROGEN-CONTAINING SATURATED HETEROCYCLIC COMPOUND**

(30) Priority: 12.09.2013 JP 2013189577
(71) Applicant: Sumitomo Chemical Company Limited, Tokyo 104-8260 (JP); Sumitomo Dainippon Pharma Co., Ltd., Osaka-shi Osaka 541-8524 (JP)
(72) Inventor: FUSANO, Akira, Suita-shi Osaka 564-0053 (JP); KOBAYASHI, Tomonori, Suita-shi Osaka 564-0053 (JP); SAITO, Yasuhiro, Osaka-shi Osaka 541-0045 (JP); KANAI, Toshio, Osaka-shi Osaka 554-0022 (JP)
(74) Representative: Hoffmann Eitle
(86) International application number: PCT/JP2014/074283
(87) International publication number: WO 2015/037716

(57) **Abstract**

The present invention provides a compound represented by the following formula (I) or its pharmaceutically acceptable salt: 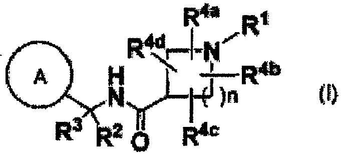 [wherein, R¹ represents optionally substituted C₁₋₄ alkyl, n shows integer of 1 to 4, R² represents optionally substituted C₁₋₄ alkyl or hydrogen atom, R³ represents optionally substituted C₁₋₄ alkyl, R^{4a}, R^{4b}, R^{4c}, and R^{4d}, similarly or differently, represent optionally substituted C₆₋₁₄ aryl, optionally substituted C₁₋₄ alkyl, or hydrogen atom and the like, A represents optionally substituted C₆₋₁₄ aryl or optionally substituted 5 to 11 membered heteroaryl].

## Description

### Technical Field

The present invention relates to a novel nitrogen-containing saturated heterocyclic compound, especially to its novel piperidine derivative, which has a somatostatin receptor subtype 4 (SSTR4)-selective agonistic activity and is useful as an agent for treating and/or preventing medical diseases. The present invention also relates to the novel nitrogen-containing saturated heterocyclic compound, especially to its novel piperidine derivative, which is useful as an agent for treating and/or preventing neurodegenerative diseases such as Alzheimer's disease with which Aβ is involved.

### Background Art

Alzheimer's disease is a neurodegenerative disease characterized by degeneration and deciduation of neurons as well as senile plaque formation and neurofibrillary tangle, and is a type of dementia that causes decline in cognitive function and personality change. Now, measures for treating Alzheimer's disease are limited to symptomatic therapies with medicines in order to improve symptoms by using such as acetylcholinesterase inhibitors. And, no effective methods for treating and/or preventing the pathogenesis of Alzheimer's disease has been found.

Neprilysin has been known as an Aβ (may be called "amyloid β protein", "amyloid β peptide", or "β amyloid") degrading enzyme. Neprilysin can degrade soluble Aβ oligomers that are considered to be a causative agent of deterioration in cognitive functions. In addition, somatostatin has been reported to activate neprilysin described above.

Somatostatin, an endogenous ligand for somatostatin receptors, has an extremely short biological half-life, and is not suitable for use as a medicine. As non-peptide type somatostatin receptor agonists, pyrrolidine derivatives (Patent Literature 1) and thiourea derivatives (Patent Literatures 2 and 3) have been presented. These pyrrolidine derivatives and thiourea derivatives have an affinity for somatostatin receptor subtype 4 (SSTR4). However, the thiourea derivatives have .structures with high hydrophilicity or relatively large molecular weights, and it is considered that they are generally difficult to obtain sufficient oral absorbability or central transferability needed for medicines. The pyrrolidine derivatives are considered to have improved oral absorbability or central transferability compared with the thiourea derivatives, but their higher-order evaluation has not been progressed.

The pyrrolidine derivatives disclosed in Patent Literature 1 have structures shown by the following formula. These compounds necessarily have a substituent (a heterocyclic group or an aryl group) shown as R^{d} on the pyrrolidine ring. 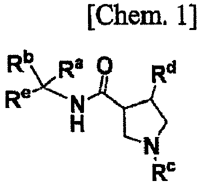 [wherein, R^{a} and R^{b} are, each independently, hydrogen atom or C₁₋₆alkyl, or R^{a} and R^{b} unitedly form C₃₋₆cycloalkyl, R^{c} is hydrogen atom or C₁₋₆alkyl, R^{d} is optionally substituted heterocycle or optionally substituted aryl (proviso that R^{d} is not p-cyanophenyl), R^{e} is optionally substituted heterocycle or optionally substituted aryl (proviso that R^{e} is neither p-methoxyphenyl nor p-chlorophenyl, or R^{e} is pyridyl or pyrimidyl, R^{a} is C₁₋₆alkyl).]

### Citation List

### Patent Literatures

Patent Literature 1: WO 2010/059922
Patent Literature 2: WO 1997/043278
Patent Literature 3: WO 2011/047165

### Summary of Invention

### Problems to be Solved by the Invention

The present invention is made in view of such a situation described above, and an objective of the present invention is to provide novel compounds having a somatostatin receptor subtype 4 (hereinafter, may be abbreviated as "SSTR4")-selective agonistic activity. In more detail, the objective of the present invention is to provide novel compounds being useful as treating agents and/or preventing agents improving symptoms of neurodegenerative diseases such as Alzheimer's disease by activation of neprilysin and acetylcholine releasement enhancing action through the action of the compounds to somatostatin receptor subtype 4 that was found in the present invention.

### Means for Solving the Problems

The present inventors have made extensive investigations, and, as a result, found that the novel compounds represented by the following formula (I) have strong SSTR4-binding action and agonist activity, and have thus completed the present invention. According to the present invention, nitrogen-containing saturated heterocyclic compounds represented by the following formula (I) or their pharmaceutically acceptable salts (hereinafter, may be called "the compound of the present invention", "the compound represented by formula (I)", or "the compound of formula (I)") can be provided:
[Item 1] A compound represented by the following formula (I): 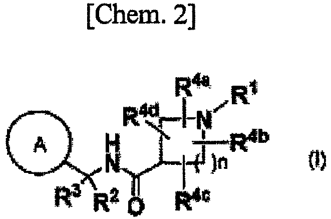
   [wherein, R¹ represents C₁₋₄ alkyl which may be substituted by 1 to 5 fluorine atoms,
   n is an integer of 1 to 4,
   R² represents C₁₋₄ alkyl which may be substituted by the same or different 1 to 5 substituents selected from the group consisting of fluorine atom and hydroxy, or hydrogen atom,
   R³ represents C₁₋₄alkyl which may be substituted by the same or different 1 to 5 substituents selected from the group consisting of fluorine atom and hydroxy,
   provided that n is 1, 3, or 4, R³ and R² may unitedly form a 3-6 membered saturated ring which may include any one of -O-, -NR⁵-, -SO-, or -SO₂-, and the saturated ring may be substituted by 1 to 5 substituents selected from the group consisting of C₁₋₄ alkyl (which may be substituted by 1 to 5 fluorine atoms), C₁₋₄ alkoxy (which may be substituted by 1 to 5 fluorine atoms), halogen, hydroxy, cyano, oxo, -CO₂R⁶ group, and -CONR⁷R⁸, provided that n is 2, R² and R³ do not unitedly form the saturated ring,
   R^{4a}, R^{4b}, R^{4c}, and R^{4d}, similarly or differently, represent C₆₋₁₄ aryl which may be substituted by the same or different 1 to 5 substituents selected from the group consisting of C₁₋₄ alkyl (which may be substituted by 1 to 5 fluorine atoms), C₃₋₆cycloalkyl, halogen, C₁₋₄ alkoxy (which may be substituted by 1 to 5 fluorine atoms), C₁₋₄ alkylthio, hydroxy, and cyano; C₁₋₄ alkyl which may be substituted by the same or different 1 to 5 substituents selected from the group consisting of fluorine atom, hydroxy, and C₁₋₃ alkoxy; C₁₋₃ alkoxy which may be substituted by 1 to 5 fluorine atoms; hydrogen atom; fluorine atom; hydroxy; or -CO₂R⁹, provided that n is 2, and R^{4a}, R^{4b}, R^{4c} and R^{4d} are hydrogen atoms, and provided that n is 1, 3, or 4, and two or more of R^{4a}, R^{4b}, R^{4c}, and R^{4d} are optionally substituted C₁₋₄ alkyl, the two or more optionally substituted C₁₋₄ alkyl may unitedly form a 4-7 membered saturated ring,
   A represents C₆₋₁₄ aryl or a 5-11 membered heteroaryl, the C₆₋₁₄ aryl and the 5-11 membered heteroaryl may be substituted by the same or different 1 to 5 substituents selected from the group consisting of C₁₋₄ alkyl (which may be substituted by 1 to 5 fluorine atoms), C₃₋₆ cycloalkyl, halogen, C₆₋₁₄ aryl, C₆₋₁₄ aryloxy, C₁₋₄ alkoxy (which may be substituted by 1 to 5 fluorine atoms), C₁₋₄ alkylthio, hydroxy, cyano, nitro, -CO₂R¹⁰, -CONR¹¹R¹², -NR¹⁰COR¹¹, -NR¹⁰CONR¹¹R¹², -SOR¹³, -SO₂R¹⁴, -SO₂NR¹¹R¹², and -NR¹¹R¹², and provided that n is 3, R² is hydrogen atom, and R³ is tert-butyl, A is not unsubstituted phenyl,
   R⁵ represents hydrogen atom, C₁₋₄ alkyl, C₃₋₆ cycloalkyl, -CONR¹⁵R¹⁶, -COR¹⁷, or -CO₂R¹⁸,
   R⁶ and R⁹ similarly or differently represent hydrogen atom or C₁₋₄ alkyl,
   R⁷ and R⁸ similarly or differently represent hydrogen atom, C₁₋₄ alkyl, and C₃₋₆cycloalkyl, and provided that R⁷ and R⁸ are C₁₋₄ alkyl, R⁷ and R⁸ may unitedly form a 3-6 membered saturated heterocycle,
   R¹⁰ represents hydrogen atom, C₁₋₄ alkyl, C₃₋₆ cycloalkyl, or C₆₋₁₄ aryl,
   R¹¹ and R¹² similarly or differently represent hydrogen atom, C₁₋₄ alkyl, C₃₋₆ cycloalkyl, or C₆₋₁₄ aryl, and provided that R¹¹ and R¹² are C₁₋₄alkyl, R¹¹ and R¹² may unitedly form a 3-6 membered saturated heterocycle,
   R¹³ represents C₁₋₄ alkyl, C₃₋₆ cycloalkyl, or C₆₋₁₄ aryl,
   R¹⁴ represents hydroxy, C₁₋₄ alkyl, C₃₋₆ cycloalkyl, C₁₋₄ alkoxy, C₆₋₁₄ aryloxy, or C₆₋₁₄ aryl,
   R¹⁵ and R¹⁶ similarly or differently represent hydrogen atom or C₁₋₄ alkyl, and provided that R¹⁵ and R¹⁶ are C₁₋₄ alkyl, R¹⁵ and R¹⁶ may unitedly form a 3-6 membered saturated heterocycle,
   R¹⁷ represents hydrogen atom, C₁₋₄ alkyl, or C₃₋₆ cycloalkyl,
   R¹⁸ represents C₁₋₄ alkyl or C₃₋₆ cycloalkyl],
   or its pharmaceutically acceptable salt.
[Item 1-2]
   A compound represented by the following formula (I): 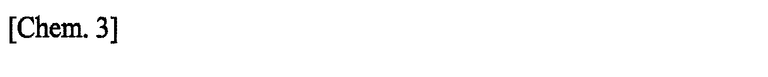 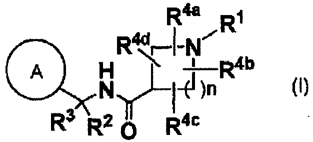 [wherein,
   R¹ represents C₁₋₄ alkyl which may be substituted by 1 to 5 fluorine atoms,
   n is an integer of 1 to 4,
   R² represents C₁₋₄ alkyl which may be substituted by the same or different 1 to 5 substituents selected from the group consisting of fluorine atom and hydroxy, or hydrogen atom,
   R³ represents C₁₋₄ alkyl which may be substituted by the same or different 1 to 5 substituents selected from the group consisting of fluorine atom and hydroxy,
   provided that n is 1, 3, or 4, R³ and R² may unitedly form a 3-6 membered saturated ring which may include any one of -O-, -NR⁵-, -SO-, or -SO₂-, and the ring may be substituted by 1 to 5 substituents selected from the group consisting of C₁₋₄ alkyl (which may be substituted by 1 to 5 fluorine atoms), C₁₋₄ alkoxy (which may be substituted by 1 to 5 fluorine atoms), halogen, hydroxy, cyano, oxo, -CO₂R⁶, and -CONR⁷R⁸, provided that n is 2, R² and R³ do not unitedly form the saturated ring,
   R^{4a}, R^{4b},R^{4c}, and R^{4d}, similarly or differently, represent C₆₋₁₄ aryl which may be substituted by the same or different 1 to 5 substituents selected from the group consisting of C₁₋₄ alkyl (which may be substituted by 1 to 5 fluorine atoms), C₃₋₆cycloalkyl, halogen, C₁₋₄ alkoxy (which may be substituted by 1 to 5 fluorine atoms), C₁₋₄ alkylthio, hydroxy, and cyano; C₁₋₄ alkyl, which may be substituted by the same or different 1 to 5 substituents selected from the group consisting of fluorine atoms, hydroxy, and C₁₋₃ alkoxy; C₁₋₃ alkoxy which may be substituted by 1 to 5 fluorine atoms; hydrogen atoms; fluorine atoms; hydroxy; or -CO₂R⁹, provided that n is 2, and R^{4a}, R^{4b}, R^{4c} and R^{4d} are hydrogen atoms, and provided that n is 1, 3, or 4, and two or more of any of R^{4a}, R^{4b}, R^{4c}, and R^{4d} are optionally substituted C₁₋₄ alkyl, the two or more optionally substituted C₁₋₄ alkyl may unitedly form a 4-7 membered saturated ring,
   A represents C₆₋₁₄ aryl or 5-11 membered heteroaryl, the C₆₋₁₄ aryl and the 5-11 membered heteroaryl may be substituted by the same or different 1 to 5 substituents selected from the group consisting of C₁₋₄ alkyl which may be substituted by 1 to 5 fluorine atoms, C₃₋₆ cycloalkyl, halogen, C₆₋₁₄ aryl, C₆₋₁₄ aryloxy, C₁₋₄ alkoxy which may be substituted by 1 to 5 fluorine atoms, C₁₋₄ alkylthio, hydroxy, cyano, nitro, -CO₂R¹⁰, -CONR¹¹R¹², -NR¹⁰COR¹¹, NR¹⁰CONR¹¹R¹², -SOR¹³, -SO₂R¹⁴, -SO₂NR¹¹R¹², and -NR¹¹R¹², and provided that n is 3, R² is a hydrogen atom, and R³ is tert-butyl, A is not unsubstituted phenyl,
   R⁵ represents a hydrogen atom, C₁₋₄ alkyl, C₃₋₆ cycloalkyl, -CONR¹⁵R¹⁶, -COR¹⁷, or -CO₂R¹⁸,
   R⁶ and R⁹ similarly or differently represent hydrogen atom or C₁₋₄ alkyl,
   R⁷ and R⁸ similarly or differently represent hydrogen atom, C₁₋₄ alkyl, and C₃₋₆ cycloalkyl, and provided that R⁷ and R⁸ are C₁₋₄ alkyl, R⁷ and R⁸ may unitedly form a 3-6 membered saturated heterocycle,
   R¹⁰ represents hydrogen atom, C₁₋₄ alkyl, C₃₋₆ cycloalkyl, or C₆₋₁₄ aryl,
   R¹¹ and R¹² similarly or differently represent hydrogen atom, C₁₋₄ alkyl, C₃₋₆ cycloalkyl, or C₆₋₁₄ aryl, and provided that R¹¹ and R¹² are C₁₋₄ alkyl, R¹¹ and R¹² may unitedly form a 3-6 membered saturated heterocycle,
   R¹³ represents C₁₋₄alkyl, C₃₋₆ cycloalkyl, or C₆₋₁₄ aryl,
   R¹⁴ represents hydroxy, C₁₋₄ alkyl, C₃₋₆cycloalkyl, C₁₋₄ alkoxy, C₆₋₁₄ aryloxy, or C₆-₁₄ aryl,
   R¹⁵ and R¹⁶ similarly or differently represent hydrogen atoms or C₁₋₄ alkyl, and provided that R¹⁵ and R¹⁶ are C₁₋₄ alkyl, R¹⁵ and R¹⁶ may unitedly form a 3-6 membered saturated heterocycle,
   R¹⁷ represents hydrogen atom, C₁₋₄alkyl, or C₃₋₆ cycloalkyl, and
   R¹⁸ represents C₁₋₄ alkyl or C₃₋₆ cycloalkyl.]
   or its pharmaceutically acceptable salt;
   proviso that the following compounds are excluded:
   N-1-(4-methanesulfonylphenyl)propyl-1-methylpiperidine-3-car boxamide;
   N-1-(4-pyrrolidine-1-ylphenyl}ethyl-1-methylpiperidine-3-carbo xamide;
   N-1-(2,4-dimethylphenyl)ethyl-1-methylpiperidine-3-carboxami de; and
   N-1-phenylethyl-1-propyl-3-(4-ethylphenyl)piperidine-5-carbox amide.
[Item 2]
   The compound or its pharmaceutically acceptable salt according to Item 1 or Item 1-2, wherein n is 1 or 3.
[Item 3]
   The compound or its pharmaceutically acceptable salt according to Item 1 or Item 1-2, wherein n is 3.
[Item 4]
   The compound or its pharmaceutically acceptable salt according to any one of Items 1 to 3 and Item 1-2, wherein R² and R³ are the same or different C₁₋₄alkyl which may be substituted by the same or different 1-5 substituents selected from the group consisting of fluorine atom or hydroxy, and R² and R³ may unitedly form a 3-6 membered saturated ring which may include any one of -O- or -SO₂-.
[Item 4-2]
   The compound or its pharmaceutically acceptable salt according to Item 4, wherein R² and R³ meet the following condition (1) or (2):
   (1) R² and R³ are C₁₋₃ alkyl;
   (2) R² and R³ unitedly form a 3-6 membered saturated ring which may be substituted by one or two fluorine atoms and may include one -O-.
[Item 4-3]
   The compound or its pharmaceutically acceptable salt according to Item 4, wherein R² and R³ are methyl.
[Item 4-4]
   The compound or its pharmaceutically acceptable salt according to any one of Items 1 to 3 and Item 1-2, wherein R² represents hydrogen atom, and R³ represents trifluoromethyl.
[Item 4-5]
   The compound or its pharmaceutically acceptable salt according to Item 4, wherein R² and R³ unitedly form a 3-6 membered saturated ring, or a tetrahydropyran ring represented by the following formula. 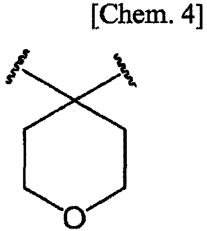
[Item 5]
   The compound or its pharmaceutically acceptable salt according to any one of Items 1 to 4, Item 1-2, Items 4-2 to 4-5, wherein A is C₆₋₁₄ aryl, and the C₆₋₁₄ aryl may be substituted by the same or different 1 to 5 substituents selected from the group consisting of C₁₋₄ alkyl (which may be substituted by 1 to 5 fluorine atoms), C₃₋₆ cycloalkyl, fluorine atom, chlorine atom, bromine atom, C₁₋₄alkoxy (which may be substituted by 1 to 5 fluorine atoms), C₁₋₄ alkylthio, cyano, -CO₂R¹⁰, -CONR¹¹R¹², -SO₂NR¹¹R¹², and -NR¹¹R¹².
[Item 5-2]
   The compound or its pharmaceutically acceptable salt according to Item 5, wherein A is phenyl or naphthyl, and the phenyl or the naphthyl may be substituted by the same or different 1 to 2 substituents selected from the group consisting of C₁₋₄ alkyl (which may be substituted by 1 to 5 fluorine atoms), fluorine atom, chlorine atom, bromine atom, C₁₋₄ alkoxyl (which may be substituted by 1 to 5 fluorine atoms) and C₁₋₄ alkylthio.
[Item 5-3]
   The compound or its pharmaceutically acceptable salt according to Item 5, wherein A is phenyl or 1-naphthyl, and the phenyl or the 1-naphthyl may be substituted by the same or different 1 to 2 substituents selected from the group consisting of methyl, ethyl, methoxy, trifluoromethoxy, methylthio, fluorine atom, chlorine atom, and bromine atom.
[Item 5-4]
   The compound or its pharmaceutically acceptable salt according to Item 5-3, wherein A is 1-naphthyl.
[Item 5-5]
   The compound or its pharmaceutically acceptable salt according to Item 5-3, wherein A is phenyl, and the phenyl is substituted by at least a chlorine atom or a bromine atom.
[Item 6-0]
   The compound or its pharmaceutically acceptable salt according to any one of Items 1 to 5, Item 1-2, Items 4-2 to 4-5, and Items 5-2 to 5-5, wherein R¹ is C₁₋₄ alkyl.
[Item 6]
   The compound or its pharmaceutically acceptable salt according to any one of Items 1 to 5, Item 1-2, Items 4-2 to 4-5, and Items 5-2 to 5-5, wherein R¹ is C₁₋₃ alkyl.
[Item 7]
   The compound or its pharmaceutically acceptable salt according to any one of Items 1 to 6, Item 1-2, Items 4-2 to 4-5, Items 5-2 to 5-5, and Item 6-0, wherein R^{4a} and R^{4b} are, similarly or differently, C₆₋₁₄ aryl which may be substituted by the same or different 1 to 5 substituents selected from the group consisting of C₁₋₄ alkyl (which may be substituted by 1 to 5 fluorine atoms), C₃₋₆cycloalkyl, halogen atom, C₁₋₄ alkoxy (which may be substituted by 1 to 5 fluorine atoms), C₁₋₄ alkylthio, hydroxy, and cyano; C₁₋₄ alkyl which may be substituted by the same or different 1 to 5 substituents selected from the group consisting of fluorine atom, hydroxy, and C₁₋₃ alkoxy; C₁₋₃ alkoxy which may be substituted by 1 to 5 fluorine atoms; hydrogen atom; fluorine atom; or -CO₂R⁹, provided that R^{4a} and R^{4b} are optionally substituted C₁₋₄ alkyl, the optionally substituted C₁₋₄ alkyl may unitedly form a 4-7 membered saturated ring, and R^{4c} and R^{4d} are hydrogen atoms.
[Item 8]
   The compound or its pharmaceutically acceptable salt according to any one of Items 1 to 7, Item 1-2, Items 5-2 to 5-5, and Item 6-0, wherein R¹ is C₁₋₃ alkyl, and R² and R³ are, similarly or differently, C₁₋₄ alkyl.
[Item 9]
   The compound or its pharmaceutically acceptable salt according to any one of Items 1 to 8, Item 1-2, Items 4-2 to 4-5, and Item 6-0, wherein A is C₆₋₁₄ aryl, and the C₆₋₁₄ aryl may be substituted by the same or different 1 to 3 substituents selected from the group consisting of C₁₋₄ alkyl (which may be substituted by 1 to 5 fluorine atoms), C₃₋₆ cycloalkyl, fluorine atom, chlorine atom, bromine atom, C₁₋₄ alkoxy (which may be substituted by 1 to 5 fluorine atoms), C₁₋₄ alkylthio, and cyano.
[Item 10]
   The compound or its pharmaceutically acceptable salt according to any one of Items 3 to 9, Items 4-2 to 4-5, Items 5-2 to 5-5, and Item 6-0, wherein, when n is 3, R^{4a} and R^{4b} are, similarly or differently, substituents at the 2, 5, or 6-positions of the piperidine ring, and are C₁₋₄ alkyl which may be substituted by the same or different 1 to 5 substituents selected from the group consisting of hydroxy and C₁₋₃ alkoxy or hydrogen atom, provided that R^{4a} and R^{4b} are optionally substituted C₁₋₄ alkyl, the C₁₋₄ alkyl may unitedly form a 4-7 membered saturated ring, and R^{4c} and R^{4d} are hydrogen atoms,
   provided that R¹ is at the 1-position and the amido is at the 3-position of the piperidine ring.
[Item 11]
   The compound or its pharmaceutically acceptable salt according to any one of Items 3 to 9, Items 4-2 to 4-5, Items 5-2 to 5-5, and Item 6-0, wherein, when n is 3, R^{4a} is the substituent at the 4-position of the piperidine ring and is C₆₋₁₄ aryl which may be substituted by the same or different 1 to 5 substituents selected from the group consisting of C₁₋₄ alkyl (which may be substituted by 1 to 5 fluorine atoms), C₃₋₆ cycloalkyl, halogen atom, C₁₋₄ alkoxy (which may be substituted by 1 to 5 fluorine atoms), C₁₋₄alkylthio, hydroxy, and cyano; or hydrogen atom, and R^{4b}, R^{4c} and R^{4d} are hydrogen atoms, provided that R¹ is at the 1-position and the amido is the 3-position of the piperidine ring.
[Item 11-2]
   The compound or its pharmaceutically acceptable salt according to Item 11, wherein R^{4a} is the substituent at the 4-position of the piperidine ring and is phenyl which may be substituted by the same or different 1 to 3 substituents selected from the group consisting of C₁₋₃ alkyl (which may be substituted by 1 to 5 fluorine atoms), halogen atom, C₁₋₃ alkoxy (which may be substituted by 1 to 5 fluorine atoms), C₁₋₃ alkylthio, and hydroxy, and R^{4b}, R^{4c}, and R^{4d} are hydrogen atoms.
[Item 12]
   The compound or its pharmaceutically acceptable salt according to any one of Items 3 to 9, Items 4-2 to 4-5, Items 5-2 to 5-5, and Item 6-0, wherein, when n is 3, R^{4a} is the substituent at the 2, or 6-position of the piperidine ring and is C₁₋₄ alkyl which may be substituted by the same or different 1 to 5 substituents selected from the group consisting of fluorine atom, hydroxy, and C₁₋₃ alkoxy; or hydrogen atom, and R^{4b}, R^{4c}, and R^{4d} are hydrogen atoms, provided that R¹ is at the 1-position and the amido is the 3-position of the piperidine ring.
[Item 12-2]
   The compound or its pharmaceutically acceptable salt according to Item 12, wherein R^{4a} is the substituent at the 2-position of the piperidine ring and is C₁₋₄ alkyl, and R^{4b}, R^{4c}, and R^{4d} are hydrogen atoms.
[Item 12-3]
   The compound or its pharmaceutically acceptable salt according to Item 12, wherein R^{4a} is the substituent at the 6-position of the piperidine ring and is C₁₋₄alkyl which may be substituted by hydroxy or C₁₋₃ alkoxy, and R^{4b}, R^{4c}, and R^{4d} are hydrogen atoms.
[Item 12-4]
   The compound or its pharmaceutically acceptable salt according to Item 12, wherein R^{4a} is the substituent at the 6-position of the piperidine ring and is C₁₋₃ alkyl which may be substituted by hydroxy, and R^{4b}, R^{4c}, and R^{4d} are hydrogen atoms.
[Item 12-5]
   The compound or its pharmaceutically acceptable salt according to any one of Items 3 to 9, Items 4-2 to 4-5, Items 5-2 to 5-5, and Item 6-0, wherein, when n is 3, both R^{4a} and R^{4b} are the substituents at the 6-position of the piperidine ring and are methyl, and R^{4c} and R^{4d} are hydrogen atoms, provided that R¹ is at the 1-position and the amido is the 3-position of the piperidine ring.
[Item 13]
   The compound or its pharmaceutically acceptable salt according to any one of Items 3 to 9, Items 4-2 to 4-5, Items 5-2 to 5-5, and Item 6-0, wherein, when n is 3, R^{4a} is the substituent at the 5-position of the piperidine ring and is C₁₋₄ alkyl which may be substituted by the same or different 1 to 5 substituents selected from the group consisting of fluorine atom, hydroxy, and C₁₋₃ alkoxy; C₁₋₃ alkoxy which may be substituted by 1 to 5 fluorine atoms; a fluorine atom; or a hydrogen atom, and R^{4b}, R^{4c}, and R^{4d} are hydrogen atoms, provided that R¹ is at the 1-position and the amido is the 3-position of the piperidine ring.
[Item 13-2]
   The compound or its pharmaceutically acceptable salt according to any one of Items 3 to 9, Items 4-2 to 4-5, Items 5-2 to 5-5, and Item 6-0, wherein, when n is 3, R^{4a} is the substituent at the 5-position of the piperidine ring and is C₁₋₃ alkyl, C₁₋₃ alkoxy, fluorine atom, or hydroxy, and R^{4b}, R^{4c}, and R^{4d} are hydrogen atoms, provided that R¹ is at the 1-position and the amido is the 3-position of the piperidine ring.
[Item 13-3]
   The compound or its pharmaceutically acceptable salt according to Item 13-2, wherein R^{4a} is the substituent at the 5-position of the piperidine ring and is methyl, methoxy, fluorine atom, or hydroxy, and R^{4b}, **R**^{4c}, and R^{4d} are hydrogen atoms.
[Item 13-4]
   The compound or its pharmaceutically acceptable salt according to any one of Items 3 to 9, Items 4-2 to 4-5, Items 5-2 to 5-5, and Item 6-0, wherein, when n is 3, both R^{4a} and R^{4b} are the substituents at the 5-position of the piperidine ring and are fluorine atom or methyl, and R^{4c} and R^{4d} are hydrogen atoms, provided that R¹ is at the 1-position and the amido is the 3-position of the piperidine ring.
[Item 14]
   The compound or its pharmaceutically acceptable salt according to any one of Items 1 to 13, Item 1-2, Items 4-2 to 4-5, Items 5-2 to 5-5, and Item 6-0, wherein R^{4a}, R^{4b}, R^{4c}, and R^{4d} are hydrogen atoms.
[Item 15]
   The compound or its pharmaceutically acceptable salt according to Item 1, wherein the compound is selected from the following compounds:
   (3S)-1-methyl-N-[2-(naphthalene-1-yl)propane-2-yl]pyperidine-3-carboxamide (Example 2);
   N-[1-(2,3-dimethylphenyl)cyclopropyl]-1-methylpiperidine-3-ca rboxamide (Example 5);
   N-[4-(3,5-dichlorophenyl)tetrahydro-2H-pyran-4-yl]-1-methylpi peridine-3-carboxamide (Example 9);
   (3S)-N-[2-(2,3-dichlorophenyl)propane-2-yl]-1-methylpiperidin e-3-carboxamide (Example 19);
   (3S)-N-[2-(2,4-dichlorophenyl)propane-2-yl]-1-methylpiperidin e-3-carboxamide (Example 20);
   (3S)-N-[2-(2,5-dichlorophenyl)propane-2-yl]-1-methylpiperidin e-3-carboxamide (Example 21);
   (3S)-N-[2-(2,3-dimethylphenyl)propane-2-yl]-1-methylpiperidin e-3-carboxamide (Example 23);
   (3S)-N-[2-(2-chloro-3-methylphenyl)propane-2-yl]-1-methylpip eridine-3-carboxamide (Example 26);
   (3S)-N-[2-(3-chloro-2-methylphenyl)propane-2-yl]-1-methylpip eridine-3-carboxamide (Example 27);
   (3S)-N-[2-(3-chloro-5-methylphenyl)propane-2-yl]-1-methylpip eridine-3-carboxamide (Example 29);
   (3S)-N-[2-(4-chloro-2-methoxylphenyl)propane-2-yl]-1-methylp iperidine-3-carboxamide (Example 30);
   (3S)-N-[2-(4-chloro-2-ethylphenyl)propane-2-yl]-1-methylpiper idine-3-carboxamide (Example 31);
   (3S)-N-[2-(2,4-diethylphenyl)propane-2-yl]-1-methylpiperidine-3-carboxamide (Example 32);
   (3S)-N-[2-(2,4-dimethylphenyl)propane-2-yl]-1-methylpiperidin e-3-carboxamide (Example 35);
   (3S)-N-[2-(2-chloro-4-methylphenyl)propane-2-yl]-1-methylpip eridine-3-carboxamide (Example 37);
   (3S)-N-[2-(2-chloro-5-methylphenyl)propane-2-yl]-1-methylpip eridine-3-carboxamide (Example 38);
   (3S)-N-[2-(2-chlorophenyl)propane-2-yl]-1-methylpiperidine-3-carboxamide (Example 40);
   (3S)-N-[2-(2-methylphenyl)propane-2-yl]-1-methylpiperidine-3-carboxamide (Example 43);
   (3S)-N-[2-(3-chloro-4-methylphenyl)propane-2-yl]-1-methylpip eridine-3-carboxamide (Example 47);
   N-[2-(4-chloro-2-methylphenyl)propane-2-yl]-1-methylpiperidi ne-3-carboxamide (Example 12);
   (3S)-N-[2-(5-chloro-2-methoxylphenyl)propane-2-yl]-1-methylp iperidine-3-carboxamide (Example 51);
   (3S)-N-[2-(5-fluoro-2-methoxylphenyl)propane-2-yl]-1-methylp iperidine-3-carboxamide (Example 54);
   (3S)-N-[2-(2,6-dichlorophenyl)propane-2-yl]-1-methylpiperidin e-3-carboxamide (Example 55);
   (3S)-N-{2-[2-(trifluoromethoxy)phenyl] propane-2-yl}-1-methylpiperidine-3-carboxamide (Example 60);
   (3S)-N-{2-[2-(methylsulfanyl) phenyl]propane-2-yl}-1-methylpiperidine-3-carboxamide (Example 61);
   (3S)-N-[2-(2-bromophenyl)propane-2-yl]-1-methylpiperidine-3-carboxamide (Example 67);
   (3S)-N-[2-(2-chloro-3-fluorophenyl)propane-2-yl]-1-methylpipe ridine-3-carboxamide (Example 71);
   (3S)-N-[2-(2-chloro-4-fluorohenyl)propane-2-yl]-1-methylpiperi dine-3-carboxamide (Example 72);
   (3S)-N-[2-(2-chloro-5-fluorohenyl)propane-2-yl]-1-methylpiperi dine-3-carboxamide (Example 73);
   (3S)-N-[2-(2-ethylphenyl)propane-2-yl]-1-methylpiperidine-3-c arboxamide (Example 76);
   (3S)-N-[2-(2-chlorophenyl)propane-2-yl]-1-ethylpiperidine-3-ca rboxamide (Example 110); or
   (3S)-N-[2-(naphthalene-1-yl)propane-2-yl]-1-(propane-2-yl)pyp eridine-3-carboxamide (Example 115);
   (3S)-N-[2-(5-chloro-2-methylphenyl)propane-2-yl]-1-methylpip eridine-3-carboxamide (Example 50).
[Item 16]
   A pharmaceutical composition containing the compound or its pharmaceutically acceptable salt according to any one of Items 1 to 15, Item 1-2, Items 4-2 to 4-5, Items 5-2 to 5-5, Item 6-0, Item 11-2, Items 12-2 to 12-5 and Items 13-2 to 13-4.
[Item 17]
   An agent for activating somatostatin receptor subtype 4 containing the compound or its pharmaceutically acceptable salt according to any one of Items 1 to 15, Item 1-2, Items 4-2 to 4-5, Items 5-2 to 5-5, Item 6-0, Item 11-2, Items 12-2 to 12-5 and Items 13-2 to 13-4.
[Item 18]
   An agent for treating or preventing a disease that can be treated or prevented by activating somatostatin receptor subtype 4, containing the compound or its pharmaceutically acceptable salt according to any one of Items 1 to 15, Item 1-2, Items 4-2 to 4-5, Items 5-2 to 5-5, Item 6-0, Item 11-2, Items 12-2 to 12-5 and Items 13-2 to 13-4, as an active ingredient.
[Item 19]
   The agent according to Item 18, wherein the disease is epilepsy, depression, behavior disorder, memory impairment, learning disabilities, attention deficit disorder, pain, neurodegenerative diseases, or neurogenic bladder.
[Item 20]
   The agent according to Item 18, wherein the disease is hyperproliferative disorder, acromegaly, melanoma, breast cancer, prostatic adenoma, prostate cancer, lung cancer, intestinal and colorectal cancer, or skin cancer.
[Item 21]
   The agent according to Item 18, wherein the disease is arthritis, rheumatoid arthritis, or rheumatoid spondylitis.
[Item 22]
   The agent according to Item 18, wherein the disease is psoriasis, atopic dermatitis, or asthma.
[Item 23]
   The agent according to Item 18, wherein the disease is Graves' disease, inflammatory bowel disease, nephropathy, diabetic angiopathy, ischemic disease, or benign prostatic hypertrophy.
[Item 24]
   The agent according to Item 18, wherein the disease is age-related macular degeneration, glaucoma, or diabetic retinopathy.
[Item 25]
   Use of the compound or its pharmaceutically acceptable salt according to any one of Items 1 to 15, Item 1-2, Items 4-2 to 4-5, Items 5-2 to 5-5, Item 6-0, Item 11-2, Items 12-2 to 12-5 and Items 13-2 to 13-4 for treating or preventing a disease that can be treated or prevented by activating somatostatin receptor subtype 4.
[Item 26]
   Use of the compound or its pharmaceutically acceptable salt according to any one of Items 1 to 15, Items 4-2 to 4-5, Item 1-2, Items 5-2 to 5-5, Item 6-0, Item 11-2, Items 12-2 to 12-5 and Items 13-2 to 13-4, in preparation of an agent for treating or preventing a disease that can be treated or prevented by activating somatostatin receptor subtype 4.
[Item 27]
   A method for treating or preventing a disease that can be treated or prevented by activating somatostatin receptor subtype 4, including administering an effective amount of the compound or its pharmaceutically acceptable salt according to any one of Items 1 to 15, Item 1-2, Items 4-2 to 4-5, Items 5-2 to 5-5, Item 6-0, Item 11-2, Items 12-2 to 12-5 and Items 13-2 to 13-4 to a patient
[Item 28]
   The compound or its pharmaceutically acceptable salt according to any one of Items 1 to 15, Item 1-2, Items 4-2 to 4-5, Items 5-2 to 5-5, Item 6-0, Item 11-2, Items 12-2 to 12-5 and Items 13-2 to 13-4 for use in treating or preventing a disease that can be treated or prevented by activating somatostatin receptor subtype 4.
[Item 29]
   A method of activating somatostatin receptor subtype 4, including administering an effective amount of the compound or its pharmaceutically acceptable salt according to any one of Items 1 to 15, Item 1-2, Items 4-2 to 4-5, Items 5-2 to 5-5, Item 6-0, Item 11-2, Items 12-2 to 12-5 and Items 13-2 to 13-4.
[Item 30]
   The compound or its pharmaceutically acceptable salt according to any one of Items 1 to 15, Item 1-2, Items 4-2 to 4-5, Items 5-2 to 5-5, Item 6-0, Item 11-2, Items 12-2 to 12-5 and Items 13-2 to 13-4 for use in activating somatostatin receptor subtype 4.
[Item 31]
   An agent for treating or preventing epilepsy, depression, behavior disorder, memory disorder, learning disorder, attention deficit disorder, pain, neurodegenerative disease, or neurogenic bladder, containing the compound or its pharmaceutically acceptable salt according to any one of Items 1 to 15, Item 1-2, Items 4-2 to 4-5, and Items 5-2 to 5-5, Item 6-0, Item 11-2, Items 12-2 to 12-5 and Items 13-2 to 13-4, as an active ingredient.
[Item 32]
   An agent for treating or preventing hyperproliferative disorder, acromegaly, melanoma, breast cancer, prostatic adenoma, lung cancer, intestinal cancer, or skin cancer, containing the compound or its pharmaceutically acceptable salt according to any one of Items 1 to 15, Item 1-2, Items 4-2 to 4-5, and Items 5-2 to 5-5, Item 6-0, Item 11-2, Items 12-2 to 12-5 and Items 13-2 to 13-4, as an active ingredient

### Effects of the Invention

According to the present invention, providing a novel compound having a somatostatin receptor subtype 4-selective agonistic activity becomes possible.

The compound of the present invention is useful as an agent for treating and/or preventing diseases caused by Aβ such as Alzheimer's disease, cognitive impairment, memory disorder, learning disorder, mild cognitive impairment, senile dementia, amyloidosis, and cerebrovascular angiopathy, pains, cancers, depression, and the like. Embodiments for Carrying Out the Invention

The compound of formula (I) may have one or a plurality of asymmetric carbon atoms, and may generate geometric isomerism or axial chirality. The compound of formula (I) accordingly may exist as several types of stereoisomers. In the present invention, these stereoisomers, their mixtures, and their racemic modifications are included into the compound of the present invention represented by formula (I). Deuterium converters of the compound represented by the formula (I) in which any one or a plurality of ¹H are converted into ²H (D) are also included into the compound represented by formula (I).

In the present invention, a hydrate, solvates such as ethanol solvate of the compound represented by the formula (I) or its pharmaceutically acceptable salt are also included into the compound represented by the formula (I).

Next, terms in the present description will be explained as follows.

The "alkyl" means a straight chain- or branched chain-saturated hydrocarbon group. For example, "C₁₋₃ alkyl" or "C₁₋₄ alkyl" means alkyl with the carbon atom number of 1-3 or 1-4. Specific examples of "C₁₋₃ alkyl" include methyl, ethyl, propyl, isopropyl. Examples of "C₁₋₄ alkyl" include, in addition to the functional groups described above, butyl, isobutyl, sec-butyl, tert-butyl.

The "cycloalkyl" means a monocyclic saturated hydrocarbon group or a polycyclic saturated hydrocarbon group. For example, "C₃₋₆ cycloalkyl" means cycloalkyl with the carbon atom number of 3-6, and also includes a partially crosslinked structure or a fused ring structure with aryl or heteroaryl. Specific examples of "C₃₋₆ cycloalkyl" include cyclopropyl, cyclobutyl, cyclopentyl, cyclohexyl.

The "saturated heterocycle" means a monocyclic group containing 1 to 2 nitrogen atoms, oxygen atoms, or sulfur atoms, other than carbon atoms. The "3-6 membered saturated heterocycle" means a saturated heterocycle composed by 3-6 atoms. For example, its examples include aziridine, azetidine, pyrrolidine, and piperidine. The "saturated ring" means "cycloalkyl" or "saturated heterocycle" described above.

The "alkoxy" means a functional group in which a straight chain- or branched chain-saturated hydrocarbon group is bound to an oxygen atom. For example, "C₁₋₃ alkoxy" or "C₁₋₄ alkoxy" means alkoxy with the carbon atom number of 1-3 or 1-4. Specific examples of "C₁₋₃ alkoxy" include methoxy, ethoxy, propoxy, isopropoxy. Examples of "C₁₋₄ alkoxy" include, in addition to the functional groups described above, butyloxy.

The "alkylthio" means a functional group in which a straight chain- or branched chain-saturated hydrocarbon group is bound to a sulfur atom. For example, "C₁₋₄ alkylthio" means alkylthio with the carbon atom number of 1-4. Specific examples of "C₁₋₄ alkylthio" include methylthio, ethylthio, propylthio, isopropylthio, butylthio.

The "halogen" means a fluorine atom, a chlorine atom, a bromine atom, or an iodine atom. Among them, the "halogen" is preferably a fluorine atom or a chlorine atom.

The "aryl" means a monocyclic aromatic hydrocarbon group or a polycyclic aromatic hydrocarbon group. For example, "C₆₋₁₄ aryl" means aryl with the carbon atom number of 6-14 composing an aromatic ring. Specifically, its examples include phenyl, 1-naphthyl, 2-naphthyl, 1-anthracenyl. Among them, its preferable examples include a 1-naphthyl group or a phenyl group.

The "aryloxy" means a functional group in which a monocyclic aromatic hydrocarbon group or a polycyclic aromatic hydrocarbon group is bound to an oxgen atom. Specifically, its examples include phenyloxy, 1-naphthyloxy, 2-naphthyloxy. Among them, its preferable example is a phenyloxy group.

Examples of "heteroaryl" include a monocyclic 5-7 membered aromatic heterocyclic group or a bicyclic 8-11 membered aromatic heterocyclic group including 1-4 atoms independently selected from the group consisting of nitrogen atom, oxygen atom, and sulfur atom. Specifically, its examples include pyridyl, pyridazinyl, isothyazolyl, pyrrolyl, furyl, thienyl, thiazolyl, imidazolyl, pyrimidinyl, thiadiazolyl, pyrazolyl, oxazolyl, isooxazolyl, pyrazinyl, triazinyl, triazolyl, imidazolidinyl, oxadiazolyl, triazolyl, tetrazolyl, indolyl, indazolyl, chromenyl, quinolyl, isoquinolyl, benzofuranyl, benzothienyl, benzoxazolyl, benzothiazolyl, benzisoxazolyl, benzisothiazolyl, benzotriazolyl, benzimidazolyl. Examples of preferable heteroaryl include pyridyl, thienyl, quinolyl, and isoquinolyl.

In the present description, when n is 3 and the nitrogen-containing saturated heterocycle represents a piperidine ring, "the 2, 4, 5, or 6-position of the piperidine ring" means each position of carbon atom having a substituent, provided that, as shown in the following formula, each carbon atom forming the ring is numbered so that the position of the nitrogen atom of the piperidine ring (the position of R¹) becomes position 1 and the position of the amido group becomes position 3. For example, "the 4-position of the piperidine ring" means the 4th position of the carbon atom where a substituent is substituted, provided that each carbon atom forming the ring is numbered so that the position of the nitrogen atom becomes position 1 and the position of the amido group becomes position 3.

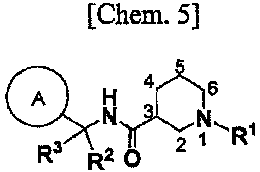

In the compound of the present invention, R¹, R², R³, R^{4a}-R^{4d}, R⁵-R¹⁸, A, and n shown in the formula (I) are what defined in Item 1 described above, and their preferable aspects will be explained as follows. However, the technical scope of the present invention is not limited by the scope of the compound shown below.

As R¹, preferably C₁₋₄alkyl is indicated, more preferably C₁₋₃ alkyl is indicated. As R¹, further preferably methyl, ethyl, propyl, or isopropyl is indicated, most preferably methyl is indicated.

As R², preferably C₁₋₄ alkyl which may be substituted by the same or different 1 to 5 substituents selected from the group consisting of fluorine atom and hydroxy is indicated, more preferably unsubstituted C₁₋₄alkyl is indicated. As R², further preferably methyl, ethyl, or isopropyl is indicated, most preferably methyl is indicated.

As R³, C₁₋₄ alkyl which may be substituted by the same or different 1 to 5 substituents selected from the group consisting of fluorine atom and hydroxy is indicated, preferably unsubstituted C₁₋₄ alkyl is indicated. As R³, more preferably methyl, ethyl, or isopropyl is indicated, further preferably methyl is indicated.

R³ together with R² may form a 3-6 membered saturated ring. The saturated ring may be substituted by 1-5 substituents. As the unsubstituted 3-6 membered saturated ring formed by R² and R³, preferably a saturated ring which may contain a -O- or -SO₂- is indicated, more preferably a saturated ring which may contain a -O-, and further more preferably 3-6 membered cycloalkyl is indicated. As the unsubstituted 3-6 membered saturated ring formed by R² and R³, most preferably cyclopropyl or cyclohexyl is indicated.

As R^{4a}, R^{4b}, R^{4c}, and R^{4d}, preferably indicated is a combination in which R^{4a} and R^{4b} are, similarly or differently, C₆₋₁₁ aryl which may be substituted by the same or different 1 to 5 substituents selected from the group consisting of C₁₋₄alkyl (which may be substituted by 1 to 5 fluorine atoms), C₃₋₆cycloalkyl, halogen, C₁₋₄ alkoxy (which may be substituted by 1 to 5 fluorine atoms), C₁₋₄ alkylthio, and cyano; C₁₋₄ alkyl which may be substituted by the same or different 1 to 5 substituents selected from the group consisting of fluorine atoms, hydroxy, and C₁₋₃ alkoxy; C₁₋₃ alkoxy which may be substituted by 1 to 5 fluorine atoms; hydrogen atoms; fluorine atoms; or -CO₂R⁹, provided that R^{4a} and R^{4b} are optionally substituted C₁₋₄ alkyl, the optionally substituted C₁₋₄ alkyl may unitedly form a 4-7 membered saturated ring, and R^{4c} and R^{4d} are hydrogen atoms. More preferably indicated is a combination in which R^{4a} and R^{4b} are, similarly or differently, C₁₋₄alkyl which may be substituted by the same or different 1 to 5 substituents selected from the group consisting of fluorine atom, hydroxy, and C₁₋₃ alkoxy; C₁₋₃ alkoxy which may be substituted by 1 to 5 fluorine atoms; fluorine atoms; hydrogen atoms; or -CO₂R⁹, and R^{4c} and R^{4d} are hydrogen atoms. Further preferably indicated is a combination in which **R^{4a}** and R^{4b} are, similarly or differently, C₁₋₄ alkyl or hydrogen atom(s), R^{4c} and R^{4d} are hydrogen atoms and most preferably indicated is a combination in which R^{4a}, R^{4b}, R^{4c}, and R^{4d} are hydrogen atoms.

A is C₆₋₁₄ aryl or 5-11 membered heteroaryl. The C₆₋₁₄ aryl and the 5-11 membered heteroaryl may be substituted by 1-5 substituents. As A, preferably C₆₋₁₄ aryl is indicated, more preferably phenyl or 1-naphthyl is indicated. As A, further preferably phenyl is indicated. When substituents exist in A, examples of the substituents preferably include C₁₋₄ alkyl (which may be substituted by 1-5 fluorine atoms), C₃₋₆ cycloalkyl, fluorine atoms, chlorine atoms, bromine atoms, C₁₋₄ alkoxy (which may be substituted by 1-5 fluorine atoms), C₁₋₄ alkylthio, cyano, -CO₂R¹⁰, -CONR¹¹R¹², -SO₂NR¹¹R¹², or -NR¹¹R¹². As the substituents described above, more preferably indicated are C₁₋₄alkyl (which may be substituted by 1-5 fluorine atoms), C₃₋₆ cycloalkyl, fluorine atoms, chlorine atoms, bromfine atoms, C₁₋₄ alkoxy (which may be substituted by 1-5 fluorine atoms), C₁₋₄ alkylthio, and cyano, further preferably indicated are C₁₋₄ alkyl (which may be substituted by 1-5 fluorine atoms), fluorine atoms, chlorine atoms, or C₁₋₄ alkoxy (which may be substituted by 1-5 fluorine atoms). As the substituents described above, most preferably indicated are methyl, ethyl, methoxy, trifluoromethoxy, fluorine atoms, or chlorine atoms. As the number of substituents substituted in A, preferably indicated are 1-4, more preferably indicated are 1-3. As the number of substituents, further preferably indicated is 1 or 2, most preferably indicated is 2.

As n, preferably indicated are 1-3, more preferably indicated is 2 or 3, further preferably indicated is 3. When n is 3, the nitrogen-containing saturated cycle is piperidine ring.

As R⁵, preferably indicated are hydrogen atom, C₁₋₄ alkyl, C₃₋₆ cycloalkyl, -CONR¹⁵R¹⁶, or -COR¹⁷, more preferably indicated are hydrogen atom, C₁₋₃ alkyl, or -COR¹⁷. As R⁵, further preferably indicated is C₁₋₃ alkyl, most preferably indicated is methyl.

R⁶ and R⁹ are the same or different, and preferably are C₁₋₄ alkyl. R⁶ and R⁹ are the same or different, and further preferably are methyl, ethyl, or tert-butyl, most preferably are methyl.

R⁷ and R⁸ are the same or different, and are preferably hydrogen atoms or C₁₋₄ alkyl, and more preferably are C₁₋₄alkyl. R⁷ and R⁸ are the same or different, and further preferably are methyl, ethyl, or isopropyl, and most preferably are methyl. As the saturated heterocycle unitedly formed by R⁷ and R⁸, preferably indicated is pyrrolidine or piperidine, more preferably indicated is pyrrolidine.

As R¹⁰, preferably C₁₋₄alkyl or C₃₋₆ cycloalkyl is indicated, more preferably C₁₋₄alkyl is indicated. As R¹⁰, further preferably methyl, ethyl, or tert-butyl is indicated, most preferably methyl is indicated.

R¹¹ and R¹² are the same or different, and are preferably hydrogen atoms or C₁₋₄ alkyl, and more preferably are C₁₋₄ alkyl. R¹¹ and R¹² are the same or different, and further preferably are methyl, ethyl, or isopropyl, and most preferably are methyl. As the saturated heterocycle unitedly formed by R¹¹ and R¹², preferably indicated is pyrrolidine or piperidine, more preferably indicated is pyrrolidine.

As R¹³, preferably C₁₋₄alkyl or C₃₋₆ cycloalkyl is indicated, and more preferably C₁₋₄ alkyl is indicated. As R¹³, further preferably methyl, ethyl, or isopropyl is indicated, and most preferably methyl is indicated.

As R¹⁴, preferably C₁₋₄ alkyl, C₃₋₆ cycloalkyl, or C₆₋₁₄ aryl is indicated, more preferably C₁₋₄alkyl or C₆₋₁₄ aryl is indicated. As R¹⁴, further preferably methyl, ethyl, isopropyl, or phenyl is indicated, and most preferably methyl is indicated.

As R¹⁵ and R¹⁶, preferably C₁₋₄ alkyl is indicated, more preferably methyl, ethyl, or isopropyl is indicated. As R¹⁵ and R¹⁶, further preferably methyl or isopropyl is indicated, and most preferably methyl is indicated. As the saturated heterocycle unitedly formed by R¹⁵ and R¹⁶, preferably indicated is pyrrolidine or piperidine, more preferably indicated is pyrrolidine.

As R¹⁷, preferably hydrogen atom or C₁₋₄ alkyl is indicated, more preferably hydrogen atom or methyl is indicated.

As R¹⁸, preferably C₁₋₄ alkyl is indicated, more preferably methyl, ethyl, or tert-butyl is indicated. As R¹⁸, further preferably methyl or ethyl is indicated, and most preferably methyl is indicated.

In the compound of the present invention, as a preferable compound, for example, the following Compound A is indicated, and as a more preferable compound, Compound B is indicated.

### [Compound A]

A compound represented by formula (I), wherein R¹ is C₁₋₃ alkyl,
R² and R³ are similarly or differently C₁₋₄ alkyl which may be substituted by the same or different 1-5 substituents selected from the group consisting of fluorine atoms and hydroxy, and R² and R³ may unitedly form a 3-6 membered saturated ring which may contain a -O-or -SO₂-,
R^{4a} and R^{4b} are, similarly or differently, C₆₋₁₄ aryl which may be substituted by the same or different 1 to 5 substituents selected from the group consisting of C₁₋₄ alkyl (which may be substituted by 1 to 5 fluorine atoms), C₃₋₆ cycloalkyl, halogen, C₁₋₄ alkoxy (which may be substituted by 1 to 5 fluorine atoms), C₁₋₄ alkylthio, hydroxy and cyano; C₁₋₄ alkyl which may be substituted by the same or different 1 to 5 substituents selected from the group consisting of fluorine atoms, hydroxy, and C₁₋₃ alkoxy; C₁₋₃ alkoxy which may be substituted by 1 to 5 fluorine atoms; hydrogen atoms; fluorine atoms; or -CO₂R⁹, provided that R^{4a} and R^{4b} are optionally substituted C₁₋₄ alkyl, the optionally substituted C₁₋₄ alkyl may unitedly form a 4-7 membered saturated ring,
R^{4c} and R^{4d} are hydrogen atoms,
A is C₆₋₁₄ aryl, the C₆₋₁₄ aryl may be substituted by the same or different 1-5 substituents selected from the group consisting of C₁₋₄ alkyl (which may be substituted by 1-5 fluorine atoms), C₃₋₆ cycloalkyl, fluorine atoms, chlorine atoms, bromine atoms, C₁₋₄ alkoxy (which may be substituted by 1-5 fluorine atoms), C₁₋₄ alkylthio, cyano, -CO₂R¹⁰, -CONR¹¹R¹², -SO₂NR¹¹R¹², or -NR¹¹R¹²,
R⁹ and R¹⁰ are methyl,
R¹¹ and R¹² are, similarly or differently, methyl or ethyl, or R¹¹ and R¹² unitedly form a pyrrolidine ring, and
n is 2 or 3, and preferably is 3,
or its pharmaceutically acceptable salt.

### [Compound B]

A compound represented by formula (I), wherein R¹ is C₁₋₃ alkyl,
R² and R³ are similarly or differently C₁₋₄ alkyl,
R^{4a} and R^{4b} are, similarly or differently, C₁₋₄ alkyl which may be substituted by the same or different 1 to 5 substituents selected from the group consisting of fluorine atoms, hydroxy, and C₁₋₃ alkoxy; C₁₋₃ alkoxy which may be substituted by 1 to 5 fluorine atoms; hydrogen atoms; or fluorine atoms,
R^{4c} and R^{4d} are hydrogen atoms,
A is C₆₋₁₄ aryl, the C₆₋₁₄ aryl may be substituted by the same or different 1-5 substituents selected from the group consisting of C₁₋₄ alkyl (which may be substituted by 1-5 fluorine atoms), C₃₋₆ cycloalkyl, fluorine atoms, chlorine atoms, bromine atoms, C₁₋₄ alkoxy (which may be substituted by 1-5 fluorine atoms), C₁₋₄ alkylthio, or cyano, and
n is 3,
or its pharmaceutically acceptable salt.

As the compound of the present invention, especially preferable are compounds selected from the group consisting of:
(3S)-1-methyl-N-[2-(naphthalene-1-yl)propane-2-yl]pyperidine-3-carboxamide;
N-[1-(2,3-dimethylphenyl)cyclopropyl]-1-methylpiperidine-3-ca rboxamide;
N-[4-(3,5-dichlorophenyl)tetrahydro-2H-pyran-4-yl]-1-methylpi peridine-3-carboxamide;
(3S)-N-[2-(2,3-dichlorophenyl)propane-2-yl]-1-methylpiperidin e-3-carboxamide;
(3S)-N-[2-(2,4-dichlorophenyl)propane-2-yl]-1-methylpiperidin e-3-carboxamide;
(3S)-N-[2-(2,5-dichlorophenyl)propane-2-yl]-1-methylpiperidin e-3-carboxamide;
(3S)-N-[2-(2,3-dimethylphenyl)propane-2-yl]-1-methylpiperidin e-3-carboxamide;
(3S)-N-[2-(2-chloro-3-methylphenyl)propane-2-yl]-1-methylpip eridine-3-carboxamide;
(3S)-N-[2-(3-chloro-2-methylphenyl)propane-2-yl]-1-methylpip eridine-3-carboxamide;
(3S)-N-[2-(3-chloro-5-methylphenyl)propane-2-yl]-1-methylpip eridine-3-carboxamide;
(3S)-N-[2-(4-chloro-2-methoxylphenyl)propane-2-yl]-1-methylp iperidine-3-carboxamide;
(3S)-N-[2-(4-chloro-2-ethylphenyl)propane-2-yl]-1-methylpiper idine-3-carboxamide;
(3S)-N-[2-(2,4-diethylphenyl)propane-2-yl]-1-methylpiperidine-3-carboxamide;
(3S)-N-[2-(2,4-dimethylphenyl)propane-2-yl]-1-methylpiperidin e-3-carboxamide;
(3S)-N-[2-(2-chloro-4-methylphenyl)propane-2-yl]-1-methylpip eridine-3-carboxamide;
(3S)-N-[2-(2-chloro-5-methylphenyl)propane-2-yl]-1-methylpip eridine-3-carboxamide;
(3S)-N-[2-(2-chlorophenyl)propane-2-yl]-1-methylpiperidine-3-carboxamide;
(3S)-N-[2-(2-methylphenyl)propane-2-yl]-1-methylpiperidine-3-carboxamide;
(3S)-N-[2-(3-chloro-4-methylphenyl)propane-2-yl]-1-methylpip eridine-3-carboxamide;
(3S)-N-[2-(5-chloro-2-methylphenyl)propane-2-yl]-1-methylpip eridine-3-carboxamide;
(3S)-N-{2-[2-(methylsulfanyl)phenyl]propane-2-yl}-1-methylpi peridine-3-carboxamide;
(3S)-N-[2-(2-bromophenyl)propane-2-yl]-1-methylpiperidine-3-carboxamide;
(3S)-N-[2-(2-chloro-3-fluorophenyl)propane-2-yl]-1-methylpipe ridine-3-carboxamide;
(3S)-N-[2-(2-chloro-4-fluorohenyl)propane-2-yl]-1-methylpiperi dine-3-carboxamide;
(3S)-N-[2-(2-chloro-5-fluorohenyl)propane-2-yl]-1-methylpiperi dine-3-carboxamide;
(3S)-N-[2-(2-ethylphenyl)propane-2-yl]-1-methylpiperidine-3-c arboxamide;
(3S)-N-[2-(2-chlorophenyl)propane-2-yl]-1-ethylpiperidine-3-ca rboxamide; and
N-[2-(naphthalene-1-yl)propane-2-yl]-1-(propane-2-yl)piperidin e-3-carboxamide, their racemic modifications or their optically active substances, or their pharmaceutically acceptable salts.

The pharmaceutically acceptable salts of compounds represented by formula (I) mean pharmaceutically acceptable acid addition salts of compounds represented by formula (I) having a functional group that can form an acid addition salt in the structure. Specific examples of acid addition salts include inorganic acid salts such as hydrochloride, hydrobromide, hydroiodide, sulfate, perchlorate, and phosphate; organic acid salts such as oxalate, malonate, maleate, fumarate, lactate, malate, citrate, tartrate, benzoate, trifluoroacetate, acetate, methanesulfonate, p-toluenesulfonate, and trifluoromethanesulfonate; and amino-acid salts such as glutamate and aspartate.

If the compound of the present invention represented by formula (I) has an acidic functional group such as carboxyl group, the compound can form salts with various bases. In such cases, examples of pharmaceutically acceptable salts include alkali metal salts such as sodium salt and potassium salt; alkaline-earth salts such as calcium salt; and ammonium salt. These salts may be obtained, after mixing the compound of the present invention represented by formula (I) with an acid or a base, through conventional methods such as recrystallization.

Here, the following abbreviations may be used, in order to simplify the description: o-, ortho-; m-, meta-; p-, para-; t-, tert-; s-, sec-; THF, tetrahydrofuran; DMF, N,N-dimethylformamide; DME, 1,2-dimethoxyethane; DMA, dimethylacetamide; NMP, N-methylpyrrolidone; DMSO, dimethyl sulfoxide; d₆-DMSO, deuterated dimethyl sulfoxide; Boc, tert-butoxycarbony; EDCI, 1-ethyl-3-(3-dimethylaminopropyl)carbodiimide; HOBt, 1-hydroxybenzotriazole; HATU, 2-(1H-7-azabenzotriazole-1-yl)-1,1,3,3-tetramethyluronium hexafluorophosphate; DCC, N,N'-dicyclohexylcarbodiimide; PyBOP, benzotriazole-1-yl-oxy-tris(pyrrolidino)phosphonium hexafluorophosphate; DMAP, 4-dimethylaminopyridine.

Producing method of the compound of the present invention will be described as follows: The compound of the present invention represented by formula (I) may be prepared by, for example, the following preparation method.

The compound represented by formula (I) may be prepared by the following preparation method. 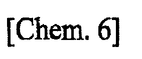 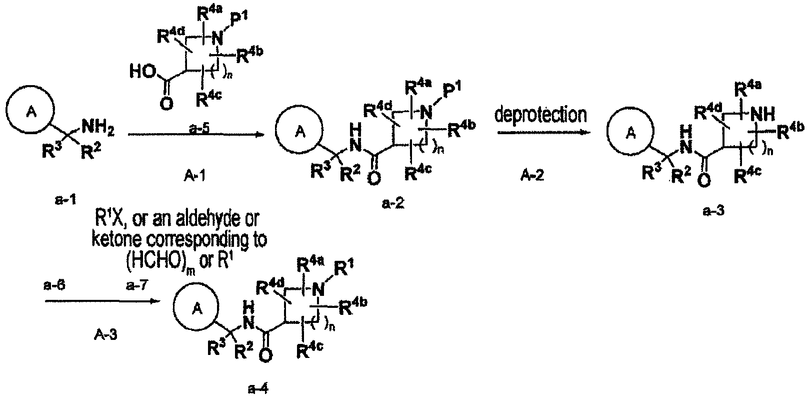 (wherein, A, R¹, R², R³, R^{4a}-R^{4d}, and n are as defined in Item 1, and P¹ means a protective group for the amino group, X means a leaving group being halogen and the like. (HCHO)ₘ means formaldehyde or paraformaldehyde.)

The compound a-1 may be commercially available, or may be synthesized with methods described in, for example, Tetrahedron Letters, 1987, 28, 6513-6516, Bioorganic and Medicinal Chemistry, 2011, 19, 5238-5246, WO 1999/19301, Journal of Medicinal Chemistry, 2011, 54, 1836-1846, and the like.

The compound a-5 may be commercially available, or may be synthesized with methods described in, for example, WO 2011/26917, Tetrahedron, 2007, 63, 10486-10496, WO 2006/91697, WO 2010/118207, WO 2011/26904, Journal of Medicinal Chemistry, 2006, 49, 942-946, WO 2012/36997, Journal of American Chemical Society, 2011, 133, 2878-2880, Bioorganic and Medicinal Chemistry, 2009, 17, 5639-5647, European Journal of Medicinal Chemistry, 1999, 34, 363-380, WO 2004/58727, and the like.

### Step [A-1]

In this step, a condensation reaction between the compound a-1 and compound a-5 is performed, and compound a-2 is obtained. This reaction may be performed according to conventional methods. For example, this reaction may be performed through converting compound a-5 into reactive derivatives (for example, lower alkyl esters, active esters, acid anhydrides, acid halides, and the like), and reacting with compound a-1. Specific examples of active ester include p-nitrophenyl ester, N-hydroxysuccinimide ester, pentafluorophenyl ester. Specific examples of acid anhydrides include mixed acid anhydrides with ethyl chlorocarbonate, isobutyl chlorocarbonate, isovaleric acid, pivalic acid.

The compound a-2 may be also prepared by reacting compound a-1 and compound a-5 in the presence of one condensing agent. Specific examples of condensing agents include DCC, EDCI, HATU, PyBOP Each of these condensing agents may be used solely or in combination with one peptide synthesizing reagent such as N-hydroxysuccinimide, HOBt, and DMAP.

The reaction between the compound a-1 and compound a-5 is performed in at least a solvent or in the absence of solvents. Specific examples of solvents, should be selected depending on types of starting compounds, but include, for example, toluene, THF, dioxane, DME, dichloromethane, chloroform; ethyl acetate, acetone, acetonitrile, DMF, DMSO. Each of the solvents described above may be used solely or as mixed solvents. The compound a-1 may be used in the form of an aqueous solution or acid addition salts such as hydrochloride, and may generate a free base in the reaction system. This reaction is normally performed in the presence of a base. Specific examples of bases to be used include inorganic bases such as potassium carbonate, sodium hydrogen carbonate, and organic bases such as triethylamine, ethyldiisopropylamine, N-methymorpholine, pyridine, 4-dimethylaminopyridine. The reaction temperature, may be different depending on types of starting compounds and the like, but, is normally about -30°C to about 150°C, preferably about -10°C to about 70°C. The reaction time, may be different depending on types of starting compounds and the like, but, is normally about 30 minutes to about 72 hours, preferably about an hour to about 12 hours.

### Step [A-2]

In this step, a protective group P¹ for amino group of the compound a-2 obtained in step A-1 described above is deprotected, and compound a-3 is obtained. This step may be performed according the method described in the Protective Groups in Organic Synthesis (written by Theodora W. Greene, Peter G. M. Wuts, published by John Wiley & Sons, Inc. in 1999), and the like.

### Step [A-3]

In this step, the amino group of the compound a-3 obtained in step A-2 described above is alkylated by, for example, reductive amination, and compound a-4 is obtained. This reaction may be performed according to conventional methods. For example, this reaction is performed, in a suitable solvent or in the absence of solvents, by reacting the compound a-3 with various ketones or aldehydes, and the like, in the presence of a reducing agent, and the like. Specific examples of reducing agents and the like include sodium borohydride, lithium aluminum hydride, sodium triacetoborohydride, sodium cyanoborohydride. Specific examples of solvents, should be selected depending on types of starting compounds, but include, for example, dichloromethane, chloroform, dichloroethane, THF, 1,4-dioxane, DME, acetonitrile, DMF, DMA, NMP, DMSO, acetic acid, water, or alcohols such as methanol, ethanol, isopropanol, 2,2,2-trifluoroethanol. Each of the solvents described above may be used solely or as mixed solvents. The reaction temperature, may be different depending on types of starting compounds and reagent, but, is normally about -20°C to about 200°C, preferably about 0°C to about 100°C. The reaction time, may be different depending on types of starting compounds and the like, but, is normally about 30 minutes to about 72 hours, preferably about an hour to about 12 hours.

The compound a-4 may be also obtained by reacting various alkyl halides and the like with compound a-3, in a suitable solvent or in the absence of solvents. Specific examples of solvents, should be selected depending on types of starting compounds, but include, for example, dichloromethane, chloroform, dichloroethane, THF, 1,4-dioxane, DME, acetonitrile, DMF, DMA, NMP, DMSO. Each of the solvents described above may be used solely or as mixed solvents. The compound a-3 may be used in the form of acid addition salts such as hydrochloride, and may generate a free base in the reaction system. This reaction is normally performed in the presence of a base. Specific examples of bases to be used include inorganic bases such as potassium carbonate, sodium hydrogen carbonate, cesium carbonate, and organic bases such as triethylamine, ethyldiisopropylamine, N-methymorpholine, pyridine, 4-dimethylaminopyridine. The reaction temperature, may be different depending on types of starting compounds and reagent, but, is normally about -20°C to about 200°C, preferably about 0°C to about 100°C. The reaction time, may be different depending on types of starting compounds and the like, but, is normally about 30 minutes to about 72 hours, preferably about an hour to about 12 hours.

The compound of the present invention represented by formula (I) and its intermediate can be separated and purified with known methods to those skilled in the art. Examples of these methods include, for example, extraction, partition, reprecipitation, various types of column chromatography (for example, silica gel column chromatography, ion-exchange column chromatography, preparative liquid column chromatography), and recrystallization. As a solvent to be used for recrystallization, for example, alcoholic solvents such as methanol, ethanol, and 2-propanol; ether-based solvents such as diethyl ether; ester-based solvents such as ethyl acetate; aromatic hydrocarbon-based solvents such as benzene and toluene; ketone-based solvents such as acetone; halogenated solvents such as dichloromethane and chloroform; hydrocarbon-based solvents such as hexane; aprotic solvents such as dimethylformamide and acetonitrile; water; and these mixtures may be used. As other purifying methods, methods described in Volume 1 of Series of Experimental Chemistry (edited by The Chemical Society of Japan, published by Maruzen Co., Ltd.) and the like may be used. The molecular structure of the compound of the present invention may be easily determined, with referring to the structure derived from each starting compound, by spectroscopic techniques such as nuclear magnetic resonance spectrometry, infrared absorption spectroscopy, and circle second optical spectrum analysis method; and mass spectrometry.

The compounds of the present invention represented by formula (I) and their pharmaceutically acceptable salts may generate asymmetry or may have a substituent possessing an asymmetric carbon, and optical isomers of such a compound may exist. The compounds of the present invention include mixtures of each of such isomers and isolated compounds, and may be prepared according to normal methods. Examples of the preparing methods include, for example, using a source material with an asymmetric carbon, or introducing an asymmetric carbon in the middle of the stage. For example, in a case of an optical isomer, by either of using an optically active source material or performing optical resolution and the like in the appropriate stage of the preparation step, the optical isomer may be obtained. As an optical resolution method, for example, indicated is diastereomer method in which, when the compound represented by formula (I) or its intermediate has a basic functional group, a salt of the compound is formed in an inert solvent (for example, alcoholic solvents such as methanol, ethanol, and 2-propanol; ether-based solvents such as diethyl ether; ester-based solvents such as ethyl acetate; hydrocarbon-based solvents such as toluene; aprotic solvents such as acetonitrile; and these mixtures) by using an optically active acid (for example, monocarboxylic acids such as mandelic acid, N-benzyloxyalanine, and lactic acid; dicarboxylic acids such as tartaric acid, o-diisopropylididenetartaric acid, and malic acid; and sulfonic acids such as camphorsulfonic acid and bromocamphorsulfonic acid). If the intermediate of the compound of the present invention represented by formula (I) has an acidic functional group such as a carboxylic acid, by forming a salt of the intermediate with using an optically active amine (for example, an organic amine such as 1-phenylethylamine, quinine, quinidine, cinchonidine, cinchonine, and strychnine), optical resolution can be performed.

The temperature at which the salt is formed is selected in the scope between room temperature and the boiling point of the solvent. In order to improve the optical purity, once raising the temperature close to the boiling point of the solvent is preferable. When filtering the separated salt, cooling as needed can improve the yield. The using amount of the optically active acid or base is appropriately in a range of 0.5-2.0 chemical equivalents per substrate, preferably 0.8-1.2 chemical equivalents. When needed, the crystal may be recrystallized in an inert solvent (for example, alcoholic solvents such as methanol, ethanol, and 2-propanol; ether-based solvents such as diethyl ether; ester-based solvents such as ethyl acetate; aromatic hydrocarbon-based solvents such as toluene; aprotic solvents such as acetonitrile; and these mixtures), and a high-purity optically active salt may be obtained. When needed, the optically resolved salt may be also treated with an acid or base, and obtained as a free body.

Even if the intermediate of the compound of the present invention represented by formula (I) has a carboxylic group, by forming amide with using an optically active amine (for example, 1-phenylethylamine, and the like), optical resolution can be performed.

The compound of the present invention is useful as an agent for activating somatostatin receptor subtype 4. Moreover, the compound of the present invention may be a useful as an agent for treating or preventing diseases that can be treated or prevented by activating somatostatin receptor subtype 4. Examples of such diseases include, for example, diseases caused by Aβ such as Alzheimer's disease, cognitive impairment, memory disorder, learning disorder, mild cognitive impairment, senile dementia, amyloidosis, and cerebrovascular angiopathy; neurodegenerative diseases such as Perkinson's disease and multiple sclerosis; epilepsy, depression, behavior disorder, attention deficit disorder, pain, neurogenic bladder, hyperproliferative disorder, acromegaly; cancers such as melanoma, breast cancer, prostatic adenoma, prostate cancer, lung cancer, intestinal cancer, and skin cancer; arthritis, rheumatoid arthritis, rheumatoid spondylitis, psoriasis, atopic dermatitis, asthma, Graves' disease, inflammatory bowel disease, nephropathy, diabetic angiopathy, ischemic disease, benign prostatic hypertrophy, age-related macular degeneration, glaucoma, and diabetic retinopathy. The compound of the present invention may be an especially useful as an agent for treating Alzheimer's disease and various neurodegenerative diseases.

The administration method of the compound of the present invention may be any of oral administration, non-oral administration, or intrarectal administration. Daily dose of the compound of the present invention varies depending on types of the compound, administration methods, symptoms and age of the patient, and the like. For example, in oral administration cases, normally, about 0.01 mg to 1000 mg per 1 kg body weight of humans or mammals, more preferably about 0.1 mg to 500 mg, may be administered bolusly or separatedly. In non-oral administration cases such as intravenous injection and the like, normally, for example about 0.01 mg to 300 mg per 1 kg body weight of humans or mammals, more preferably about 1 mg to 100 mg, may be administered.

When used for pharmaceutical applications as described above, the compound of the present invention is normally administered in the form of a prepared formulation mixed with a carrier. As the carrier, non-toxic materials that are commonly used in this field and do not react with the compound of the present invention are used. Specific examples of the carrier include, for example, citric acid, glutamic acid, glycine, lactose, inositol, glucose, mannitol, dextran, sorbitol, cyclodextrin, starch, partially pregelatinized starch, sucrose, methyl parahydroxybenzoate, propyl parahydroxybenzoate, magnesium aluminometasilicate, synthetic aluminum silicate, crystalline cellulose, sodium carboxymethylcellulose, hydroxypropyl starch, carboxymethylcellulose calcium, ion-exchange resins, methylcellulose, gelatin, gum arabic, pullulan, hydroxypropylcellulose, less substituted hydroxypropylcellulose, hydroxypropylmethylcellulose, polyvinylpyrrolidone, polyvinyl alcohol, alginic acid, sodium alginate, light anhydrous silicic acid, magnesium stearate, talc, tragacanth, bentonite, Veegum, carboxy vinyl polymer, titanium oxide, sorbitan fatty acid ester, sodium lauryl sulfate, glycerin, glycerin-fatty acid ester, purified lanolin, glycerogelatin, polysorbate, macrogol, vegetable oil, wax, propylene glycol, ethanol, benzyl alcohol, sodium chloride, sodium hydroxide, hydrochloric acid, water.

Examples of its dosage form include tablet, capsule, granules, powders, syrup, suspensions, injections, suppository, eye drops, ointment, embrocation, application, inhalation. These formulations may be prepared according to conventional methods. In cases of liquid formulations, before using, it may be solved or suspended in water or other appropriate medium. In cases of tablets or granules, the compound may be coated with well-known methods. Moreover, these formulations may contain therapeutically valuable other ingredients. Examples

Hereinafter, the present invention will be more specifically explained by using Reference Examples, Examples, and Test Examples, however, they do not limit the present invention. Compounds were identified based on elemental analysis values, data from mass spectra, a high performance liquid chromatography-mass spectrometer (LCMS), IR spectra, NMR spectra, high performance liquid chromatography (HPLC), and the like.

The following abbreviations may be used in Tables of Reference Examples, Examples, and Test Examples, in order to simplify the description. As abbreviations used for substituents, Me means methyl, Et means ethyl, Ph means phenyl, Bn means benzyl, Tf means trifluoromethylsulfonyl, Ac means acetyl, TBS means tert-butyldimethylsilyl. As abbreviations used for reagents, TFA means trifluoroacetic acid, TBAF means tetra-n-butylammonium fluoride, Boc₂O means di-tert-butyl dicarbonate, WSCD means 1-ethyl-3-(3-dimethylaminopropyl) carbodiimide, DBU means diazabicycloundecene, XtalFluor-E means (diethylamino) difluorosulfonium tetrafluoroborate. As abbreviations used for NMR, s means singlet, d means doublet, dd means double doublet, t means triplet, td means double triplet, q means quartet, m means multiplet, br means broadness, brs means broad singlet, brd means broad doublet, brt means brod triplet, and J means coupling constant.

Measuring conditions with the high performance liquid chromatography-mass spectrometer (LCMS) were as follows: Observed mass spectrometry value [MS(m/z)] is indicated with MH+, and retention time is indicated with Rt (minutes). As for each observed values, measuring conditions A to C were as follows;

### Measuring condition A

Detector, Agilent 1100 series for API series (manufactured by Applied Biosystems Co.,);
HPLC, API 150EX LC/MS system (manufactured by Applied Biosystems Co.,);
Column, YMC CombiScreen Hydrosphere C18 (s-5 µm, 12 nm, 4.6 x 50 mm);
Solvent, solution A is 0.05% TFA/H₂O, solution B is 0.05% TFA/MeOH;
Gradient Condition, 0.0-6.0 minutes, A/B = 75:25-1:99 (linear gradient);
Flow rate, 3.5 mL/minute;
UV, 254 nm;
Column temperature, 40°C

### Measuring condition B

Detector, Waters ACQUITY UPLC;
Column, ACQUITY UPLC BEH C18 1.7 µm 2.1 mm x 50 mm column;
Solvent, solution A is 0.05% HCOOH/H₂O, solution B is CH₃CN;
Gradient Condition, 0.0-1.3 minutes, A/B = 90:10-1:99 (linear gradient)
1.3-1.5 minutes, A/B = 1:99
1.5-2 minutes, A/B = 90:10;
Flow rate, 0.75 mL/minute;
UV, 220 nm, 254 nm;
Column temperature, 50°C

### Measuring condition C

Detector, Shimadzu LCMS-2020;
Column, Phenomenex Kinetex 1.7 µm C18 2.1 mm x 50 mm;
Solvent, solution A is MeOH, solution B is 0.05% TFA/H₂O;
Gradient Condition,
0.0 minute, A/B = 30:70
0.0-1.90 minutes, A/B = 99:1
1.91-3.00 minutes, A/B = 30:70;
Flow rate, 0.5 mL/minute;
UV, 220 nm;
Column temperature, 40°C

Reference Example 1

### 2-(2-fluorophenyl)propane-2-ol

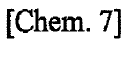

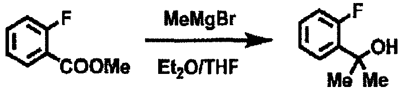

A THF solution (9 mL) of methyl 2-fluorobenzoate (1424 mg) was cooled on ice bath while stirring, a 3 mol/L methylmagnesium bromide/diethyl ether solution (9.2 mL) was dropped. After stirring for 1.5 hours at room temperature, a saturated aqueous solution of ammonium chloride (25 mL) and water (50 mL) was added sequentially to the reaction solution. After the product was extracted with ethyl acetate (40 mL), the organic layer was dried with anhydrous sodium sulfate, filtered and concentrated under reduced pressure to give 2-(2-fluorophenyl)propane-2-ol (1472 mg) as an oil.

¹H NMR (300 MHz, CDCl₃) δ 1.65 (s, 6H), 2.12-2.14 (m, 1H), 6.99-7.06 (m, 1H), 7.10-7.15 (m, 1H), 7.21-7.26 (m,1H), 7.53-7.57 (m, 1H)

### Reference Example 2

### 2-(2-fluorophenyl)propane-2-amine

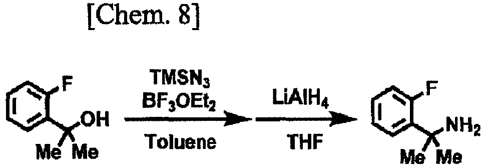

To a toluene solution (6 mL) of 2-(2-fluorophenyl)propane-2-ol (925 mg) while stirring, trimethylsilyl azide (830 mg) and boron trifluoride-diethyl ether complex (1021 mg) were sequentially dropped, and stirred for 15 minutes at room temperature. After disappearance of starting materials was verified, a saturated aqueous solution of sodium hydrogen carbonate (50 mL) was added to the reaction solution, and the product was extracted with ethyl acetate (40 mL). The organic layer was dried with anhydrous sodium sulfate, filtered and concentrated under reduced pressure. The obtained residue was dissolved in THF (15 mL), the solution was gradually dropped into a THF suspension (15 mL) of lithium aluminium hydride (228 mg) at room temperature. After stirring for 2 hours, to the reaction solution, water (230 µL), a 2 mol/L sodium hydroxide aqueous solution (230 µL), and water (690 µL) were gradually and sequentially dropped to the reaction solution. After being filtered with Celite, the mixture was concentrated under reduced pressure. The obtained residue was purified with silica gel column chromatography (chloroform/methanol = 92/8 to 85/15) to give 2-(2-fluorophenyl)propane-2-amine (410 mg) as an oil.

¹H NMR (300 MHz, CDCl₃) δ 1.55 (s, 6H), 6.99-7.12 (m, 2H), 7.18-7.27 (m, 1H), 7.41-7.46 (m, 1H)

### Reference Examples 3-63

The Corresponding starting compounds were used, reacted and treated in the same way as Reference Example 2 to give compounds shown in Tables 1 to 3.

**[Table 1]**

| Reference Example | Structure | Reference Example | Structure | Reference Example | Structure |
|---|---|---|---|---|---|
| 3 | 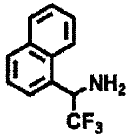 | 4 | 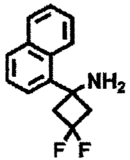 | 5 | 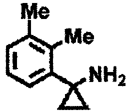 |
| 6 | 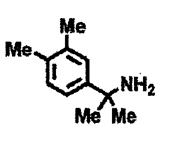 | 7 | 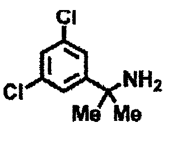 | 8 | 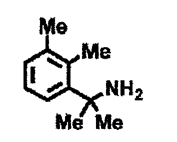 |
| 9 | 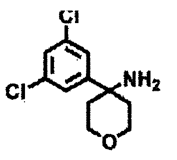 | 10 | 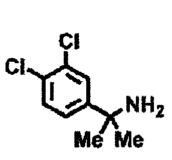 | 11 | 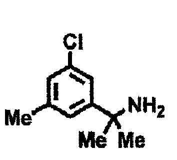 |
| 12 | 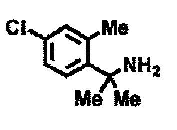 | 13 | 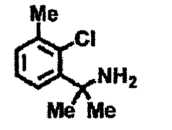 | 14 | 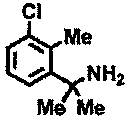 |
| 15 | 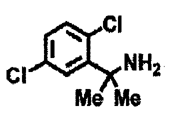 | 16 | 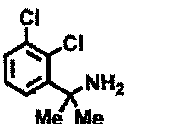 | 17 | 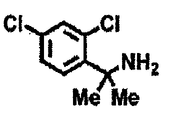 |
| 18 | 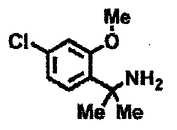 | 19 | 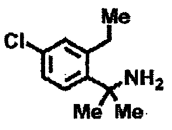 | 20 | 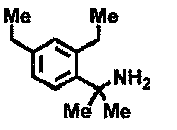 |
| 21 | 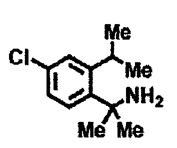 | 22 | 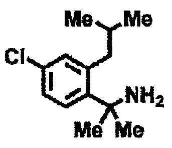 | 23 | 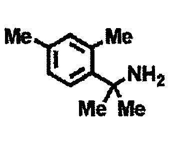 |

**[Table 2]**

| Reference Example | Structure | Reference Example | Structure | Reference Example | Structure |
|---|---|---|---|---|---|
| 24 | 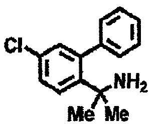 | 25 | 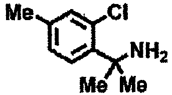 | 26 | 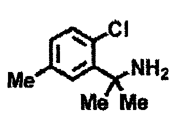 |
| 27 | 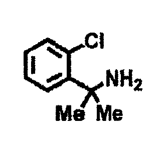 | 28 | 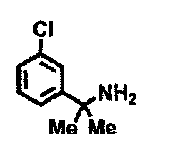 | 29 | 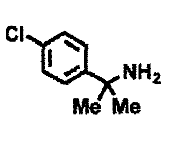 |
| 30 | 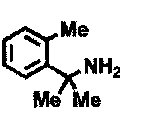 | 31 | 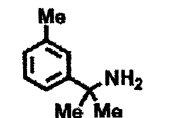 | 32 | 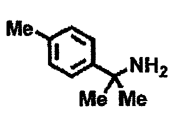 |
| 33 | 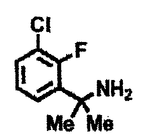 | 34 | 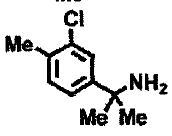 | 35 | 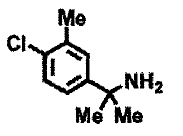 |
| 36 | 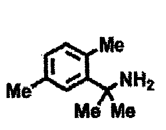 | 37 | 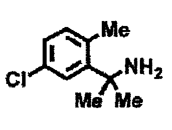 | 38 | 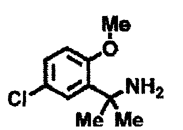 |
| 39 | 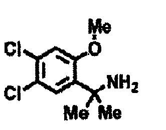 | 40 | 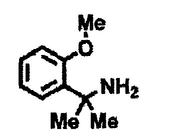 | 41 | 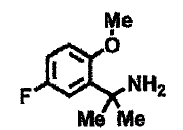 |
| 42 | 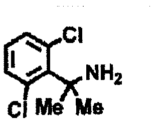 | 43 | 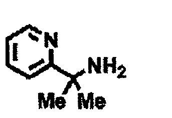 | 44 | 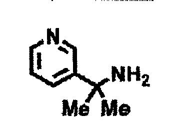 |

**[Table 3]**

| Preference Example | Structure | Reference Example | Structure | Reference Example | Structure |
|---|---|---|---|---|---|
| 45 | 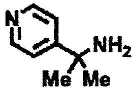 | 46 | 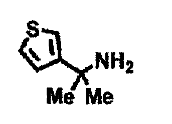 | 47 | 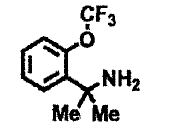 |
| 48 | 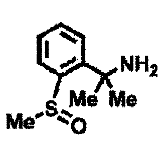 | 49 | 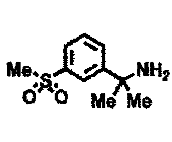 | 50 | 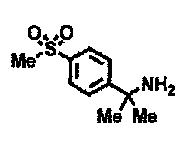 |
| 51 | 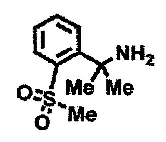 | 52 | 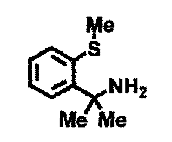 | 53 | 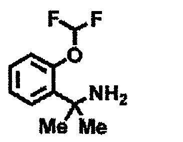 |
| 54 | 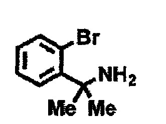 | 55 | 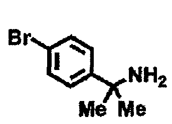 | 56 | 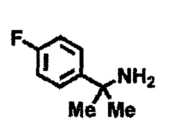 |
| 57 | 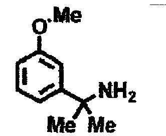 | 58 | 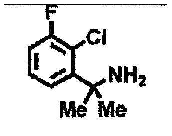 | 59 | 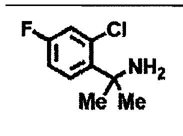 |
| 60 | 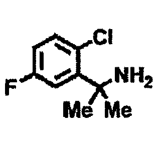 | 61 | 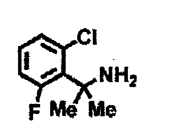 | 62 | 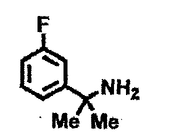 |
| 63 | 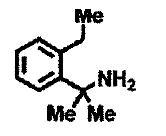 | | | | |

### Reference Example 64

### tert-butyl (3S)-3-{[2-(2-fluorophenyl) propane-2-yl]carbamoyl}piperidine-1-carboxylate

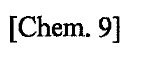

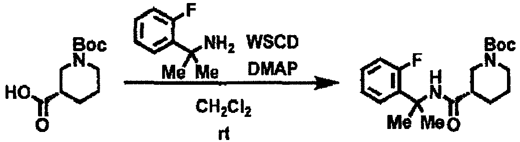

A dichloromethane solution (1 mL) of (3S)-1-(tert-butoxycarbonyl) piperidine-3-carboxylic acid (68 mg), WSCD (30 mg), 2-(2-fluorophenyl) propane-2-amine synthesized in Reference Example 2 (46 mg) and DMAP (11 mg) were stirred for 5 hours at room temperature. The reaction solution was directly purified with silica gel column chromatography (hexane/ethyl acetate = 79/21 to 0/100) to give tert-butyl (3 S)-3-{[2-(2-fluorophenyl)propane-2-yl]carbamoyl}piperidine-1-carbo xylate (90 mg) as a solid.

¹H NMR (300 MHz, CDCl₃) δ 1.30-1.95 (m, 4H), 1.47 (s, 9H), 2.26 (br, 1H), 2.60-3.45 (br, 2H), 3.45-4.00 (m, 2H), 6.95-7.00 (m, 1H), 7.07-7.09 (m 1H), 7.18-7.21 (m, 1H), 7.32-7.36 (m, 1H)

### Reference Example 65

### 1-phenyl-N-(propane-2-ylidene)methanamine

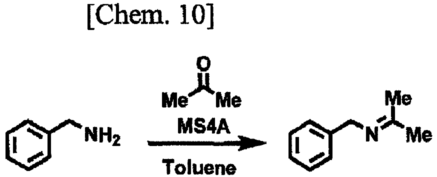

To a toluene (5 mL) solution of benzylamine (1.07 g), acetone (1.74 g) and molecular sieve 4A (3.0 g) were added, and the reaction solution was stirred under reflux for 2 hours at 120°C. After cooled to room temperature, the reaction solution was filtered with Celite. The filtrate was concentrated to give 1-phenyl-N-(propane-2-ylidene)methanamine (1.34 g) as an oil.

¹H NMR (300 MHz, CDCl₃) δ 1.93-1.95 (m, 3H), 2.09-2.10 (m, 3H), 4.45 (s, 2H), 7.12-7.44 (m, 5H)

### Reference Example 66

### N-benzyl-2-methylpent-4-ene-2-amine

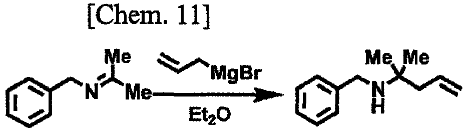

To a diethyl ether (20 mL) solution of 1-phenyl-N-(propane-2-ylidene)methanamine (1.34 g) at 0°C under a nitrogen atmosphere, a 1 mol/L allylmagnesium bromide/diethyl ether solution (22 mL) was dropped over 20 minutes, and the reaction solution was stirred for an hour at 0°C. After stirring for 3 hours at room temperature, a saturated aqueous solution of ammonium chloride (50 mL) was added to the reaction solution under cooling at 0°C. After the reaction solution was separated, and the product was extracted with diethyl ether (40 mL). After the organic layer was dried with anhydrous sodium sulfate, filtered and concentrated under reduced pressure to give N-benzyl-2-methylpent-4-ene-2-amine (1.45 g) as an oil.

¹H NMR (300 MHz, CDCl₃) δ 1.14 (s, 6H), 2.25 (d, 2H), 3.71 (s, 2H), 5.09-5.13 (m, 2H), 5.80-5.94 (m,1H) 7.18-7.35 (m, 5H)

### Reference Example 67

### ethyl 2-{[benzyl (2-methylpent-4-ene-2-yl)amino]methyl}prop-2-enoate

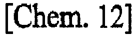

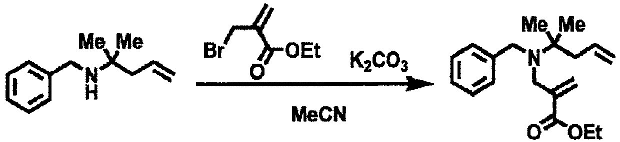

To an acetonitrile (15 mL) solution of N-benzyl-2-methylpent-4-ene-2-amine (1.45 g), potassium carbonate (1.11 g) and ethyl 2-bromomethylacrylate (1.06 g) were added sequentially. The reaction solution was stirred 18 hours at room temperature. After the reaction solution was filtered, the filtrate was concentrated under reduced pressure. The obtained residue was purified with silica gel column chromatography (hexane/ethyl acetate = 1/99) to give ethyl 2-{[benzyl (2-methylpent-4-ene-2-yl)amino]methyl}prop-2-enoate (1.24 g) as an oil.

¹H NMR (300 MHz, CDCl₃) δ 1.09 (s, 6H), 1.22 (t, 3H), 2.31 (d, 2H), 3.44 (s, 2H), 3.72 (s, 2H), 4.10 (q, 2H), 5.02-5.08 (m, 2H), 5.81 (q, 1H), 5.90-6.04 (m, 2H), 7.11-7.29 (m, 5H)

### Reference Example 68

### ethyl 1-benzyl-6,6-dimethyl-1,2,5,6-tetrahydropyridine-3-carboxylate

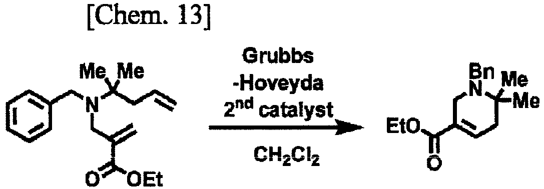

To a dichloromethane (20 mL) solution of ethyl 2-{[benzyl (2-methylpent-4-ene-2-yl)amino]methyl}prop-2-enoate (301 mg), Grubbs-Hoveyda 2^{nd} catalyst (31 mg) was added, and the reaction solution was refluxed for 3 hours at 50°C. The reaction solution was concentrated and purified with silica gel column chromatography (hexane/ethyl acetate = 5/95) to give ethyl 1-benzyl-6,6-dimethyl-1,2,5,6-tetrahydropyridine-3-carboxylate (157 mg) as an oil.

¹H NMR (300 MHz, CDCl₃) δ 1.13 (s, 6H), 1.22 (t, 3H), 2.19-2.22 (m, 2H), 3.16-3.19 (m, 2H), 3.63 (s, 2H), 4.12 (q, 2H), 6.91-6.94 (m,1H), 7.17-7.36 (m, 5H)

### Reference Example 69

### 1-(tert-butoxycarbonyl)-6,6-dimethylpiperidine-3-calboxylic acid

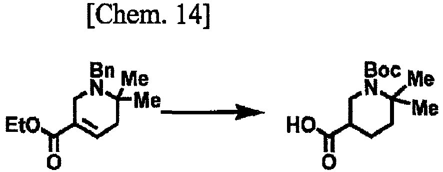

### [Step 1]

To a methanol (20 mL) solution of ethyl 1-benzyl-6,6-dimethyl-1,2,5,6-tetrahydropyridine-3-carboxylate (584 mg), magnesium (519 mg) was added. After stirred for 4 hours at room temperature, magnesium (550 mg) was added to the reaction solution. The solution was stirred for further 16 hours at room temperature. Then, to the reaction solution, a saturated aqueous solution of ammonium chloride (100 mL) was added. The product was extracted with dichloromethane (160 mL), and the organic layer was dried with anhydrous sodium sulfate, and filtered. The filtrate was concentrated under reduced pressure to give ethyl 1-benzyl-6,6-dimethylpiperidine-3-calboxylate (375 mg) as an oil. This product was not purified, and used for the next reaction.

### [Step 2]

Ethyl 1-benzyl-6,6-dimethylpiperidine-3-calboxylate obtained in Step 1 (137 mg) was dissolved to methanol (5 mL) in a pressure-resistant glass vessel. To the mixture, 20% palladium hydroxide/carbon carrier (68 mg) was added. After stirred for 16 hours at room temperature under a medium pressure-hydrogen atmosphere (about 0.40 MPa), the reaction mixture was filtered with Celite. The filtrate was concentrated under reduced pressure to give ethyl 6,6-dimethylpiperidine-3-calboxylate. This product was not purified, and directly used for the next reaction.

### [Step 3]

To a dichloromethane (5 mL) solution of ethyl 6,6-dimethylpiperidine-3-calboxylate obtained in Step 2, triethyl amine (77 µL) and Boc₂O (120 mg) were added. After stirred for 5 hours at room temperature, the reaction mixture was concentrated under reduced pressure to give 1-tert-butyl 3-ethyl 6,6-dimethylpiperidine-1,3-dicalboxylate. This product was not purified, and directly used for the next reaction.

### [Step 4]

To a methanol (2 mL) solution of 1-tert-butyl 3-ethyl 6,6-dimethylpiperidine-1,3-dicalboxylate, a 2 mol/L sodium hydroxide aqueous solution (2 mL) was added. After stirred for 30 minutes at 70°C, the reaction solution was cooled to room temperature. The reaction solution was neutralized by adding 2 mol/L hydrochloric acid (5 mL). The product was extracted with dichloromethane (60 mL), and the organic layer was dried with anhydrous sodium sulfate, filtered, and concentrated under reduced pressure. The residue was purified with silica gel column chromatography (chloroform/methanol = 95/5) to give 1-(tert-butoxycarbonyl)-6,6-dimethylpiperidine-3-carboxylic acid (100 mg) as an oil.

¹H NMR (300 MHz,, CDCl₃) δ 1.30-2.00 (m, 19H), 2.63-2.73 (m, 1H), 3.30-3.38 (m, 4H), 3.93-3.99 (m, 1H), 9.61 (br,1H)

### Reference Example 70

### 1-tert-butyl 3-methyl 4-{[trifluoromethyl]sunfonyl}oxy}-5,6-dihydropyridine-1,3(2H)-dicarb oxylate

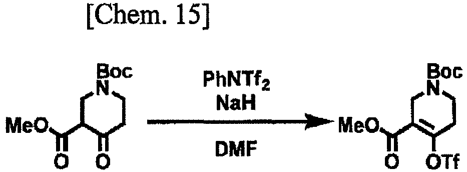

To a dimethylformamide solution (20 mL) of 1-tert-butyl 3-methyl 4-oxopiperidine-1,3-dicarboxylate (3.91 g), sodium hydride (0.36 g) was gradually added under cooling on ice bath. After the reaction solution was stirred for 10 minutes at 0°C, N-phenyltrifluoromethylsulfonimide (5.42 g) was gradually added. After the reaction solution was warmed to room temperature and stirred for 24 hours, N-phenyltrifluoromethylsulfonimide (2.71 g) was further added. After stirred for further 21 hours, water (100 mL) was added to the reaction solution, the product was extracted with ethyl acetate/hexane (90 mL/60 mL) twice. The organic layer was washed with water, dried with anhydrous sodium sulfate, filtered, and concentrated under reduced pressure. The obtained residue was purified with silica gel column chromatography to give 1-tert-butyl 3-methyl 4-{[trifluoromethyl] sunfonyl)oxy}-5,6-dihydropyridine-1,3(2H)-dicarboxylate (4.45 g) as an oil.

¹H NMR (CDCl₃) δ 1.48 (s, 9H), 2.46-2.58 (m, 2H), 3.63 (t, 2H), 3.84 (s, 3H), 4.28 (bs, 2H)

### Reference Example 71

### 1-tert-butyl 3-methyl 4-phenyl-5,6-dihydropyridine-1,3(2H)-dicarboxylate

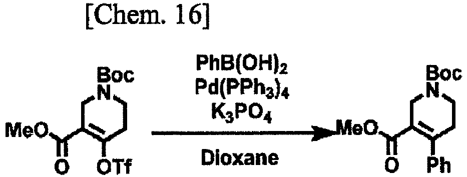

To a 1,4-dioxane solution (8 mL) of 1-tert-butyl 3-methyl 4-{[trifluoromethyl] sunfonyl} oxy}-5,6-dihydropyridine-1,3(2H)-dicarboxylate (417 mg), phenylboronic acid (170 mg), and tetrakis (triphenylphosphine) palladium (62 mg), tripotassium phosphate (455 mg) was added under a nitrogen atmosphere. The reaction solution was stirred for 3.5 hours at 85°C. After the reaction solution was cooled to room temperature, water (30 mL) was added, and the product was extracted with ethyl acetate (50 mL). The organic layer was dried with anhydrous sodium sulfate, and filtered. Then, the filtrate was concentrated under reduced pressure, the residue was purified with silica gel column chromatography (hexane/ethyl acetate = 90/10) to give 1-tert-butyl 3-methyl 4-phenyl-5,6-dihydropyridine-1,3(2H)-dicarboxylate (258 mg) as an oil.

¹H NMR (300 MHz, CDCl₃) δ 1.50 (s, 9H), 2.46-2.56 (m, 2H), 3.49 (s, 3H), 3.59-3.63 (m, 2H), 4.21-4.30 (m, 2H)

### Reference Example 72

### trans-1-(tert-butoxycarbonyl)-4-phenylpiperidine-3-calboxylic acid

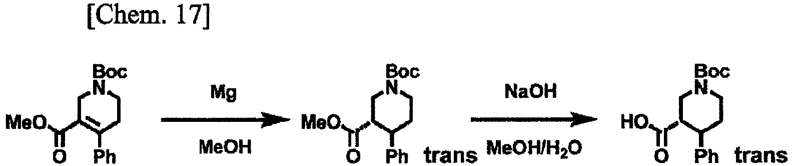

To a methanol solution (8 mL) of 1-tert-butyl 3-methyl 4-phenyl-5,6-dihydropyridine-1,3(2H)-dicarboxylate (255 mg), magnesium (195 mg) was added. After stirred for 15 hours at room temperature, ice water (50 mL), 2 mol/L hydrochloric acid (15 mL) were added sequentially to the reaction solution, and the product was extracted with dichloromethane (50 mL). The organic layer was dried with anhydrous sodium sulfate, and filtered. The filtrate was concentrated under reduced pressure to give 1-tert-butyl 3-methyl-trans-4-phenylpiperidine-1,3-dicarboxylate as a colorless oil. The obtained oil was dissolved in methanol (2 mL), a 2 mol/L sodium hydroxide aqueous solution (2 mL) was added to the solution. After stirred for 2.5 hours at 70°C, the reaction solution was neutralized by adding 2 mol/L hydrochloric acid (5 mL). The product was extracted with dichloromethane (50 mL), and the organic layer was dried with anhydrous sodium sulfate, and filtered. Then, the filtrate was concentrated under reduced pressure, the residue was purified with silica gel column chromatography (chloroform/methanol = 99/1 to 90/10) to give trans-1-(tert-butoxycarbonyl)-4-phenylpiperidine-3-calboxylic acid (196 mg) as an amorphous product.

MS m/z 180 [M-Boc], 302 [M+Na]

### Reference Example 73

### 1-(tert-butoxycarbonyl)-4-phenyl-1,2,5,6-tetrahydropyridine-3-c alboxylic acid

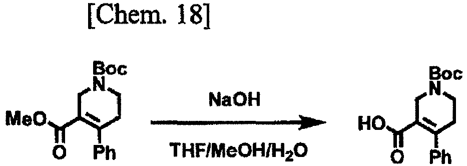

To a methanol solution (5 mL) of 1-tert-butyl 3-methyl 4-phenyl-5,6-dihydropyridine-1,3(2H)-dicarboxylate (429 mg), a 2 mol/L sodium hydroxide aqueous solution (5 mL) and THF (5 mL) were added, and the reaction mixture was stirred for 40 minutes at 70°C. After cooled, the reaction solution was neutralized by adding 2 mol/L hydrochloric acid (15 mL) and water (20 mL). The product was extracted with dichloromethane/methanol (50 mL/5 mL), and the obtained organic layer was dried with anhydrous sodium sulfate, and filtered. Then, the filtrate was concentrated under reduced pressure, the residue was purified with silica gel column chromatography (chloroform/methanol = 100/0 to 93/7) to give 1-(tert-butoxycarbonyl)-4-phenyl-1,2,5,6-tetrahydropyridine-3-carboxyl ic acid (321 mg) as a solid.

¹H NMR (300 MHz, CDCl₃) δ 1.41 (s, 9H), 2.37-2.46 (m, 2H), 3.46-3.54 (m, 2H), 4.10-4.18 (m, 2H), 7.01-7.08 (m, 2H), 7.17-7.27 (m, 3H)

### Reference Example 74

### cis-1-(tert-butoxycarbonyl)-4-phenylpiperidine-3-calboxylic acid

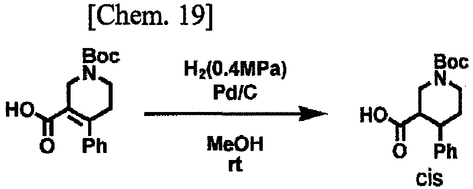

To a methanol solution (5 mL) of 1-(tert-butoxycarbonyl)-4-phenyl-1,2,5,6-tetrahydropyridine-3-calboxyl ic acid (39 mg), palladium/carbon (100 mg) was added, and the reaction mixture was stirred for 28 hours at room temperature under a medium pressure-hydrogen atmosphere (0.40 MPa). The reaction mixture was filtered with Celite. Then, the filtrate was concentrated under reduced pressure to give cis-1-(tert-butoxycarbonyl)-4-phenylpiperidine-3-calboxylic acid (39 mg) as an amorphous product.

¹H NMR (300 MHz, CDCl₃) δ 1.38 (s, 9H), 1.50-1.85 (m, 2H), 2.35-3.20 (m, 4H), 4.00-4.20 (d, 1H), 4.20-4.40 (d, 1H), 7.00-7.30 (m, 5H)

### Reference Example 75

### ethyl 2-phenylnicotinate

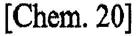

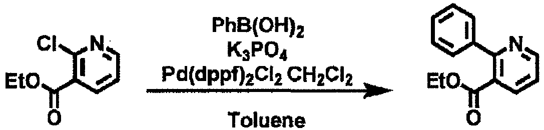

A toluene solution (300 mL) of ethyl 2-chloronictinate (5.57 g), phenylboronic acid (4.76 g), tripotassium phosphate (8.91 g), 1,1'-bis (diphenylphosphino) ferrocene-palladiumdichloride dichloromethane complex (439 mg) was stirred under reflux for 4 hours in a nitrogen atmosphere. Water (100 mL) was added to the reaction solution, and the reaction solution was separated. The obtained organic layer was dried with anhydrous sodium sulfate , filtered, and the filtrate was concentrated under reduced pressure. The obtained residue was purified with silica gel column chromatography (hexane/ethyl acetate = 96/4 to 75/25) to give ethyl 2-phenylnicotinate (5.27 g) as an oil.

MS m/z 229 [M+H]

### Reference Example 76

### 1-(tert-butoxycarbonyl)-2-phenylpiperidine-3-calboxylic acid

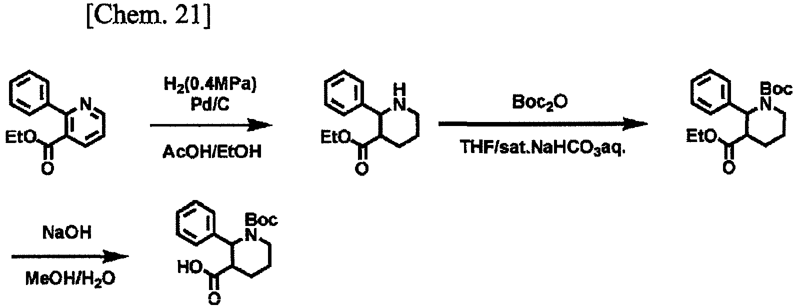

### [Step 1]

To an ethanol solution (30 mL) of ethyl 2-phenylnicotinate (3.41 g), palladium/carbon (1.30 g) and acetic acid (24 mL) were added, and the reaction mixture was stirred for 30 hours at room temperature under a medium pressure-hydrogen atmosphere (about 0.40 MPa). The mixture was filtered with Celite. Then the filtrate was concentrated under reduced pressure to give ethyl 2-phenylpiperidine-3-carboxylate as an oil.

### [Step 2]

To a THF solution (15 mL) of ethyl 2-phenylpiperidine-3-carboxylate obtained in Step 1, saturated sodium hydroxide aqueous solution (30 mL) was added. To the mixture, a THF solution (15 mL) of Boc₂O (3.60 g) was dropped. The reaction solution was heavily stirred for 2 hours at room temperature, and the solution was separated. The obtained organic layer was washed with saturated sodium chloride aqueous solution, dried with anhydrous sodium sulfate. The mixed solution was filtered, and the filtrate was concentrated under reduced pressure. The obtained residue was purified with silica gel column chromatography (Rf, 0.24; hexane/ethyl acetate = 5/95) to give 1-tert-butyl 3-ethyl 2-phenylpiperidine-1,3-dicarboxylate (2.97 g).

### [Step 3]

To a methanol solution (15 mL) of 1-tert-butyl 3-ethyl 2-phenylpiperidine-1,3-dicarboxylate (2.97 g), a 2 mol/L sodium hydroxide aqueous solution (15 mL) was added, and the reaction mixture was stirred for 20 minutes at 70°C. The reaction mixture was concentrated under reduced pressure, then, neutralized by 2 mol/L hydrochloric acid (20 mL). The product was extracted with chloroform (90 mL), and the obtained organic layer was dried with anhydrous sodium sulfate, and filtered. Then, the filtrate was concentrated under reduced pressure to give 1-(tert-butoxycarbonyl)-2-phenylpiperidine-3-carboxylic acid (2.76 g) as an amorphous product.

MS m/z 307 [M+H]

### Reference Example 77

### 1-(tert-butoxycarbonyl)-5-hydroxypiperidine-3-calboxylic acid

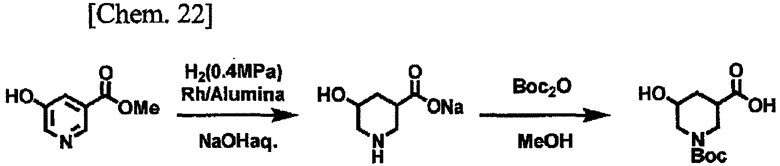

Methyl 3-hydroxynicotinate (1.51 g) was dissolved in a mixture of water (40 mL) and 6 mol/L sodium hydroxide aqueous solution (5 mL) by sonication. After adding of rhodium (0.75 g; supported by 5% weight alumina), the reaction mixture was stirred for 24 hours at room temperature under a medium pressure-hydrogen atmosphere (0.4 MPa). The reaction solution was filtered with Celite, and the filtrate was concentrated under reduced pressure to give sodium 5-hydroxypiperidine-3-carboxylate as a white solid. In a 200mL recovery flask, the sodium 5-hydroxypiperidine-3-carboxylate described above was suspended into methanol (10 mL). To the mixture, a methanol solution (5 mL) of Boc₂O (2.18 g) was added while stirring. After stirried for 1.5 hours at room temperature, the reaction solution was neutralized with 0.2 mol/L hydrochloric acid (20 mL). After extraction with ethyl acetate (150 mL), the organic layer was dried with anhydrous sodium sulfate, and filtered. The filtrate was concentrated under reduced pressure, the obtained residue was purified with silica gel column chromatography (chloroform/methanol = 96/4 to 88/12) to give 1-(tert-butoxycarbonyl)-5-hydroxypiperidine-3-calboxylic acid (794 mg) as a solid.

¹H NMR (300 MHz, CDCl₃) δ 1.46 (s, 9H), 1.70-1.90 (m, 1H), 2.15-2.30 (m, 1H), 2.55-2.70 (m, 1H), 3.00-3.15 (m, 1H), 3.35 (br, 1H), 3.60-3.95 (m, 4H)

MS m/z 244 [M-H]

### Reference Example 78

### 1-(tert-butoxycarbonyl)-5-fluoropiperidine-3-calboxylic acid

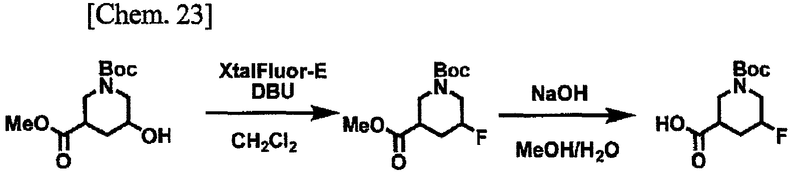

### [Step 1]

To a dichloromethane solution (3 mL) of 1-(tert-butoxycarbonyl)-5-hydroxypiperidine-3-calboxylic acid (259 mg), DBU (228 mg) and XTalFluor-E (344 mg) were added at -78°C while stirring in an argon atmosphere. After stirred for 30 minutes, the reaction mixture was warmed to room temperature, and stirred for 24 hours. To the reaction mixture, dichloromethane (30 mL) was added, and the reaction solution was washed with a saturated aqueous solution of sodium hydrogen carbonate (50 mL) and 0.2 mol/L hydrochloric acid (50 mL). The obtained organic layer was dried with anhydrous sodium sulfate, and filtered. The filtrate was concentrated under reduced pressure, the obtained residue was purified with silica gel column chromatography (hexane/ethyl acetate = 97/3 to 76/24; Rf = 0.59) to give 1-tert-butyl 3-methyl 5-fluoropiperidine-1,3-dicarboxylate (80 mg).

### [Step 2]

To a methanol solution (1 mL) of 1-tert-butyl 3-methyl 5-fluoropiperidine-1,3-dicarboxylate (80 mg), a 2 mol/L sodium hydroxide aqueous solution (1 mL) was added and stirred for 6 hours at 70°C. The reaction solution was neutralized by adding 2 mol/L hydrochloric acid (5 mL). The product was extracted with dichloromethane (50 mL). The obtained organic layer was dried with anhydrous sodium sulfate, filtered, then the filtrate was concentrated under reduced pressure to give 1-(tert-butoxycarbonyl)-5-fluoropiperidine-3-carboxylic acid (65 mg) as a solid.

### Reference Example 79

### 1-tert-butyl 3-methyl 5-oxopiperidine-1,3-dicarboxylate

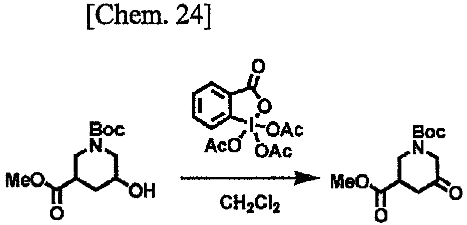

To a dichloromethane solution (25 mL) of 1-tert-butyl 3-methyl 5-hydroxypiperidine-1,3-dicarboxylate (1.30 g), Dess-Martin reagent (2.33 g) was added. After stirring for 3 hours at room temperature, Dess-Martin reagent (1.31 g) was further added to the reaction solution, and the mixture was stirred for further 2.5 hours. Then, saturated aqueous solution of sodium hydrogen carbonate (50 mL) and a 10% sodium thiosulfate aqueous solution (50 mL) were added sequentially to the mixture, and the organic layer was separated. The product was further extracted from the aqueous layer with dichloromethane (10 mL) four times. The obtained organic layer was dried with anhydrous sodium sulfate, filtered. The filtrate was concentrated under reduced pressure, the residue was purified with silica gel column chromatography (chloroform/methanol = 100/0 to 98/2) to give 1-tert-butyl 3-methyl 5-oxopiperidine-1,3-dicarboxylate (1.14 g) as an oil.

¹H NMR (300 MHz, CDCl₃) δ 1.46 (s, 9H), 2.58-2.79 (m, 2H), 3.03-3.11 (m, 1H), 3.73 (s, 3H), 3.77-3.93 (m, 2H), 4.02 (s, 2H)

### Reference Example 80

### 1-tert-butyl 3-methyl 5,5-difluoropiperidine-1,3-dicarboxylate

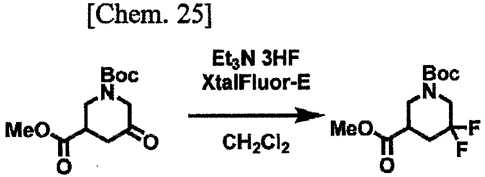

To a dichloroethane solution (6 mL) of triethylamine hydrogen trifluoride complex (326 µL) and XtalFluor-E (687 mg), a dichloroethane solution (1 ml) of 1-tert-butyl 3-methyl 5-oxopiperidine-1,3-dicarboxylate (515 mg) was added, and the reaction mixture was stirred under reflux for 2.5 hours. After cooling the reaction solution to room temperature, a saturated aqueous solution of sodium hydrogen carbonate (30 mL) was added to the reaction solution. The product was extracted with dichloromethane (20 mL) three times, and the organic layer was dried with anhydrous sodium sulfate, and filtered. Then, the filtrate was concentrated under reduced pressure, the residue was purified with silica gel column chromatography (chloroform/methanol = 100/0 to 93/7) to give 1-tert-butyl 3-methyl 5,5-difluoropiperidine-1,3-dicarboxylate (326 mg) as an oil.

MS m/z 180 [M-Boc], 302 [M+Na]

### Reference Example 81

### 1-(terf-butoxycarbonyl)-5,5-difluoropiperidine-3-calboxylic acid

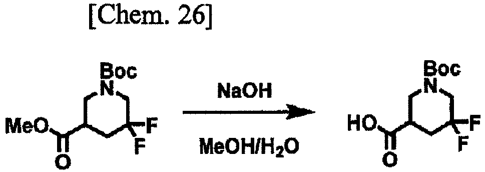

To a methanol solution (6 mL) of 1-tert-butyl 3-methyl 5,5-difluoropiperidine-1,3-dicarboxylate (326 mg), a 2 mol/L sodium hydroxide aqueous solution (2 mL) was added, and the reaction mixture was stirred for 2 hours at 70°C. The reaction solution was neutralized with 2 mol/L hydrochloric acid (10 mL), and the product was extracted with dichloromethane (50 mL). The product was further extracted with dichloromethane (10 mL) twice, the obtained organic layer was dried with anhydrous sodium sulfate, and filtered. Then, the filtrate was concentrated under reduced pressure, the residue was purified with silica gel column chromatography (chloroform/methanol = 99/1 to 91/9) to give 1-(tert-butoxycarbonyl)-5,5-difluoropiperidine-3-carboxylic acid (263 mg) as a solid.

¹H NMR (300 MHz, CDCl₃) δ 1.48 (s, 9H), 1.90-2.20 (m, 1H), 2.40-2.60 (m, 1H), 2.70-3.15 (m, 3H), 4.10-4.60 (m, 2H)

### Reference Example 82

### 1-(tert-butoxycarbonyl)-5-methoxypiperidine-3-calboxylic acid

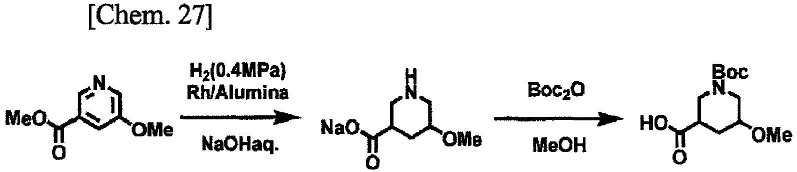

Methyl 3-hydroxynicotinate (1.00 g) was dissolved in a mixture of water (20 mL) and 6 mol/L sodium hydroxide aqueous solution (3 mL) by sonication. To the reaction solution, rhodium (0.44 g; supported by 5% weight alumina) was added, and the mixture was stirred for 3 days at room temperature under a medium pressure-hydrogen atmosphere (0.4 MPa). The reaction solution was filtered with Celite, the filtrate was concentrated under reduced pressure to give sodium 5-methoxypiperidine-3-carboxylate as a white solid. The obtained sodium 5-methoxypiperidine-3-carboxylate was suspended into methanol (20 mL). To the solution, a methanol solution (4 mL) of Boc₂O (1.31 g) was dropped while stirring. The reaction solution was stirred for 6 hours at room temperature, and Boc₂O (1.31 g) was added, and the reaction solution was further stirred for 3 hours. To the reaction solution, 2 mol/L hydrochloric acid (50 mL) was added, and the reaction solution was separated by adding dichloromethane (50 mL). After further extraction from aqueous layer with dichloromethane (30 mL) twice, the combined organic layer was dried with anhydrous sodium sulfate, and filtered. The filtrate was concentrated under reduced pressure, the obtained residue was purified with silica gel column chromatography (chloroform/methanol = 99/1 to 91/9) to give 1-(tert-butoxycarbonyl)-5-methoxypiperidine-3-calboxylic acid (1.26 g) as a solid.

MS m/z 260.2 [M+H]

### Reference Example 83

### cis-1-(tert-butoxycarbonyl)-2-methylpiperidine-3-calboxylic acid

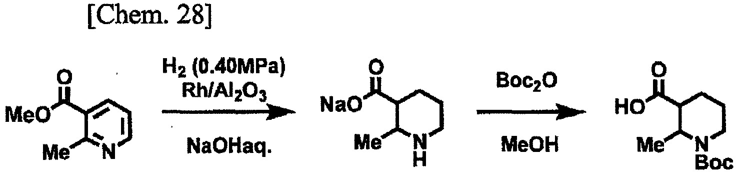

By using methyl 2-methylnicotinate (1.51 g), according to the similar method as Reference Example 82, cis-1-(tert-butoxycarbonyl)-2-methylpiperidine-3-calboxylic acid (689 mg) was obtained as a solid.

¹H NMR (300 MHz, CDCl₃) δ 1.10 (d, 1H), 1.30-1.56 (m, 1H), 1.47 (s, 9H), 1.60-1.95 (m, 3H), 2.60-2.90 (m, 2H), 3.96 (br, 1H), 4.55-5.00 (br, 1H)

### Reference Example 84

### 1-tert-butyl 3-ethyl-cis-2-methylpiperidine-1,3-dicarboxylate

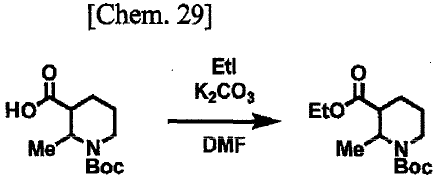

To a DMF mixture (5 mL) of cis-1-(tert-butoxycarbonyl)-2-methylpiperidine-3-calboxylic acid (401 mg) and potassium carbonate (683 mg), iodoethane (386 mg) was added, and the reaction solution was stirred for 4 hours at room temperature. To the reaction solution, ethyl acetate (25 mL) and hexane (25 mL) were added, and the organic layer was washed with water (100 mL) three times, dried with anhydrous sodium sulfate, and filtered. Then, the filtrate was concentrated under reduced pressure, the obtained residue was purified with silica gel column chromatography (hexane/ethyl acetate = 100/0 to 79/21) to give 1-tert-butyl 3-ethyl -cis-2-methylpiperidine-1,3-dicarboxylate (389 mg) as a solid.

¹H NMR (300 MHz, CDCl₃) δ 1.04 (d, 3H), 1.26 (t, 3H), 1.40 (m, 1H), 1.47 (s, 9H), 1.64-1.90 (m, 3H), 2.61 (dt, 1H), 2.68-2.95 (m, 1H), 3.79-4.05 (brd, 1H), 4.15 (q, 2H), 4.57-4.93 (brd, 1H)

### Reference Example 85

### 1-tert-butyl 3-ethyl-trans-2-methylpiperidine-1,3-dicarboxylate

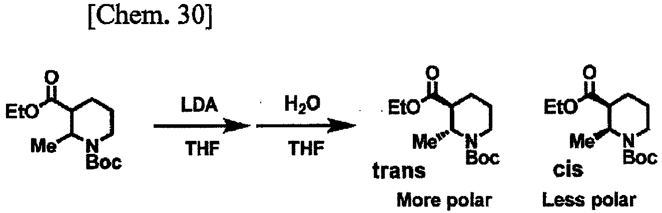

To a THF (10 mL) solution of diisopropylamine (174 mg) was cooled to 0°C under an argon atmosphere while stirring, a 2.69 mol/L n-butyllithium/hexane solution was dropped. The reaction solution was stirred for 10 minutes at 0°C, and then cooled to -78°C. To the reaction solution, a THF solution (4 mL) of 1-tert-butyl 3-ethyl-cis-2-methylpiperidine-1,3-dicarboxylate (389 mg) was dropped, and the reaction mixture was stirred for an hour. Then, the reaction was quenched by adding water (5 mL), the reaction mixture was neutralized by adding saturated aqueous solution of ammonium chloride at room temperature. Then, the product was extracted from the neutralized reaction solution with dichloromethane (70 mL), the organic layer was dried with anhydrous sodium sulfate, and filtered. Then, the filtrate was concentrated under reduced pressure, and the residue was purified with silica gel column chromatography (hexane/ethyl acetate = 4/96 to 20/80) to give 1-tert-butyl 3-ethyl -trans-2-methylpiperidine-1,3-dicarboxylate (100 mg) as an amorphous product.

¹HNMR (300 MHz, CDCl₃) δ 1.22 (d, 3H), 1.26 (t, 3H), 1.48 (s, 9H), 1.40-1.55 (m, 1H), 1.61-1.80 (m, 2H), 2.00-2.10 (m, 1H), 2.39 (bs, 1H),2.82 (td, 1H), 3.94 (bd, 1H), 4.10-4.20 (m, 2H), 4.86 (q, 1H)

### Reference Example 86

### trans-1-(tert-butoxycarbonyl)-2-methylpiperidine-3-calboxylic acid

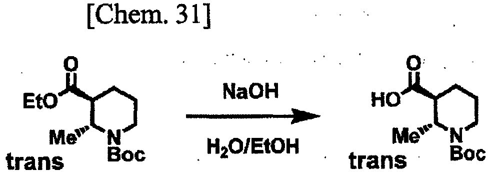

To an ethanol solution (3 mL) of 1-tert-butyl 3-ethyl-trans--2-methylpiperidine-1,3-dicarboxylate (100 mg), a 2 mol/L sodium hydroxide aqueous solution (1mL) was added and stirred for an hour at 70°C. The reaction mixture was cooled to room temperature, and neutralized by adding 2 mol/L hydrochloric acid (5 mL). Then, the product was extracted with dichloromethane/methanol (30 mL/3 mL) from the neutralized reaction solution. After further extraction with dichloromethane (20 mL), the combined organic layer was dried with anhydrous sodium sulfate, and filtered. Then the filtrate was concentrated under reduced pressure to give trans-1-(tert-butoxycarbonyl)-2-methylpiperidine-3-carboxylic acid (93 mg) as a solid.

¹H NMR (300 MHz, CDCl₃) δ 1.16 (d, 1H), 1.37 (s, 9H), 1.30-1.85 (m, 3H), 2.00 (d, 1H), 2.37 (bs, 1H), 2.75 (td, 1H), 3.89 (d, 1H), 4.81 (q,1H), 9.50 (b, 1H)

### Reference Example 87

### 1-(tert-butoxycarbonyl)-5-methylpiperidine-3-calboxylic acid

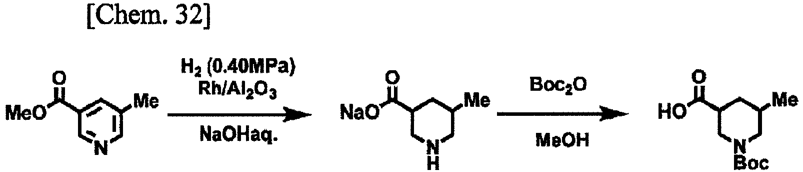

By using methyl 3-methylnicotinate (1.51 g), according to the similar method as Reference Example 82, 1-(tert-butoxycarbonyl)-5-methylpiperidine-3-calboxylic acid (961 mg) was obtained as a solid.

### Reference Example 88

### 1-tert-butyl 3-methyl-cis-6-methylpiperidine-1,3-dicarboxylate

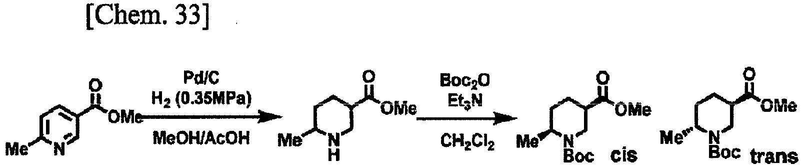

### [Step 1]

To a methanol/acetic acid solution (40 mL/50 mL) of methyl 2-metylnicotinate (5.00 g), palladium/carbon (2.50 g) was added, and the reaction mixture was stirred for 20 hours at room temperature under a medium pressure-hydrogen atmosphere (0.35 MPa). The reaction solution was filtered with Celite, the filtrate was concentrated under reduced pressure. To the obtained residue, a saturated sodium hydrogen carbonate aqueous solution (150 mL) and a 0.2 mol/L sodium hydroxide aqueous solution (50 mL) were added. The product was extracted with dichloromethane (150 mL), and chloroform/methanol (90 mL)/10 mL). The organic layer was dried with anhydrous sodium sulfate, and filtered. Then, the filtrate was concentrated under reduced pressure to give methyl 6-methylpiperidine-3-carboxylate (3.90 g).

### [Step 2]

To a dichloromethane solution (40 mL) of methyl 6-methylpiperidine-3-carboxylate (3.90 g) obtained in Step 1, triethylamine (2.64 g) was added, then, a dichloromethane solution (5 mL) of Boc₂O (5.68 g) was added while stirring at room temperature. After stirred for 70 hours at room temperature, the reaction mixture was washed with 1 mol/L hydrochloric acid (100 mL). The organic layer was dried with anhydrous sodium sulfate, and filtered. The filtrate was concentrated under reduced pressure, the residue was purified with silica gel column chromatography ( hexane/ethyl acetate = 100/0 to 13/87) to give 1-tert-butyl 3-methyl-cis-6-methylpiperidine-1,3-dicarboxylate (2.66 g, low polarity) and 1-tert-butyl 3-methyl-trans-6-methylpiperidine-1,3-dicarboxylate (1.39 g, high polarity) respectively as an oil.

cis-isomer, ¹H NMR (300 MHz, CDCl₃) δ 1.13 (d, 3H), 1.46 (s, 9H), 1.50-1.95 (m, 4H), 2.30-2.45 (m, 1H), 2.80-3.00 (m, 1H), 3.69 (s, 3H), 4.15 (br, 1H), 4.40 (br, 1H);

trans-isomer, ¹H NMR (300 MHz, CDCl₃) δ 1.14 (d, 3H), 1.30-1.40 (m, 1H), 1.46 (s, 9H), 1.72-1.96 (m, 2H), 1.96-2.06 (br, 1H), 2.58 (br, 1H), 3.04-3.10 (m, 1H), 3.68 (s, 3H), 4.30-4.45 (m, 2H)

### Reference Example 89

### cis-1-(tert-butoxycarbonyl)-6-methylpiperidine-3-calboxylic acid

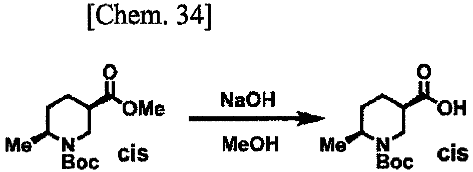

To a methanol solution (20 mL) of -1-tert-butyl 3-methyl-cis-6-methylpiperidine-1,3-dicarboxylate (2.57 g), a 2 mol/L sodium hydroxide aqueous solution (10 mL) was added, and the reaction mixture was stirred for 24 hours at room temperature. The reaction solution was neutralized by adding water (50 mL) and 2 mol/L hydrochloric acid (15 mL)and a product was extracted with dichloromethane (70 mL), the organic layer was dried with anhydrous sodium sulfate, and filtered. The filtrate was concentrated under reduced pressure to give cis-1(tert-butoxycarbonyl)-6-methylpiperidine-3-carboxylic acid (2.32 g) as a solid.

¹H NMR (300 MHz, CDCl₃) δ 1.14 (d, 3H), 1.33-1.50 (m, 1H), 1.45 (s, 9H), 1.80-2.04 (m, 3H), 2.62 (s, 1H), 3.07 (dd, 1H), 4.30-4.45 (m,2H)

### Reference Example 90

### trans-1-(tert-butoxycarbonyl)-6-methylpiperidine-3-calboxylic acid

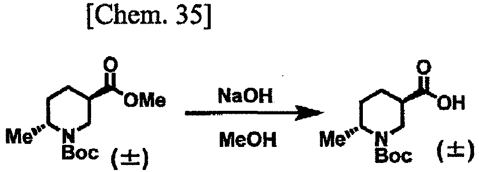

By using 1-tert-butyl 3-methyl-trans-6-methylpiperidine-1,3-dicarboxylate (1.29 g), according to the similar method as Reference Example 89, trans-1-(tert-butoxycarbonyl)-6-methylpiperidine-3-calboxylic acid (1.07 g) was obtained as a solid.

¹H NMR (300 MHz, CDCl₃) δ 1.14 (d, 1H), 1.45 (s, 9H), 1.56-1.85 (m, 3H), 1.90-2.00 (m, 1H), 2.32-2.50 (m, 1H), 2.91 (t, 1H), 4.17 (d, 1H), 4.41 (br, 1H), 8.55 (br, 1H)

### Reference Example 91

### methyl 6-hydroxymethyl-1-methylpiperidine-3-calboxylic acid

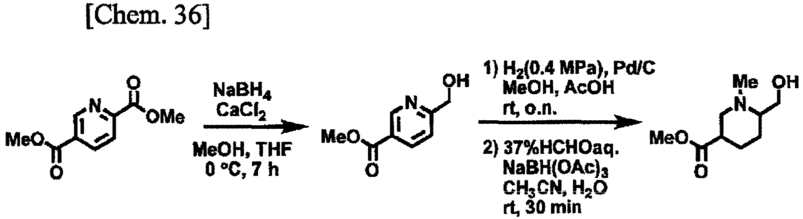

### [Step 1]

To a methanol (68 mL)-THF (34 mL) solution of methyl 6-methoxycarbonylnicotinate (3.00 g, 15.4 mmol), potassium chloride (6.82 g, 61.5 mmol) was added. Then, the reaction solution was cooled to 0°C, and sodium borohydride (1.46 g, 38.4 mmol) was added. After stirred for 7 hours at 0°C, water (100 mL) was added to the reaction solution, and the product was extracted with chloroform (100 mL) three times. The obtained organic layer was washed with water (100 ml) three times, and dried with sodium sulfate. The solvent was removed under reduced pressure to give a crude crystal of methyl 6-hydroxymethylnicotinate (2.01 g; crude yield, 78%). The product was used for the next reaction without further purification.

### [Step 2]

To a methanol (16 mL)-acetic acid (20 mL) solution of methyl 6-hydroxymethylnicotinate (1.16 g, 6.94 mmol), 10% palladium/carbon (580 mg) was added. Then, the reaction solution was stirred for 12 hours at room temperature under a 4 atmospheres (0.4 MPa)-hydrogen atmosphere, the reaction solution was filtered with Celite. The solvents of the filtrate were removed under reduced pressure to give desired piperidine derivative. The product was used for the following reaction without further purification. In a 200 mL recovery flask, to an acetonitrile (46 mL) solution of the piperidine derivative obtained in the reaction described above, a 37% formalin aqueous solution (1.24 mL) and sodium triactoborohydride (3.97 g, 18.7 mmol) were added. The reaction mixture was stirred for 30 minutes at room temperature. After adding 20% potassium carbonate aqueous solution 20 mL, the product was extracted with chloroform/methanol solution (9/1, 50 mL) six times. The combined organic layer was dried with sodium sulfate, and the solvents were removed under reduced pressure. The residue was purified with aminosilica gel column chromatography (chloroform/methanol = 9/1) to give crystalline methyl 6-hydroxymethyl-1-methypiperidine-3-carboxylate (1.02 g; total yield of two steps, 79%).

LCMS (M+H = 188)

### Reference Example 92

### methyl trans-6-(tert-butyldimethyksiloxy)methyl-1-methylpiperidine-3-calboxy late

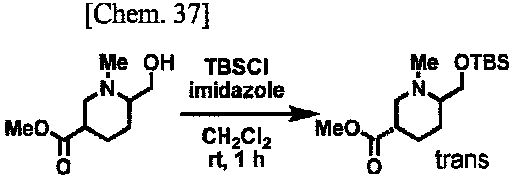

To a dichloromethane (8 mL) solution of methyl 6-hydroxymethyl-1-methylpiperidine-3-carboxylate (300 mg, 1.60 mmol), tert-butyldimethylsilyl chloride (481 mg, 3.20 mmol) and imidazole (436 mg, 6.41 mmol) were added, and the reaction mixture was stirred for an hour at room temperature. The reaction solution was washed with water (10 mL), and the product was extracted from the aqueous layer with dichloromethane (10 mL) five times. The combined organic layer was dried with sodium sulfate, and concentrated under reduced pressure. The residue was purified with silica gel column chromatography (chloroform/methanol = 95/5) to give oily methyl trans-6-(tert-butyldimethyksiloxy)methyl-1-methylpiperidine-3-calboxy late (185 mg; yield, 38%).

LCMS (M+H = 302)

### Example 1

### (3S)-N-[2-(2-fluorophenyl)propane-2-yl]-1-methylpiperidine-3-carboxyamide

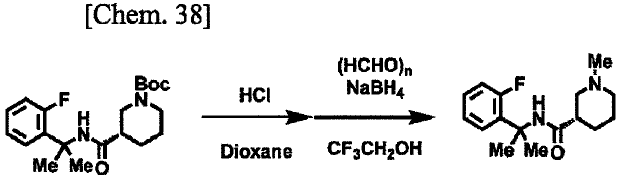

To tert-butyl (3S)-3-{[2-(2-fluorophenyl)propane-2-yl]carbamoyl}piperidine-1-carbo xylate (182 mg) obtained in Reference Example 64, 4 mol/L hydrogen chloride/1,4-dioxane solution (3 mL) was added, and the reaction mixture was stirred for an hour at room temperature. The reaction mixture was concentrated under reduced pressure, and the residue was dissolved in 2,2,2-trifluoroethanol (3 mL). To the obtained reaction mixture, paraformaldehyde (150 mg) and sodium borohydride (95 mg) were added sequentially, and the reaction mixture was stirred for 3 hours at 80°C. The reaction mixture was filtrated with Celite, and concentrated under reduced pressure. The obtained residue was purified with aminosilica gel column chromatography (hexane/chloroform = 79/21 to 0/100) to give (3S)-N-[2-(2-fluorophenyl)propane-2-yl]-1-methylpiperidine-3-carboxy amide (95 mg) as a solid.

### Examples 2 to 108

After doing the reaction of Reference Example 64 by using the corresponding starting carboxylic acid compounds (commercialized products or synthesized compounds in Reference Examples 65 to 90) and the corresponding starting amine compounds (commercialized products or synthesized compounds in Reference Examples 2 to 63), the reaction and treatment were performed with the similar method as Example 1 to give compounds of Examples 2 to 108 shown in Tables 4 to 21.

### Example 109

### trans-6-hydroxymethyl-1-methyl-N-[2-(naphthalene-1-yl)propan e-2-yl]piperidine-3-calboxamide

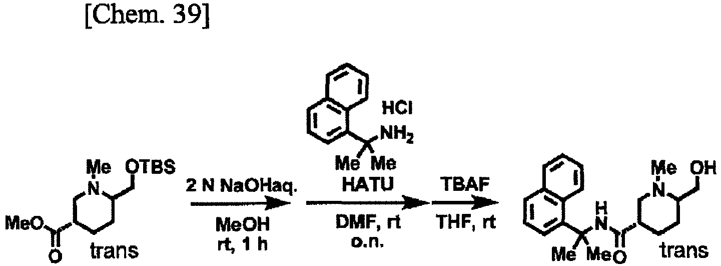

To a methanol (0.3 mL) solution of methyl trans-6-(tert-butyldimethyksiloxy)methyl-1-methylpiperidine-3-calboxy late (30 mg, 0.10 mmol) obtained in Reference Example 92, a 2 mol/L sodium hydroxide aqueous solution (0.055 mL) was added, and the reaction mixture was stirred for 24 hours at room temperature. The solvents were removed under reduced pressure to give a carboxylic acid derivative. The derivative was used for the following reaction without further purification.

To a dimethylformamide (0.5 mL) solution of the carboxylic acid derivative obtained in the above reaction, 2-(naphthalene-1-yl)propane-2-amine hydrochloride (22.2 mg, 0.10 mmol) and HATU (57.0 mg, 0.15 mmol) were added, and the reaction mixture was stirred for 12 hours at room temperature. After adding a 0.1 mol/L sodium hydroxide aqueous solution (10 mL) to the reaction solution, the product was extracted with methylene chloride (10 mL) five times. The combined organic layer was dried with sodium sulfate, and its solvents were removed under reduced pressure. The obtained residue was roughly purified with aminosilica gel column chromatography (hexane/ethyl acetate = 1/9), and directly used for the following reaction without further purification.

To a THF (0.3 mL) solution of the silyl ether derivative obtained in the above reaction, a THF solution (32 µL) of 1 mol/L TBAF was added. After stirring for 4 hours at room temperature, the solvent was removed under reduced pressure. The residue was purified with aminosilica gel column chromatography (chloroform/methanol = 95/5) to give colorless, amorphous trans-6-hydroxymethyl-1-methyl-N-[2-(naphthalene-1-yl)propane-2-yl] piperidine-3-calboxamide (15 mg; yield of 3 steps, 44%).

### Example 110

### (3S)-N-[2-(2-chlorophenyl)propane-2-yl]-1-ethylpiperidine-3-ca rboxyamide

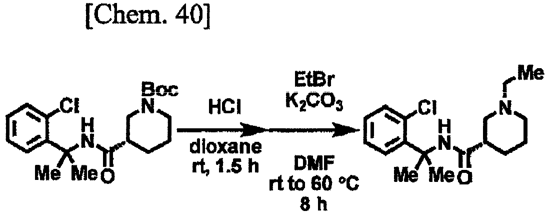

To tert-butyl (3 S)-3-{[2-(2-chlorophenyl)propane-2-yl)carbamoyl}piperidine-1-carbo xylate (4.83 g), a 4 mol/L hydrogen chloride/1,4-dioxane solution (100 mL) was added. After stirred for 1.5 hours at room temperature, the reaction mixture was concentrated under reduced pressure to give crude crystals of (3S)-N-[2-(2-chlorophenyl)propane-2-yl]-piperidine-3-carboxyamide quantitatively. The product was used for the following reaction without further purification.

To a dimethylformamide (0.7 mL) solution of (3 S)-N-[2-(2-chlorophenyl)propane-2-yl]-piperidine-3-carboxyamide (100 mg, 0.36 mmol), potassium carbonate (52 mg, 0.37 mmol) and ethyl bromide (78 mg, 0.71 mmol) were added. After stirred for 5 hours at room temperature, the reaction solution was heated to 60°C, and stirred for 3 hours. To the reaction solution, water (5 mL) was added, and the product was extracted with a mixed solution of ethyl acetate (4 mL) and hexane (2 mL) twice. The combined organic layer was dried with sodium sulfate, and concentrated under reduced pressure. The obtained residue was purified with aminosilica gel column chromatography (hexane/chloroform = 1/1) to give white crystalline (3 S)-N-[2-(2-chlorophenyl)propane-2-yl]-1-ethylpiperidine-3-carboxya mide (76 mg; yield, 69%).

### Examples 111 to 122

After doing the reaction of Reference Example 64, the reaction and treatment were performed with the similar method as Example 110 by using the corresponding starting carboxylic acid compounds (commercialized products) and the corresponding starting amine compounds (commercialized products) to give compounds of Examples 111 to 122 shown in Tables 22 to 24.

**[Table 4]**

| Example | Structural formula | ¹H-NMRδ: (LC-MS : [M+H]⁺ / Rt / Measuring condition) |
|---|---|---|
| 1 | 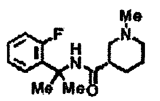 | ¹H NMR (300 MHz, CDCl₃)δ1.45-1.88 (m, 4H), 1.72 (s, 3H), 1 .76 (s, 3H), 2.04-2.82 (m, 5H), 2.25 (s, 3H), 6.90-7.03 (m, ¹H ), 7.03-7.14 (m, 1H), 7.14-7.24 (m, 1H), 7.31-7.42 (m, 1H). (LC-MS : 279.6 / 0.650 / B) |
| 2 | 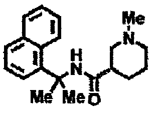 | ¹H NMR (300 MHz, CDCl₃)δ1,41-1.61 (m, 2H), 1.61-1.85 (m, 2H), 1.85-2.73 (m, 5H), 1.96 (s, 6H), 2.02 (s, 3H), 7.40-7.48 ( m, 3H), 7.61 (d, J = 6.8 Hz, 1H), 7.76 (d, *J* = 8.3 Hz), 7.8 6-7.87 (m, 1H), 8.49-8.51 (m, 1H). (LC-MS : 311.3 / 0.521 / B) |
| 3 | 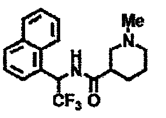 | ¹H NMR (400 MHz, CDCl₃)δ1.18 (brs, 0.34H), 1.32 (brs, 0.67 H), 1.45-1.62 (m, 2H), 1.72-1.88 (m, 1H),2.03-2.21 (m, 2H). 2.24 (s, 2H), 2.29 (s, 1H), 2.49-2.53 (m, 0.66H), 256-2.60 (m 0.34H), 2.79-2.91 (m, 2H), 6.51-6.60 (m, 1H), 7.41-7.55 (m, 4H), 7.82 (d, J = 8.0 Hz, 2H), 8.11 (d, J = 8.5 Hz, 1H). |
| 4 | 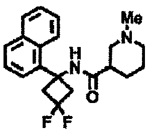 | ¹H NMR (400 MHz, CDCl₃)δ1.33 (brs, 2H), 1.44 (brs, 1H), 1.6 3 (brs, 1H), 1.97 (brs, 1H), 2.18 (s. 3H), 2.25 (brs, 1H), 2.34 (brs, 1H), 2.60 (brs, 2H), 3.24-3.38 (m, 2H), 3.41-3.52 (m, 2 H), 7.37-7.43 (m, 3H), 7.61 (d, J = 7.1 Hz, 1H), 7.70-7.74 ( m, 2H). 7.80-7.84 (m, 1H), 9.66 (brs, 1H). |
| 5 | 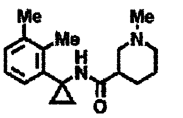 | ¹H NMR (300 MHz, CDCl₃)δ1.04-1.27 (m, 4H), 1.37-1.57 (m, 3H), 1.73 (bs, 1H), 1.90 (bs, 1H),2.03-2.27 (m, 2H), 2.24 (s, 3H). 2.27 (s, 3H), 2.38 (s. 3H), 2.50-2.75 (m, 2H), 7.01-7.11 (m, 2H), 7.46-7.55 (m, 1H) |
| 6 | 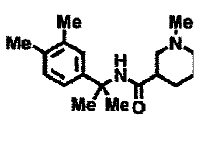 | ¹H NMR (300 MHz, CDCl₃)δ1.48-1.57 (m, 2 H), 1.60 (s, 3 H), 1.63 (s, 3 H), 1.66-1.87 (m, 2 H), 2.26 (s, 3 H), 2.28-2.41 ( m, 2 H). 2.66-2.74 (m, 2 H), 7.18 (t, *J* = 1.8 Hz, 1 H), 7.21 (d, J = 1.8 Hz, 2 H) |
| 7 | 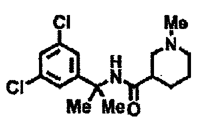 | ¹H NMR (300 MHz, CDCl₃)δ1.49-1.58 (m, 2H), 1.60 (s, 3 H), 1.63 (s, 3 H), 1.66-1.88 (m, 2 H), 207-221 (m, 1 H), 2.26 ( s, 3 H), 2.28-2.41 (m, 2 H), 2.64-2.76 (m, 2 H), 7.18 (t, J = 1.8 Hz, 1 H), 7.21 (d, J = 1.8 Hz, 2 H), 8.32 (br, 1 H) |

**[Table 5]**

| Example | Structural formula | ¹H-NMRδ: (LC-MS : [M+H]⁺ / Rt / Measuring condition) |
|---|---|---|
| 8 | 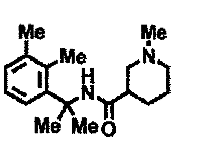 | ¹H NMR (300 MHz, CDCl₃)δ1.46-1.73 (m, 5 H), 1.7 4 (s, 3 H), 1.81 (s, 3 H), 2.18 (s, 3 H), 2.24 (s, 3 H), 2.25-2.36 (m, 2 H). 2.38 (s, 3 H), 2.39-2.62 ( m, 2 H), 7.06 (d, *J* = 4.8 Hz, 2 H), 7.29 (t, *J =* 4.8 Hz, 12 H), 7.61 (br, 1 H) |
| 9 | 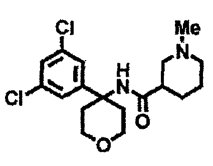 | ¹H NMR (300 MHz, CDCl₃)δ1.45-1.75 (m, 2H), 1.75 -1.95 (m, 2H), 1.95-2.40 (m, 9H), 2.33 (s, 3H), 2.4 0-2.49 (m, 1H), 2.70-3.01 (m, 2H), 3.56-3.81 (m, 2 H), 3.89-3.97 (m, 2H), 7.14-7.29 (m, 3H) |
| 10 | 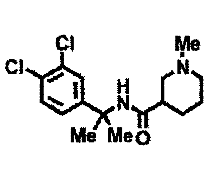 | ¹H NMR (300 MHz, CDCl₃)δ1.49-1.69 (m, 4H), 1.62 (s, 3H), 1.64 (s, 3H), 2.11-2.49 (m, 3H), 2.27 (s, 3H), 2.49-2.70 (m, 2H), 7.20 (dd, J = 8.4 Hz, 2.4 Hz, 1H),7.36 (d, J = 8.4 Hz, 1H), 7.43 (d, *J* = 2.4 Hz, 1H) |
| 11 | 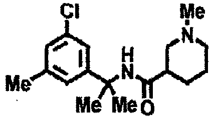 | ¹H NMR (300 MHz, CDCl₃)δ1.47-1.70 (m, 2H), 1.64 (s, 3H), 1.66 (s, 3H), 1.70-1.89 (m, 2H), 2.09-2.45 (m, 3H), 2.26 (s, 3H), 2.32 (s, 3H), 2.45-2.89 (m, 2H), 7.01 (s, 1H), 7.05 (s, 1H), 7.15 (s, 1H) |
| 12 | 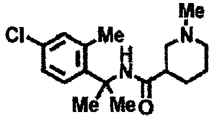 | ¹H NMR (300 MHz, CDCl₃)δ1.47-1.86 (m, 4H), 1.65 (s, 3H), 1.76 (s. 3H), 2.08-2.73 (m, 5H), 2.23 (s, 3H), 2.45 (s, 3H), 7.05-7.17 (m, 2H), 7.32 (d, *J* = 8.3 Hz, 1H) |
| 13 | 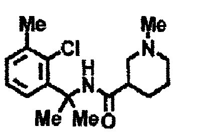 | ¹H NMR (300 MHz, CDCl₃)δ1.46-1.88 (m, 4H), 1.76 (s, 3H), 1.84 (s, 3H), 2.14-2.74 (m, 5H), 2.24 (s, 3H), 2.36 (s, 3H), 7.07-7.17 (m, 2H), 7.33-7.42 (m, 1H) |
| 14 | 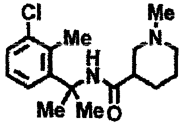 | ¹H NMR (300 MHz, CDCl₃)1,47-1.86 (m, 4H), 1.70 (s, 3H), 1.79 (s, 3H), 2.10-2.74 (m, 5H), 2.22 (s, 3 H), 2.52 (s, 3H), 7.09 (t, *J* = 8.0 Hz, 1H), 7.27 (d *J* = 7.9 Hz, 1H), 7.35 (d, J = 8.1 Hz, 1H) |

**[Table 6]**

| Example | Structural formula | ¹H-NMRδ: (LC-MS : [M+H]⁺ / Rt / Measuring condition) |
|---|---|---|
| 15 | 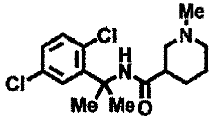 | ¹H NMR (300 MHz, CDCl₃)δ1.47-1.68 (m, 2 H), 1.7 1 (s, 3 H), 1.72-1.78 (m, 2 H), 1.79 (s, 3 H), 2.23 (s, 3 H), 2.26-2.67 (m, 5 H), 7.10 (dd, *J* = 2.4, 8.4 Hz, 1 H), 7.21 (d, *J* = 8.4 Hz, 1 H), 7.46 (d, *J* = 2.4 Hz, 1 H), 7.99 (br, 1 H) |
| 16 | 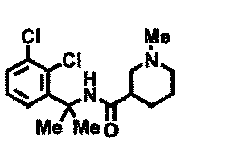 | ¹H NMR (300 MHz, CDCl₃)δ1.50-1.70 (m, 4 H), 1.7 3 (s, 3 H), 1.75-1.81 (m, 1 H), 1.83 (s, 3 H), 2.24 (s, 3 H), 2.29-2.71 (m, 4 H), 7.15 (t, *J* = 8.0 Hz, 1 H), 7.34 (dd, *J* = 8.0, 1.6 Hz, 1 H), 7.43 (dd, *J* = 8.0, 1.6 Hz, 1 H), 8.10 (br, 1 H) |
| 17 | 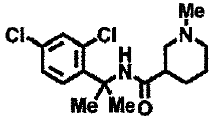 | ¹H NMR (300 MHz, CDCl₃)δ1.50-1.68 (m, 3 H), 1.7 0 (s, 3 H), 1.72-1.78 (m, 2 H), 1.80 (s, 3 H), 2.23 (s, 3 H), 2.59-2.69 (m, 4 H), 7.18 (dd, *J* = 8.6, 2.2 Hz, 1 H), 7.30 (d, *J* = 2.2 Hz, 1 H), 7.42 (d, *J =* 8.6 Hz, 1 H), 8.04 (br, 1 H) |
| 18 | 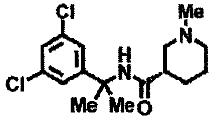 | ¹H NMR (300 MHz, CDCl₃)δ1.41-1.93 (m, 4H), 1.62 (s, 3H), 1.65 (s, 3H), 2.08-2.50 (m, 3H), 2.28 (s, 3 H), 2.50-2.92 (m, 2H), 7.16-7.26 (m, 3H) (LC-MS : 331 / 0.685 / B) |
| 19 | 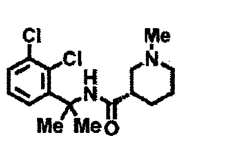 | ¹H NMR (300 MHz, CDCl₃)δ1.50-1.70 (m, 4 H), 1.7 3 (s, 3 H), 1.75-1.81 (m, 1 H), 1.83 (s, 3 H), 2.24 (s, 3 H), 2.29-2.71 (m, 4 H), 7.15 (t, *J* = 8.0 Hz, 1 H), 7.34 (dd, *J* = 8.0, 1.6 Hz, 1 H), 7.43 (dd, *J* = 8.0, 1.6 Hz, 1 H), 8.10 (br, 1 H) |
| 20 | 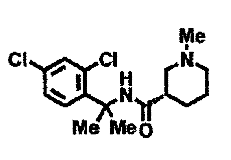 | ¹H NMR (300 MHz, CDCl₃)δ1.50-1.68 (m, 3 H), 1.7 0 (s, 3 H), 1.72-1.78 (m, 2 H), 1.80 (s, 3 H), 2.23 (s, 3 H), 2.59-2.69 (m, 4 H), 7.18 (dd, *J* = 8.6, 2.2 Hz, 1 H), 7.30 (d, *J* = 2.2 Hz, 1 H), 7.42 (d, *J* = 8.6 Hz, 1 H), 8.04 (br, 1 H) |
| 21 | 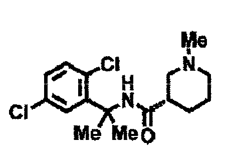 | ¹H NMR (300 MHz, CDCl₃)δ1.49-1.68 (m, 4 H), 1.6 9 (s, 3 H), 1.71-1.77 (m, 2 H), 1.78 (s, 3 H), 2.15-2.22 (m, 1 H), 2.22 (s, 3 H), 2.30-2.66 (m, 2 H), 7. 09 (dd, *J* = 8.5, 2.4 Hz, 1 H), 7.20 (d, *J* = 2.4 Hz, 1 H), 7.45 (d, *J* = 8.5 Hz, 1 H), 8.01 (br, 1 H) |

**[Table 7]**

| Example | Structural formula | ¹H-NMRδ : (LC-MS : [M+H]⁺ / Rt / Measuring condition) |
|---|---|---|
| 22 | 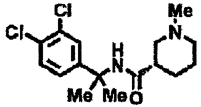 | ¹H NMR (300 MHz, CDCl₃)δ1.42-1.57 (m, 2 H), 1. 59 (s, 3 H), 1.61 (s, 3 H), 1.67-1.80 (m, 2 H). 2. 23 (s, 3 H), 2.30-2.40 (m, 2 H), 2.59-2.69 (m, 2 H), 7.17 (dd, *J* = 8.4, 2.1 Hz, 1 H), 7.33 (dd, *J* = 8.4, 2.1 Hz, 1 H), 7.40 (d, *J* = 2.1 Hz, 1 H), 8.3 6 (br, 1 H) |
| 23 | 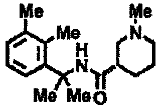 | ¹H NMR (300 MHz, CDCl₃)δ1.46-1.73 (m, 5 H), 1. 74 (s, 3 H), 1.81 (s, 3 H), 2.18 (s, 3 H), 2.24 (s, 3 H), 2.25-2.36 (m, 2 H), 2.38 (s, 3 H), 2.39-2.6 2 (m, 2 H), 7.06 (d, *J* = 4.8 Hz, 2 H), 7.29 (t, *J* = 4.8 Hz,12 H), 7.61 (br, 1 H) |
| 24 | 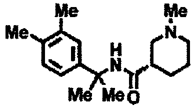 | ¹H NMR (300 MHz ,CDCl₃)δ1.48-1.57 (m, 2 H), 1. 60 (s, 3 H), 1.63 (s, 3 H), 1.66-1.87 (m, 2 H), 2. 26 (s, 3 H), 2.28-2.41 (m, 2 H), 2.66-2.74 (m, 2 H), 7.18 (t, *J* = 1.8 Hz, 1 H), 7.21 (d, *J* = 1.8 Hz , 2 H) |
| 25 | 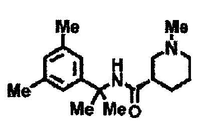 | ¹H NMR (400 MHz, CDCl₃)δ1.53-1.64 (m, 2 H), 1. 66 (s, 3 H), 1.67 (s, 3 H), 1.68-1.82 (m, 4 H), 2. 14-2.19 (m, 1 H), 2.22 (s, 3 H), 2.29 (s, 6 H), 6. 84 (s, 1 H), 6.97 (s, 2 H), 8.10 (br, 1 H) |
| 26 | 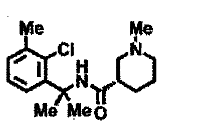 | ¹H NMR (400 MHz, CDCl₃)δ1.49-1.72 (m, 4 H), 1. 74 (s, 3 H), 1.75-1.80 (m, 1 H), 1.83 (s, 3 H), 2. 23 (s, 3 H), 2.25-2.33 (m, 2 H), 2.34 (s, 3 H), 2. 37-2.63 (m, 2 H), 7.10 (d, *J* = 4.8 Hz, 2 H), 7.36 (t, *J* = 4.8 Hz, 1 H), 7.86 (br, 1 H) |
| 27 | 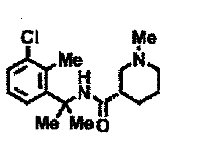 | ¹H NMR (400 MHz, CDCl₃)δ1.48-1.66 (m, 2 H), 1. 69 (s, 3 H), 1.70-1.77 (m, 2 H), 1.78 (s, 3 H), 2. 12-2.17 (m, 1 H), 2.20 (s, 3 H), 2.23-2.47 (m, 3 H), 2.50 (s, 3 H), 2.53-2.73 (m, 2 H), 7.07 (t, *J* = 8.0 Hz, 1 H), 7.25 (d, *J* = 8.0 Hz, 1 H), 7.34 (d , *J* = 8.0 Hz, 1 H), 8.08 (br, 1 H) |
| 28 | 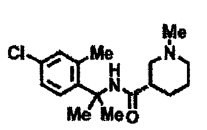 | ¹H NMR (400 MHz, CDCl₃)δ1.48-1.62 (m, 3 H), 1. 64 (s, 3 H), 1.66-1.74 (m, 2 H), 1.75 (s, 3 H), 2. 21 (s, 3 H), 2.22-2.37 (m, 2 H), 2.44 (s, 3 H), 2. 47-2.64 (m, 2 H), 7.07-7.11 (m, 2 H), 7.25 (d, *J* = 8.0 Hz, 1 H), 7.31 (d, *J* = 8.3 Hz, 1 H) |

**[Table 8]**

| Example | Structural formula | ¹H-NMRδ : (LC-MS : [M+H]⁺ / Rt / Measuring condition) |
|---|---|---|
| 29 | 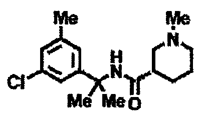 | ¹H NMR (400 MHz, CDCl₃)δ1.55-1.59 (m, 2 H), 1. 62 (s, 3 H), 1.63 (s, 3 H), 1.68-1.78 (m, 2 H), 1. 99-2.20 (m, 2 H), 2.23 (s, 3 H), 2.29 (s, 3 H), 2. 32-2.41 (m, 2 H), 2.52-2.75 (m, 1 H), 6.99 (s, 1 H), 7.02 (s, 1 H), 7.12 (s, 1 H), 8.16 (br, 1 H) |
| 30 | 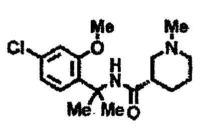 | ¹H NMR (400 MHz, CDCl₃) δ1.53-1.63(m, 3H), 1.6 8 (s, 3H), 1.69 (s, 3H), 1.71-1.78 (m, 1H), 2.27 ( s, 3H), 2.33-2.53 (m, 5H), 3.80 (s, 3H), 6.82 (d, *J* = 2.2 Hz, 1H), 6.88 (d, *J* = 8.4, 2.2 Hz, 1H), 7.24 (d, *J* = 8.4 Hz, 1H) |
| 31 | 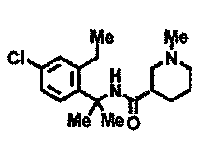 | ¹H NMR (400 MHz, CDCl₃) δ1.22 (t, *J* = 7.6 Hz, 3 H), 1.56-1.64(m, 2H), 1.66 (s, 3H), 1.69-1.74 ( m, 2 H), 1.76 (s, 3H), 2.26 (s, 3H), 2.31-2.70 (m, 5H), 2.74-2.93 (m, 2 H), 7.08 (dd, *J* = 8.5, 2.4 Hz, 1H), 7.17 (d, *J* = 2.4 Hz, 1H), 7.31 (d, *J* = 8 .4 Hz, 1H) |
| 32 | 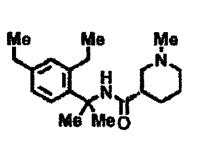 | ¹H NMR (400 MHz, CDCl₃) δ1.21 (t, *J* = 7.6 Hz, 3 H), 1.28 (t, J = 7.6 Hz, 3 H), 1.66-1.70(m, 1H ), 1.72 (s, 3H), 1.74-1.77 (m, 2 H), 1.78 (s, 3H). 2.32-2.48 (m, 4H), 2.53-2.63 (m, 4 H), 2.82 (q, J = 7.6 Hz, 2 H), 6.96 (dd, J = 8.2, 2.0 Hz, 1H), 7 .04 (d, J = 2.0 Hz, 1H), 7.29 (d, *J* = 8.2 Hz, 1H) |
| 33 | 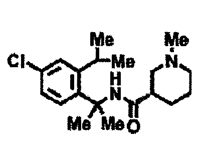 | ¹H NMR (400 MHz, CDCl₃) δ1.18 (d, *J* = 6.8 Hz, 3 H), 1.22 (d, *J* = 6.8 Hz, 3 H), 1.54-1.58 (m, 3 H), 1.65 (s, 3H), 1.68-1.74 (m, 2 H), 1.77 (s, 3H) , 2.22 (s, 3H), 2.25-2.53 (m, 4 H), 3.61 (7, *J* = 6 .8 Hz, 1 H), 7.07 (dd, *J* = 8.8, 2.4 Hz, 1H), 7.22 (d, *J* = 2.4 Hz, 1H), 7.30 (d. *J* = 8.4 Hz, 1H) |
| 34 | 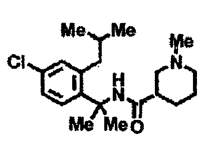 | ¹H NMR (400 MHz, CDCl₃) δ0.93 (d, *J* = 6.6 Hz, 6 H), 1.50-1.58 (m, 2H), 1.84-1.83 (m, 11 H), 2.0 1-2.11 (m, 1 H), 2.21 (s, 3 H), 2.26-2.40 (m, 1 H) , 2.43-2.65 (m, 1 H), 2.72 (d, *J* = 7.6 Hz, 2 H), 7.09 (dd, *J* = 8.6, 2.2 Hz, 1H), 7.19 (d, *J* = 2.2 Hz, 1H), 7.33 (d, *J* = 8.6 Hz, 1H) |

**[Table 9]**

| Example | Structural formula | ¹H-NMRδ : (LC-MS : [M+H]⁺ / Rt / Measuring condition) |
|---|---|---|
| 35 | 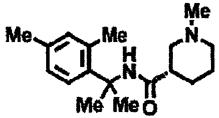 | ¹H NMR (400 MHz, CDCl₃) δ1.51-1.63 (m, 4 H), 1.69 (s, 3 H), 1.72-1.75 (m, 2H), 1.77 (s, 3 H), 2. 19 (s, 3 H), 2.25 (s, 3 H), 2.29-2.42 (m, 2 H), 2. 44 (s, 3 H), 2.52-2.60 (m, 1 H), 6.93 (s, 1H), 6.9 5 (d, *J* = 8.0 Hz, 1H), 7.28 (d, *J* = 8.0 Hz, 1H) |
| 36 | 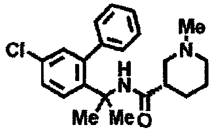 | ¹H NMR (400 MHz, CDCl₃) δ1.41-1.45 (m, 2 H), 1.48 (s, 3 H), 1.52-1.58 (m, 2H), 1.61 (s, 3 H), 1. 77-2.03 (m, 3 H), 2.10 (s, 3 H), 2.17-2.30 (m, 2 H), 6.96 (d, *J* = 2.4 Hz, 1H), 7.20-7.24 (m, 2 H), 7.25 (dd, *J* = 8.5, 2.4 Hz, 1H), 7.28-7.33 (m, 3 H ), 7.42 (d, *J* = 8.5 Hz, 1H) |
| 37 | 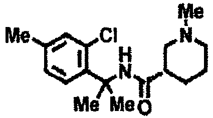 | ¹H NMR (300 MHz, CDCl₃)δ1.46-1.85 (m, 4H), 1.7 2 (s, 3H), 1.85 (s, 3H), 2.04-2.69 (m, 5H), 2.24 (s , 3H), 2.26 (s, 3H), 7.01 (dd, *J* = 8.1 Hz, 1.1 Hz, 1H), 7.12 (d, *J* = 1.1 Hz, 1H), 7.38 (d, *J* = 8.1 Hz, 1H) |
| 38 | 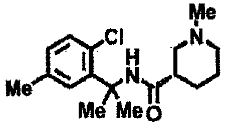 | ¹H NMR (300 MHz, CDCl₃)δ1.43-1.89 (m, 4H), 1.7 3 (s, 3H), 1.81 (s, 3H), 2.09-2.69 (m, 5H), 2.24 (s , 3H), 2.31 (s, 3H), 6.94 (dd, *J* = 8.0 Hz, 2.1 Hz, 1H), 7.17 (d, *J* = 8.0 Hz, 1H), 7.30 (d, *J* = 2.1 Hz, 1H) |
| 39 | 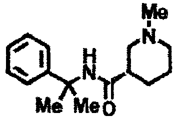 | ¹H NMR (300 MHz, CDCl₃)δ1.38-1.90 (m, 4H), 1.6 8 (s, 3H), 1.70 (s, 3H), 2.07-2.79 (m, 5H), 2.23 (s , 3H), 7.12-7.45 (m, 5H) (LC-MS : 261 / 0.523 / B) |
| 40 | 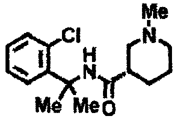 | ¹H NMR (400 MHz, CDCl₃) δ1.49-1.68 (m, 3 H), 1.73 (s, 3 H), 1.76-1.79 (m, 1H), 1.82 (s, 3 H), 2. 23 (s, 3 H), 2.26-2.61 (m, 5 H), 7.12 (td, *J* = 7.6, 1.2 Hz, 1 H), 7.20 (td, *J* = 7.6, 1.6 Hz, 1 H), 7. 28 (dd, *J* = 7.6, 1.2 Hz, 1 H), 7.49 (dd, *J* = 7.6, 1.6 Hz, 1 H) (LC-MS : 295 / 0.732 / C) |
| 41 | 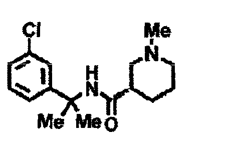 | ¹H NMR (400 MHz, CDCl₃) δ1.54-1.60 (m, 3 H) , 1.62 (s, 3 H), 1.65 (s, 3 H), 1.69-1.81 (m, 2H ), 2.13-2.22 (m, 1 H), 2.24 (s, 3 H), 2.31-2.40 ( m, 2 H), 2.59-2.71 (m, 1 H), 7.14-7.23 (m, 3H), 7.31-7.33 (m, 1 H), 8.28 (br, 1 H) |

**[Table 10]**

| Example | Structural formula | ¹H-NMRδ : (LC-MS : [M+H]⁺ / Rt / Measuring condition) |
|---|---|---|
| 42 | 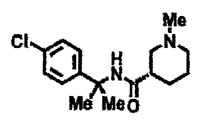 | ¹H NMR (300 MHz, CDCl₃)δ1.44-1.88 (m, 4H), 1.6 3 (s, 3H), 1.66 (s, 3H), 2.10-2.80 (m, 5H), 2.25 ( s, 3H), 7.18-7.41 (m, 4H) (LC-MS : 295 / 0.624 / B) |
| 43 | 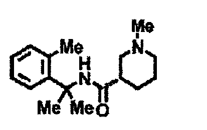 | ¹H NMR (400 MHz, CDCl₃) δ1.57-1.65 (m, 2 H), 1 .71 (s, 3 H), 1.73-1.77 (m, 2 H), 1.79 (s, 3 H), 2. 24 (s, 3 H), 2.28-2.43 (m, 3H) 2.48 (s, 3 H), 2.51 -2.63 (m, 2 H), 7.09-7.17 (m, 3H), 7.38-7.40 (m, 1 H) |
| 44 | 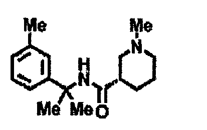 | ¹H NMR (300 MHz, CDCl₃)δ1.47-1.89 (m, 4H), 1.6 7 (s, 3H), 1.69 (s, 3H), 2.09-2.79 (m, 5H), 2.23 ( s, 3H), 2.34 (s, 3H), 6-98-7.06 (m, 1H), 7.12-7.26 (m, 3H) |
| 45 | 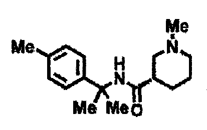 | ¹H NMR (300 MHz, CDCl₃)δ1.47-1.88 (m, 4H), 1.6 7 (s, 3H), 1.69 (s, 3H), 2.13-2.77 (m, 5H), 2.24 ( s, 3H), 2.31 (s, 3H), 7.12 (d, J = 8.1 Hz, 2H), 7. 27 (d, *J* = 8.1 Hz, 2H) |
| 46 | 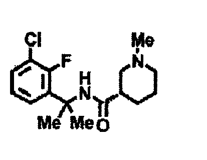 | ¹H NMR (400 MHz, CDCl₃) δ1.52-1.64 (m, 4 H), 1 .68 (s, 3 H), 1.74 (s, 3 H), 1.76-1.88 (m, 2 H), 2. 29 (s, 3 H), 2.35-2.68 (m, 3 H), 7.00 (t, *J* = 7.9 Hz, 1 H), 7.22-7.27 (m, 3 H) |
| 47 | 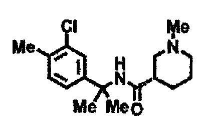 | ¹H NMR (300 MHz, CDCl₃)δ1.48-1.88 (m, 4H), 1.6 3 (s, 3H), 1.65 (s, 3H), 2.10-2.81 (m, 5H), 2.25 ( s, 3H), 2.32 (s, 3H), 7.09-7.18 (m, 2H), 7.33 (s, 1H) |
| 48 | 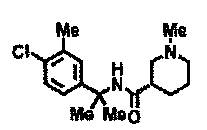 | ¹H NMR (300 MHz, CDCl₃)δ1.46-1.88 (m, 4H), 1.6 3 (s, 3H), 1.65 (s, 3H), 2.12-2.81 (m, 5H), 2.25 ( s, 3H), 2.35 (s, 3H), 7.13 (dd, J = 8.3 Hz, 2.4 H z, 1H), 7.22-7.28 (m, 2H) |

**[Table 11]**

| Example | Structural formula | ¹H-NMRδ : (LC-MS : [M+H]⁺ / Rt I Measuring condition) |
|---|---|---|
| 49 | 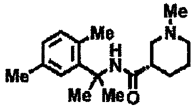 | ¹H NMR (400 MHz, CDCl₃) δ1.51-1.75 (m, 4H), 1. 68 (s, 3H), 1.80 (s, 3H), 2.21 (s, 3H), 2.19-2.66 ( m, 5H), 2.31 (s, 3H), 2.45 (s, 3H), 6.95 (d, *J* = 7 .6 Hz, 1H), 7.02 (d, *J* = 7.6 Hz, 1H), 7.21 (s, 1H ) (LC-MS : 269 / 3.39 / A) |
| 50 | 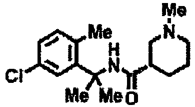 | ¹H NMR (400 MHz, CDCl₃) δ1.52-1.76 (m, 4H), 1. 67 (s, 3H), 1.77 (s, 3H), 2.23 (s, 3H),2.13-2.28 ( m, 1H), 2.33-2.71 (m, 4H), 2.44 (s, 3H), 7.02 (d, *J* = 8.0 Hz, 1H), 7.09 (d, *J* = 8.0 Hz, 1H), 7.36 ( s, 1H). (LC-MS : 309 / 3.59 / A) |
| 51 | 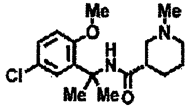 | ¹H NMR (400 MHz, CDCl₃) δ1.51-1.78 (m, 4H), 1. 72 (s, 3H), 2.25 (s, 3H), 2.30-2.37 (m, 2H), 2.48-2.58 (m, 3H), 6.77 (d, *J* = 8.7 Hz, 1H), 7.14 (dd, *J* = 8.7, 2.7 Hz, 1H), 7.29 (d, *J* = 2.7 Hz, 1H). (LC-MS : 325 / 0.589 / B) |
| 52 | 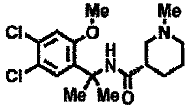 | ¹H NMR (400 MHz, CDCl₃) δ1.54-1.82 (m, 4H), 1. 70 (s, 3H), 1.74 (s, 3H), 2.19-2.34 (m, 1H), 2.27 (s, 3H), 2.36-2.71 (m, 4H), 7.23 (d, *J* = 2.4 Hz, 1 H), 7.25 (d, *J* = 2.4 Hz, 1H). (LC-MS : 359 / 0.697 / B) |
| 53 | 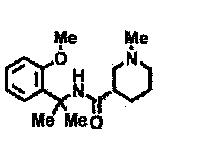 | ¹H NMR (300 MHz, CDCl₃) δ1.45-1.80 (m, 5H), 1. 75 (s, 3H), 1.76 (s, 3H), 2.13-2.77 (m, 5H), 2.24 (s, 3H), 2.24-2.26 (m, 4H), 3.83 (s, 3H), 6.86-6.95 (m, 2H). 7.19-7.24 (m, 1H), 7.36 (dd, *J* = 7.7 Hz , 1.7 Hz, 1H). (LC-MS : 291 / 0.583 / B) |
| 54 | 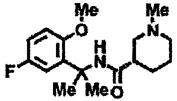 | ¹H NMR (400 MHz, CDCl₃) δ1.51-1.78 (m, 4H), 1. 72 (s, 3H), 1.73 (s, 3H), 2.25 (s, 3H), 2.29-2.56 ( m, 5H), 6.77 (dd, *J* = 9.0, 4.6 Hz, 1H), 6.86-6.91 (m, 1H), 7.07 (dd, *J* = 10.5, 3.1 Hz, 1H). (LC-M S : 309 / 0.498 / B) |
| 55 | 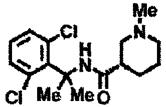 | ¹H NMR (400 MHz, CDCl₃) δ1.53-1.78 (m, 4 H), 1 .87 (s, 3 H), 2.06 (s, 3 H), 2.25 (s, 3 H), 2.32-2. 60 (m, 5 H), 6.98 (t, J = 7.9 Hz, 1H), 7.24 (d, *J* = 7.9 Hz, 2 H), 7.88 (br, 1 H). |

**[Table 12]**

| Example | Structural formula | ¹H-NMRδ : (LC-MS : [M+H]⁺ / Rt / Measuring condition) |
|---|---|---|
| 56 | 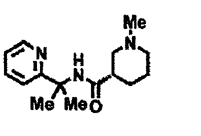 | ¹H NMR (400 MHz, CDCl₃) δ1.55-1.67 (m, 3 H), 1 .71 (s, 6 H), 1.72-1.78 (m, 1 H), 1.98-2.23 (m, 2 H), 2.27 (s, 3 H), 2.39-2.69 (m, 3 H), 7.14 (ddd, *J =* 7.8, 4.9, 1.0 Hz, 1 H), 7.36 (td, *J* = 1.0, 7.8 Hz, 1 H), 7.66 (td, *J* = 7.8, 2.0 Hz, 1 H), 8.44 (b r, 1 H), 8.48 (ddd, *J* = 4.9, 2.0, 1.0 Hz, 1 H). |
| 57 | 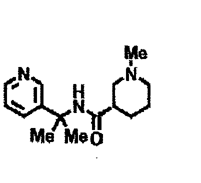 | ¹H NMR (400 MHz, CDCl₃) δ1.49-1.61 (m, 2 H), 1 .64 (s, 3 H). 1.67 (s, 3 H), 1.71-1.77 (m, 2 H). 2. 12-2.20 (m, 1 H), 2.24 (s, 3 H), 2.29-2.39 (m, 2 H), 2.57-2.69 (m, 2 H), 7.20 (ddd, *J* = 8.0, 4.8, 0 .8 Hz, 1 H), 7.64 (td, *J* = 1.8, 8.0 Hz, 1 H), 8.42 (ddd, *J* = 4.8, 1.8, 0.8 Hz, 1 H), 8.44 (br, 1 H), 8.61 (td, *J* = 0.8, 1.8 Hz, 1 H). |
| 58 | 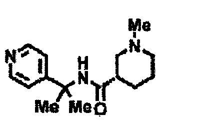 | ¹H NMR (400 MHz, CDCl₃) δ1.53-1.57 (m, 1 H), 1 .59 (s, 3 H), 1.62 (s, 3 H), 1.71-1.78 (m, 2 H), 2. 23-2.23 (m, 2 H), 2.25 (s, 3 H), 2.29-2.39 (m, 2 H), 2.60-2.72 (m, 2 H), 7.21-7.23 (m, 2 H), 8.48-8 .51 (m, 2 H). |
| 59 | 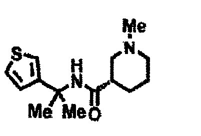 | ¹H NMR (400 MHz, CDCl₃) δ1-52-1.57 (m, 2 H), 1 .69 (s, 3 H), 1.69 (s, 3 H), 1.72-1.92 (m, 3 H), 2. 21 (s, 3 H), 2.34-2.65 (m, 4 H), 7.04-7.08 (m, 2 H), 7.23-7.25 (m, 1 H). |
| 60 | 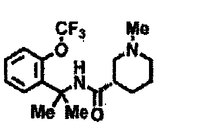 | ¹H NMR (400 MHz, CDCl₃) 61.54-1.63 (m, 4 H), 1 .69 (s, 3 H), 1.75 (s, 3 H), 1.85-1.91 (m, 1 H), 2. 25 (s, 3 H), 2.34-2.59 (m, 4 H), 7.17-7.24 (m, 3 H), 7.47 (dd, J = 8.0, 1.7 Hz. 1 H). (LC-MS : 345 / 0.867 / C) |
| 61 | 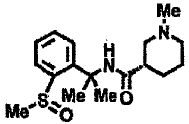 | ¹H NMR (300 MHz, CDCl₃)δ1.42-2.01 (m, 4H), 1.7 0 (s, 3H), 1.85 (s, 3H), 2.00-2.39 (m, 3H), 2.32 ( s, 3H), 2.58 (s, 3H), 2.65-2.93 (m, 2H), 7.38-7.53 (m, 3H), 8.10-8.24 (m, 1H). |

**[Table 13]**

| Example | Structural formula | ¹H-NMRδ : (LC-MS : [M+H]⁺ / Rt / Measuring condition) |
|---|---|---|
| 62 | 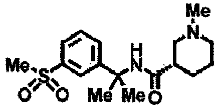 | ¹H NMR (300 MHz, CDCl₃)δ1.51-1.93 (m, 4H), 1. 67 (s, 3H), 1.69 (s, 3H), 2.12-2.80 (m, 5H), 2.28 (s, 3H), 3.05 (s, 3H), 7.52 (t, *J* = 7.8 Hz, 1H), 7.66 (d, *J* = 7.8 Hz, 1H), 7.77 (d. *J* = 7.8 Hz, 1H), 7.93 (s, 1H). |
| 63 | 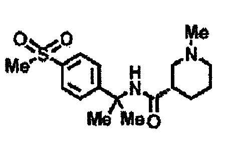 | ¹H NMR (300 MHz, CDCl₃)δ1.50-1-90 (m, 4H), 1. 64 (s, 3H), 1.68 (s, 3H), 2.12-2.49 (m, 3H), 2.28 (s, 3H), 2.49-2.83 (m, 2H), 3.04 (s, 3H), 7.55 ( d, J = 8.3 Hz, 2H), 7.87 (d, *J* = 8.3 Hz, 2H). |
| 64 | 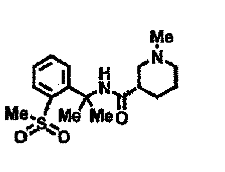 | ¹H NMR (300 MHz, CDCl₃)δ1.48-2.02 (m, 4H), 1. 84 (s, 3H), 1.91 (s, 3H), 2.19-2.71 (m, 5H), 2.28 (s, 3H), 3.02 (s, 3H), 7.36-7.47 (m, 1H), 7.51-7 .63 (m, 1H). 7.69 (d, *J* = 8.1 Hz, 1H), 8.03-8.14 (m, 1H). |
| 65 | 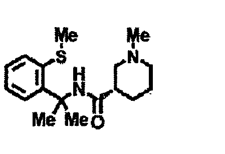 | ¹H NMR (300 MHz, CDCl₃)δ1.47-1.91 (m, 4H), 1. 75 (s, 3H), 1.83 (s, 3H), 1.98-2.77 (m, 5H), 2.26 (s, 3H), 2.45 (s, 3H), 7.06-7.27 (m, 3H), 7.40 ( d, *J* = 7.7 Hz, 1H). (LC-MS : 307 I 0.578 / B) |
| 66 | 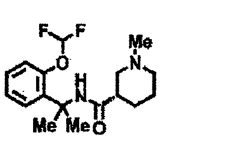 | ¹H NMR (300 MHz, CDCl₃)δ1.44-1.85 (m, 4H), 1. 71 (s, 3H), 1.77 (s, 3H), 2.03-2.73 (m, 5H), 2.25 (s, 3H), 6.52 (t, *J* = 75 Hz, 1H), 6.98 (d, *J* = 7.9 Hz ,1H), 7.08-7.26 (m, 2H), 7.46 (dd, *J* = 7. 7 Hz, 1.8 Hz, 1H). (LC-MS : 327 / 0.610 / B) |
| 67 | 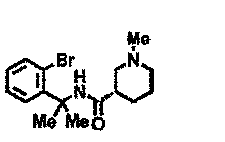 | ¹H NMR (400 MHz, CDCl₃) δ1.54-1.76(m, 4H), 1. 77 (s, 3H), 1.86 (s, 3H), 2.25 (s, 3H),2.30-2.52 (m, 4H),2.56-2.72 (brs, 1H),7.06 (t, *J* = 8.0 Hz, 1H), 7.23-7.31(m, 2H), 7.54 (d, *J* = 8.0 Hz, 1 H). (LC-MS : [M]⁺ 388 / 0.498 / B) |

**[Table 14]**

| Example | Structural formula | ¹H-NMRδ : (LC-MS : [M+H]⁺ / Rt / Measuring condition) |
|---|---|---|
| 68 | 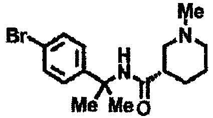 | ¹H NMR (300 MHz, CDCl₃)δ1.47-1.88 (m, 4H), 1.6 3 (s, 3H), 1.66 (s, 3H), 2.06-2.44 (m, 3H), 2.25 (s , 3H), 2.57-2.85 (m, 2H), 7.18-7.28 (m, 2H), 7.36-7.46 (m, 2H). |
| 69 | 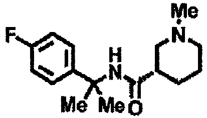 | ¹H NMR (400 MHz, CDCl₃)δ1.49-1.87 (m, 4H), 1.6 6 (s, 3H), 1.68 (s, 3H), 2.04-2.83 (m, 5H), 2.25 (s , 3H), 6.97-7.01 (m, 2H), 7.32-7.36 (m, 2H). |
| 70 | 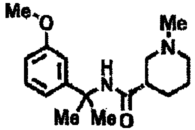 | ¹H NMR (400 MHz, CDCl₃)δ1.47-1.89 (m, 4H), 1.6 7 (s, 3H), 1.69 (s, 3H), 2.07-2.78 (m, 5H), 2.24 (s , 3H), 3.80 (s, 3H), 6.76 (dd, *J* = 8.2 Hz, 2.2 Hz, 1H), 6.93 (t, *J* = 2.2 Hz, 1H), 6.97 (dd, *J* = 7.8 Hz, 2.2 Hz, 1H), 7.23-7.26 (m, 1H). |
| 71 | 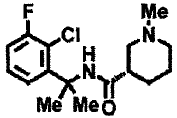 | ¹H NMR (400 MHz, CDCl₃)δ1.52-1.90 (m, 4H), 1.7 5 (s, 3H), 1.84 (s, 3H), 2.12-2.76 (m, 5H), 2.26 (s , 3H), 7.00-7.07 (m, 1H), 7.17-7.23 (m, 2H), 7.31 (d, *J* = 8.0 Hz, 1H). |
| 72 | 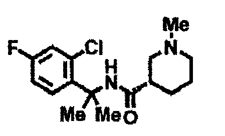 | ¹H NMR (400 MHz, CDCl₃)δ1.48-1.90 (m, 4H), 1.7 3 (s, 3H), 1.80 (s, 3H), 2.16-2.78 (m, 5H), 2.25 (s , 3H), 6.92-6.97 (m, 1H), 7.06 (dd, *J* = 8.4 Hz, 2. 8 Hz, 1H), 7.48 (dd, *J* = 8.8 Hz, 6.1 Hz, 1H). |
| 73 | 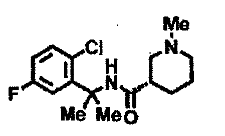 | ¹H NMR (400 MHz, CDCl₃)δ1.50-1.84 (m, 4H), 1.7 3 (s, 3H), 1.82 (s, 3H), 2.12-2.79 (m, 5H), 2.26 (s , 3H), 6.84-6.89 (m, 1H), 7.21-7.27 (m, 2H). |
| 74 | 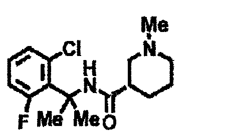 | ¹H NMR (400 MHz, CDCl₃)δ1.46-1.85 (m, 4H), 1.8 0 (d, *J* = 3.2 Hz, 3H), 1.91 (d, *J* = 2.8 Hz, 3H), 2.19-2.69 (m, 5H), 2.26 (s, 3H), 6.91-6.97 (m, 1H) , 7.04-7.13 (m, 2H). |

**[Table 15]**

| Example | Structural formula | ¹H-NMRδ : (LC-MS : [M+H]⁺ / Rt / Measuring condition) |
|---|---|---|
| 75 | 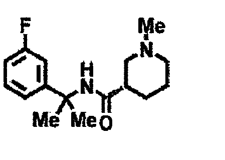 | ¹M NMR (400 MHz, CDCl₃)δ1.46-1.92 (m, 4H), 1.6 5 (s, 3H), 1.68 (s, 3H), 2.11-2.85 (m, 5H), 2.26 (s , 3H), 6.90 (td, *J =* 8.2 Hz, 2.4 Hz, 1H), 7.04-7.0 8 (m, 1H), 7.15 (d, *J* = 7.8 Hz, 1H), 7.25-7.30 (m , 1H). |
| 76 | 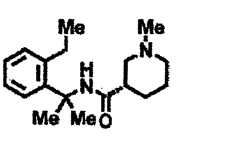 | ¹H NMR (300 MHz, CDCl₃)δ1.25 (t, *J* = 7.5 Hz, 3 H), 1.47-1.91 (m, 4H), 1.75 (s, 3H), 1.83 (s, 3H), 2.06-2.75 (m, 5H), 2.21 (s, 3H), 2.75-3.02 (m, 2H) , 7.06-7.33 (m, 3H), 7.40-7.43 (m, 1H). |
| 77 | 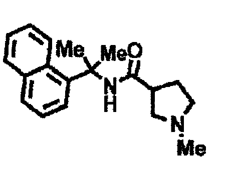 | ¹H NMR (300 MHz, CDCl₃) δ 1.82-2.13 (m, 2H), 1 .94 (s, 6H), 2.18-2.49 (m, 2H), 2.23 (s, 3H), 2.62-2.86 (m, 3H), 7.33-7.51 (m, 4H), 7.59 (d, *J* = 7.5 Hz, 1H), 7.74 (d, *J =* 8.3 Hz, 1H), 7.80-7.91 (m, 1H), 8.39-8.50 (m, 1H). |
| 78 | 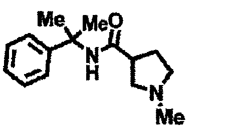 | ¹H NMR (300 MHz, CDCl₃) δ 1.65 (s, 3H), 1.66 ( s, 3H), 1.91-2.21 (m, 2H), 2.30-2.44 (m, 1H), 2.36 (s, 3H), 2.44-2.52 (m, 1H), 2.67-2.90 (m, 3H), 7.0 4-7.08 (m, 6H). |
| 79 | 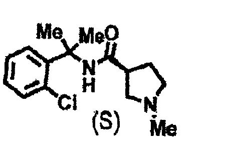 | (LC-MS: [M+1]⁺ 281 / 0.509 / B) |
| 80 | 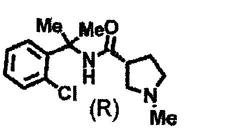 | (LC-MS : [M+1]⁺ 281 0.494 / B) |

**[Table 16]**

| Example | Structural formula | ¹H-NMRδ : (LC-MS : [M+H]⁺ / Rt / Measuring condition) |
|---|---|---|
| 81 | 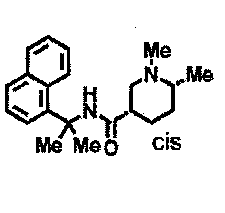 | ¹H NMR (300 MHz, CDCl₃) δ0.88 (d, *J* = 6.1 Hz, 3H), 1.39-1.60 (m, 3H), 1.80-2.01 (m, 2H), 1.92 (s , 3H), 1.95 (s, 3H), 2.03 (s, 3H), 2.16 (dd, *J* = 12 .1 Hz, 3.1 Hz, 1H), 2.28-2.38 (m, 1H), 2.90-3.03 ( m, 1H), 7.39-7.46 (m, 3H), 7.61 (d, *J* = 7.5 Hz, 1 H), 7.74 (d, *J* = 8.1 Hz, 1H), 7.84-7.87 (m, 1H), 8 .47-8.57 (m. 1H), 9.48 (bs, 1H). |
| 82 | 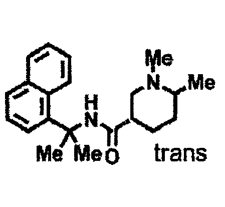 | ¹H NMR (300 MHz, CDCl₃) δ0.87 (d, *J* = 7.2 Hz, 3H), 1.16-1.36 (m, 2H), 1.43-1.56 (m, 1H), 1.63-2. 38 (m, 4H), 1.98 (s, 6H), 2.19 (s, 3H), 2.85-2.96 ( m, 1H), 6.25 (bs, 1H). 7.41-7.52 (m, 3H), 7.60 (d, *J* = 7.5 Hz, 1H), 7.78 (d, *J* = 8.1 Hz, 1H), 7.87-7.90 (m, 1H), 8.39-8.48 (m, 1H). |
| 83 | 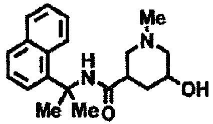 | ¹H NMR (300 MHz, CDCl₃) δ1.47-2.75 (m, 7H), 1. 99 (s, 3H), 2.00 (s, 3H), 3.73-3.87 (m, 1H), 6.68 ( bs, 1H), 7.42-7.51 (m, 3H), 7.61 (dd, *J* = 7.4 Hz, 1.0 Hz, 1H), 7.79 (d, *J* = 8.3 Hz, 1H), 7.85-7.9 4 (m, 1 H), 8.43-8.50 (m, 1H). |
| 84 | 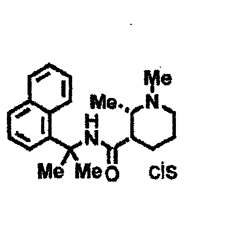 | ¹H NMR (300 MHz, CDCl₃) δ1.18 (t, *J* = 6.8 Hz, 3H), 1.41-1.64 (m, 2H), 1.84-2.07 (m, 3H), 1.96 ( s, 3H), 2.00 (s, 3H), 2.02 (s, 3H), 2.07-2.25 (m, 1 H), 2.25-2.36 (m, 1H), 2.62-2.77 (m, 1H), 7.40-7.4 8 (m, 3H), 7.63 (dd, *J* = 7.4 Hz, 1.2 Hz, 1H), 7. 76 (d, *J* = 8.1 Hz, 1H), 7.82-7.91 (m, 1H), 8.50-8.60 (m, 1H), 8.92 (bs, 1H). |
| 85 | 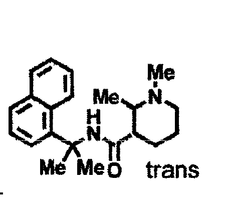 | ¹H NMR (300 MHz, CDCl₃) δ1.03 (t, *J* = 6.4 Hz, 3H), 1.49-1.83 (m, 4H), 1.91-2.06 (m, 1H), 1.98 ( s, 3H), 2.02 (s, 3H), 2.06-2.22 (m, 1H), 2.10 (s, 3 H), 2.38-2.54 (m, 1H), 2.54-2.72 (m, 1H), 7.09 (bs 1H), 7.41-7.49 (m, 3H), 7.61 (dd, *J =* 7.3 Hz, 1 .2 Hz, 1H), 7.78 (d, *J* = 8.1 Hz, 1H), 7.87-7.90 ( m, 1H), 8.47-8.50 (m, 1H). |

**[Table 17]**

| Example | Structural formula | ¹H-NMRδ : (LC-MS : [M+H]⁺ / Rt /Measuring condition |
|---|---|---|
| 86 | 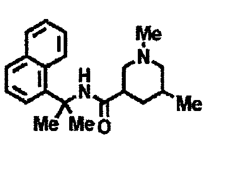 | ¹H NMR (300 MHz, CDCl₃) δ0.86 (t, *J* = 6.6 Hz, 3H), 1.04 (q, *J* = 12.3 Hz, 1H). 1.46 (t, *J* = 11.0 Hz, 1H), 1.49-1.75 (m, 1H), 1.75-1.8 5 (m, 1H), 1.90 (t, *J* = 11.2 Hz, 1H), 1.98 (s , 6H), 2.23 (s, 3H), 2.29-2.39 (m, 1H), 2.67-2. 77 (m, 1H), 2.87-2.97 (m, 1H), 5.91 (s, 1H), 7 .41-7.50 (m, 3H), 7.60 (dd, *J* = 7.5 Hz, 1.1 Hz, 1H), 7.79 (d, *J* = 8.1 Hz, 1H), 7.86-7.92 (m, 1H), 8.39-8.47 (m, 1H). |
| 87 | 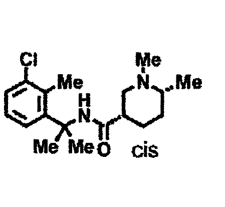 | ¹H NMR (300 MHz, CDCl₃)δ 1.08 (d, *J* = 6.3 Hz, 3 H), 1.32-1.69 (m, 3 H), 1.71 (s, 3 H), 1.73 (s, 3 H), 1.79-1.84 (m, 1 H), 2.21-2.24 ( m, 2 H), 2.27 (s, 3 H), 2.28 (s, 3 H), 2.41 (t t, *J* = 10.8, 3.6 Hz, 1 H), 2.48 (s, 3H), 2.91-2.95 (m, 1 H), 6.09 (br, 1 H), 7.07 (t, *J* = 8. 1 Hz, 1 H), 7.21 (d, *J* = 8.1 Hz, 1 H), 7.27 (d, *J* = 8.1 Hz, 1 H). |
| 88 | 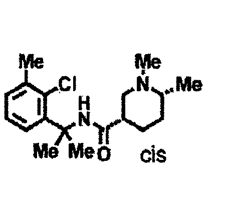 | ¹H NMR (300 MHz, CDCl₃)δ 1.07 (s, 3 H), 1.0 9 (s, 3 H). 1.55-1.23 (m, 2 H), 1.65-1.72 (m, 1 H), 1.76 (s, 3 H), 1.76 (s, 3 H), 1.83-1.89 ( m, 1 H), 1.98-1.95 (m, 1 H), 2.19 (t, *J* = 11. 3 Hz, 3 H), 2.27 (s, 3 H), 2.33 (s, 3 H), 2.41 (tt, *J* = 11.0, 3.7 Hz, 1 H), 2.96 (m, 1 H), 6 .19 (br, 1 H)7.12-7.09 (m, 2 H), 7.33 (m, 1 H) |
| 89 | 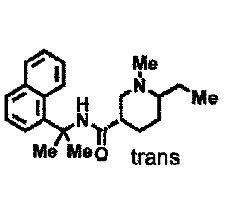 | ¹H NMR (300 MHz, CDCl₃)δ 0.39 (t, *J* = 7.6 Hz, 3 H), 1.23-1.59 (m, 5 H), 1.73 (s, 3 H), 1 .94 (s, 3 H), 2.04 (s, 3 H), 2.00-2.15 (m, 3 H ), 2.32 (br, 1 H), 2.97 (dt, *J* = 12, 2.1 Hz, 1 H), 7.36-7.46 (m, 3 H), 7.58 (dd, *J* = 7.5, 0 9 Hz, 1 H), 7.73 (d, *J* = 8.1 Hz, 1 H), 7.83-7.86 (m, 1 H), 8.50-8.54 (m, 1 H), 9.49 (br, 1 H). |
| 90 | 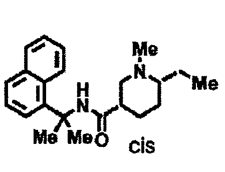 | ¹H NMR (300 MHz, CDCl₃)δ 0.84 (t, *J* = 7.5 Hz, 3 H), 1.28-1.91 (m, 8 H), 1.96 (s, 6 H), 2 .22 (s, 3 H), 2.25-2.38 (m, 1 H), 2.92-2.96 (m , 1 H), 6.31 (br, 1 H), 7.39-7.45 (m, 3 H), 7.5 8 (dd, *J* = 7.5, 0.9 Hz, 1 H), 7.76 (d, *J* = 8 .1 Hz, 1 H), 7.83-7.88 (m, 1 H), 8.40-8.43 (m, 1 H). |

**[Table 18]**

| Example | Structural formula | ¹H-NMRδ : (LC-MS : [M+H]⁺ / Rt / Measuring condition) |
|---|---|---|
| 91 | 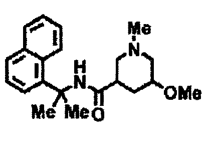 | ¹H NMR (300 MHz, CDCl₃)δ 1.37-1.60 (m, 1H), 1 .86-2.22 (m, 2H), 1.97 (s, 3H), 1.99 (s, 3H), 2.0 3 (s, 3H), 2.22-2.42 (m, 1H). 2.47-2.68 (m, 1H), 3.28 (s, 3H), 3.34-3.48 (m, 1 H), 7.40-7.48 (m, 3 H), 7.60 (dd, *J* = 7.6 Hz, 1.2 Hz, 1H), 7.76 (d, *J* = 8.1 Hz, 1H), 7.82-7.92 (m, 1H). 8.41-8.50 (m, 1H). |
| 92 | 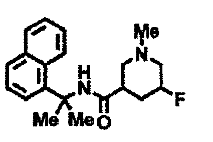 | ¹H NMR (400 MHz, CDCl₃)δ 1.75-2.14 (m, 2H), 1 .92 (s, 3H), 2.01 (s, 3H), 2.02 (s, 3H), 2.26-2.71 (m, 5H), 4.55-4.76 (m, 1H), 7.41-7.48 (m, 3H), 7.60 (d, *J* = 7.3 Hz, 1H), 7.76 (d, *J* = 8.0 Hz, 1H), 7.84-7.89 (m, 1H), 8.49-8.57 (m, 1H). |
| 93 | 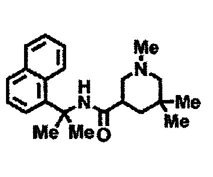 | ¹H NMR (300 MHz, CDCl₃)δ 0.90 (t, *J* = 7.7 Hz , 3H), 1.24-1.34 (m, 1H), 1.58-2.09 (m, 3H), 1.92 (s, 3H), 1.96 (s, 3H), 2.03 (s, 3H), 2.25-2.31 ( m, 1 H), 2.54-2.68 (m, 2H), 7.40-7.48 (m, 3H), 7. 61 (d, *J* = 6.4 Hz, 1H), 7.76 (d, *J* = 8.1 Hz, 1 H), 7.82-7.92 (m, 1H), 8.46-8.56 (m, 1H), 8.63 (b s, 1H). |
| 94 | 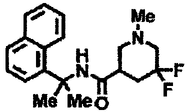 | ¹H NMR (300 MHz, CDCl₃)δ 1.85-2.21 (m, 1H). 1 .93 (s, 3H), 1.97 (s, 3H), 2.11 (s, 3H), 2.33-2.51 (m. 2H), 2.51-2.67 (m, 3H), 7.17 (bs, 1H), 7.40-7.49 (m, 3H), 7.59 (d, *J* = 6.4 Hz, 1H), 7.77 (d , *J* = 8.3 Hz, 1H), 7.82-7.92 (m, 1H), 8.37-6.51 (m, 1H). |
| 95 | 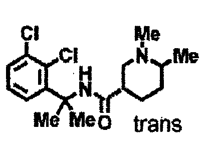 | ¹H NMR (400 MHz, CDCl₃)δ 1.11 (d, *J* = 6.4 Hz , 3 H), 1.54-1.66 (m, 4H), 1.70 (s, 3 H), 1.87 (s, 3 H). 1.89-1.91 (m, 1 H), 2.23-2.26 (m, 1 H), 2. 28 (s, 3 H), 2.37 (br, 1 H), 3.04-3.07 (m, 1 H), 7.15 (t, *J* = 8.0 Hz, 1 H), 7.34 (dd, *J* = 8.0, 1 .6 Hz, 1 H), 7.43 (dd, *J* = 8.0, 1.6 Hz, 1 H). |
| 96 | 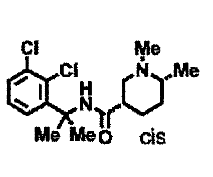 | ¹H NMR (400 MHz, CDCl₃)δ 1.18 (d, *J* = 6.0 Hz , 3 H), 1.46-1.66 (m, 2H), 1.74 (s, 3 H), 1.76 (s, 3 H), 1.89-2.18 (m, 3 H), 2.23-2.26 (m, 1 H), 2. 34 (t, *J* = 11.2 Hz, 1 H), 2.40 (s, 3 H), 2.64 ( m, 1 H), 3.05-3.08 (m, 1 H), 6.37 (br, 1 H), 7.15 (t, *J* = 8.0 Hz, 1 H), 7.35 (dd, *J* = 8.0, 1.6 H z, 1 H), 7.40 (dd, *J* = 8.0, 1.6 Hz, 1 H). |

**[Table 19]**

| Example | Structural formula | ¹H-NMRδ: (LC-MS : [M+H]⁺ / Rt / Measuring condition) |
|---|---|---|
| 97 | 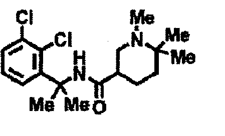 | ¹H NMR (400 MHz, CDCl₃)δ 1.15 (s, 3 H), 1.30 (s, 3 H), 1.19-1.24 (m, 1H). 1.61-1,69 (m, 2 H), 1.72 (s, 3 H), 1.73-1.76 (m, 1 H), 1.79 (s, 3 H), 2.45 (s, 3 H), 2.83-2.94 (m, 2 H), 7.16 (t, *J* = 8.0 Hz, 1 H), 7.35 (dd, *J =* 8.0, 1.6 Hz, 1 H), 7.41 (dd, *J* = 8.0, 1.6 Hz, 1 H). |
| 98 | 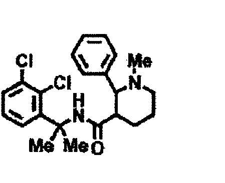 | ¹H NMR (400 MHz, CDCl₃)δ 1.32 (s, 3 H), 1.34 (s, 3 H), 1.56-1.64 (m, 1 H), 1.68-1.76 (m, 2 H), 1.84-1.92 (m, 1 H), 1.94 (s, 3 H), 2.09-2.16 (m , 1 H) 2.27 (ddd, *J* = 12.8, 9.0, 2.8 Hz, 1 H), 2.92-2.99 (m, 1 H), 7.05 (t, *J* = 8.0 Hz, 1 H), 7.16 (dd, *J* = 8.0, 1.6 Hz, 1 H), 7.20-7.32 (m, 6 H), 7.94 (br, 1 H). |
| 99 | 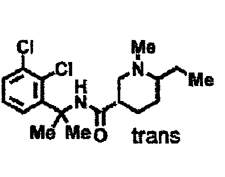 | ¹H NMR (400 MHz, CDCl₃)δ0.86 (t, *J* = 7.6 Hz, 3 H), 1.43-1.67 (m, 7 H), 1.69 (s, 3 H), 1.89 ( S, 3 H), 1.90-1.97 (m, 1 H), 2.23 (s, 3 H), 2.24-2.30 (m, 1 H), 3.10 (dt, *J* = 12.0, 2.2 Hz, 1 H) , 7.14 (t, *J* = 8.0 Hz, 1 H), 7.34 (dd, *J* = 8.0, 1.6 Hz, 1 H), 7.43 (dd, *J* = 8.0, 1.6 Hz, 1 H), 9.31 (br, 1 H). |
| 100 | 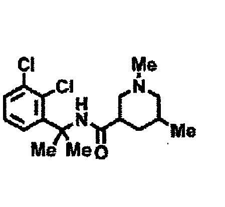 | ¹H NMR (400 MHz, CDCl₃)δ0.86 (d, *J* = 6.6 Hz, 3 H), 1.00 (q, *J* = 11.8 Hz, 1 H), 1.46 (t, *J* = 11.8 Hz, 1 H), 1.58-1.73 (m, 1 H). 1,75 (s, 6 H), 1.81-1.89 (m, 2 H), 2.23 (s, 3 H), 2.39 (tt, *J* = 11.8, 3.7), 2.71-2.76 (m, 1 H), 2.90-2.95 (m. 1 H), 5.97 (br, 1 H), 7.15 (t, *J* = 8.0 Hz, 1 H ), 7.34 (d, *J* = 8.0 Hz, 1 H), 7.40 (d, *J* = 8.0 Hz, 1 H). |
| 101 | 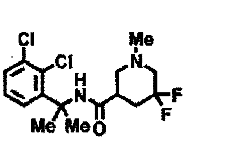 | ¹H NMR (400 MHz, CDCl₃)δ1.72 (s, 3 H), 1.78 ( s, 3 H), 1.95-2.12 (m, 2 H), 2.34 (s, 3 H), 2.38-2.51 (m, 2 H),2.59 (tt, 8.0, 4.4 Hz, 1 H), 2.64-2. 79 (m, 2 H), 6.85 (br, 1 H), 7.16 (t, *J* = 8.0 H z, 1 H), 7.36 (dd, *J* = 8.0, 1.6 Hz, 1 H), 7.42 (dd, *J* = 8.0, 1.6 Hz, 1 H). |

**[Table 20]**

| Example | Structural formula | ¹H-NMRδ : (LC-MS : [M+H]⁺ / Rt / Measuring condition) |
|---|---|---|
| 102 | 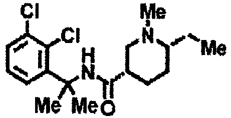 | ¹H NMR (400 MHz, CDCl₃)δ0.85 (t, *J* = 7.2 Hz, 3 H ), 1.22-1.73 (m, 5 H), 1.75 (s, 3 H), 1.76 (s, 3 H). 1. 78-1.88 (m, 2 H), 2.18 (t, *J* = 11.0 Hz, 1 H). 2.23 (s , 3 H), 2.34 (tt, *J* = 11.0, 3.5 Hz, 1 H), 2.90-295 (m , 1H), 6.26 (br, 1 H), 7.14 (t, *J* = 8.0 Hz, 1 H), 7.3 4 (dd, J = 8.0, 1.2 Hz, 1 H), 7.41 (dd, *J* = 8.0, 1.2 Hz, 1 H). |
| 103 | 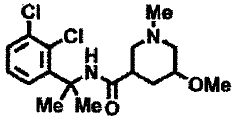 | ¹H NMR (400 MHz, CDCl₃)δ1.49-1.70 (m, 1 H), 1.73 ( s, 3 H), 1.78 (s, 3 H), 1.95-2.26 (m, 2 H), 2.28 (s, 3 H), 2.30-2.41 (m, 1 H), 2.53-2,85 (m, 2 H), 3.28-3.40 (m, 4 H), 6.67 (br, 1 H), 7.15 (t, *J* = 8.0 Hz, 1 H), 7.34 (d, *J* = 8.0 Hz, 1 H), 7.42 (d, *J* = 8.0 Hz, 1 H). |
| 104 | 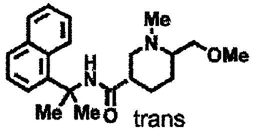 | ¹H NMR (400 MHz, CDCl₃)δ1.45-1.84 (m, 4 H), 1.91 ( s, 3 H), 1.93-1.98 (m, 1 H), 2.00 (s, 3 H), 2.02 (s, 3 H), 2.21-2.24 (m, 1 H), 2.30 (br, 1 H), 296 (dt, *J* = 12.0, 2.3 Hz, 1 H), 3.16 (dd, *J* = 10.1, 3.6 Hz), 3.1 8 (s, 3 H), 3.23 (dd, *J* = 10.1, 3.6 Hz), 7.37-7.44 (m , 3 H), 7.59 (d, *J* = 7.6 Hz, 1 H), 7.72 (d, *J* = 7.6 Hz, 1 H), 7.81-7.87 (m, 1 H), 8.48-8.54 (m, 1 H), 9.4 2 (br, 1 H). |
| 105 | 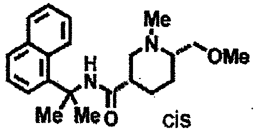 | ¹H NMR (400 MHz, CDCl₃)δ1.41-1.49 (m, 2 H), 1.67-1.71 (m, 1 H), 1.79-1.86 (m, 1 H), 1.95 (s, 6 H), 1.98 (m, 1 H), 2.20 (t, *J* = 11.1 Hz, 1 H), 2.24 (s, 3 H), 227-2.36 (m, 1 H), 2.89-2.95 (m, 1 H), 3.29 (s, 3 H ), 3.32 (dd, *J* = 10.0, 4.0 Hz, 1 H), 3.40 (dd, *J* = 1 0.0, 4.0 Hz, 1 H), 6.10 (br, 1 H), 7.39-7.45, (m, 3 H), 7.58 (d, *J* = 7.2 Hz, 1 H), 7.76 (d, *J* = 8.0 Hz, 1 H), 7.84-7.88 (m, 1 H), 8.38-8.42 (m, 1 H). |
| 106 | 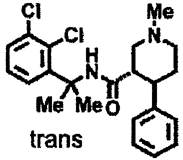 | ¹H NMR (300 MHz, CDCl₃)δ1.32-1.40 (m, 1H), 1.40 (s, 6H), 1.79-1.99 (m, 2H), 2.02-2.11 (m, 1H), 2.19 (t, *J =* 11.1 Hz, 1H), 2.31 (s, 3H), 2.48-2.57 (m, 1H), 2.67-2.76 (m, 1H), 2.90-3.03 (m, 2H), 5.59 (s, 1H), 7.06 (t, 1H), 7.17-7.36 (m, 7H). (LC-MS : [M+1]⁺ 405 / 0.757 / B) |

**[Table 21]**

| Example | Structural formula | ¹H-NMRδ : (LC-MS : [M+H]⁺ / Rt /Measuring condition) |
|---|---|---|
| 107 | 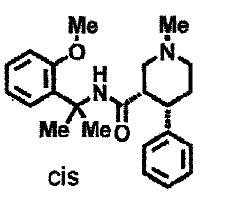 | ¹H NMR (300 MHz, CDCl₃)δ1.65-1.81 (m, 1H). 1 .73 (s, 3H), 1.74 (s, 3H), 2.05-2.17 (m, 1H), 2.1 9-2.37 (m, 1H), 2.31 (s, 3H), 2.37-2.53 (m, 1H), 2.57 (bs, 1H), 2.71-2.84 (m, 1H), 2.99-3.15 (m, 2H), 8.84-6.89 (m, 2H), 7.03-7.22 (m, 5H), 7.33 (dd, *J* = 8,1 Hz, 1.7 Hz, 1 H), 9.07 (bs, 1H). (LC-MS : [M+1]⁺ 367 0.768 / B) |
| 108 | 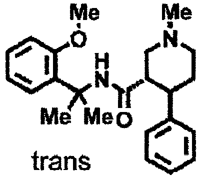 | ¹H NMR (300 MHz, CDCl₃)δ1.38 (s, 3H), 1.51 ( s, 3H), 1.81-1.98 (m, 2H), 2.09 (t, *J* = 11.0 Hz , 4.0 Hz, 1H), 2.23 (t, *J* = 11.2 Hz, 1H), 2.33 (s, 3H), 2.46 (td, *J* = 11.0 Hz, 3.6 Hz, 1H), 2. 74-2.83 (m, 1H), 2.91-2.99 (m, 2H), 5.68 (s, 1H) , 6.76-6.81 (m, 2H), 6.99 (dd, *J* = 8.0 Hz, 1.7 Hz 1H), 7.12-7.31 (m, 6H). (LC-MS : [M+1⁺ 367 / 0.744 / B) |
| 109 | 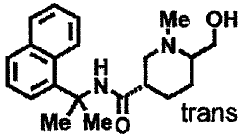 | ¹H NMR (400 MHz, CDCl₃)δ1.59-1.65 (m, 2 H), 1.82-1.94 (m, 3 H), 1.96 (s, 6 H), 2.20-2.28 (m, 5 H), 2.93-2.95 (m, 1 H), 3.34 (dd, *J* = 11.0, 1.8 Hz, 1 H), 3.80 (dd, *J* = 11.0, 3.9 Hz, 1 H), 6.03 (br, 1 H), 7.39-7.47, (m, 3 H), 7.58 (d, *J* = 7.6 Hz, 1 H), 7.76 (d, *J* = 8.4 Hz, 1 H), 7. 86-7.88 (m, 1 H), 8.40-8.42 (m, 1 H). |
| 110 | 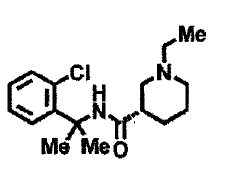 | ¹H NMR (400 MHz, CDCl₃) δ1.06 (t, *J* = 7.2 H z, 3 H), 1.52-1.60 (m, 2 H), 1.74 (s, 3 H), 1.76-1.80 (m, 2H), 1.83 (s, 3 H), 1.87-1.93 (m, 1 H), 2.19-2.40 (m, 5 H), 2.62-2.81 (m, 1 H), 7.12 (t d, *J =* 7.9, 1.6 Hz, 1 H), 7.21 (td, *J* = 7.9, 1. 6 Hz, 1 H), 7.28 (dd, *J* = 7.9, 1.6 Hz, 1 H), 7. 49 (dd, *J* = 7.9, 1.6 Hz, 1 H), 8.33 (br, 1 H). (LC-MS : 309 / 0.767 / C) |

**[Table 22]**

| Example | Structural formula | ¹H-NMRδ : (LC-MS : [M+H]⁺ / Rt I Measuring condition) |
|---|---|---|
| 111 | 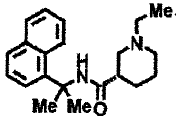 | (LC-MS : 325 / 1.065 C) |
| 112 | 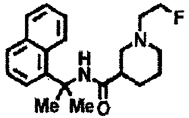 | (LC-MS : 343 / 0.974 / C) |
| 113 | 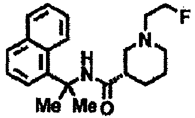 | (LC-MS : 343 I 0.988 / C) |
| 114 | 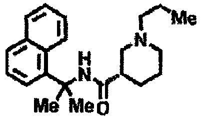 | ¹H NMR (300 MHz, CDCl₃)δ0.48 (t, *J* = 7.3 Hz, 3H), 0.76-0.93 (m, 1H), 0.93-1.12 (m, 1H), 1.41-1.58 (m, 2H), 1.65-2.10 (m, 5H), 1.97 (s, 3H), 2. 04 (s, 3H), 2.18 (d, *J* = 11.7 Hz, 1H), 2.32-2.41 (m, 1H), 2.65 (d, *J* = 10.3 Hz, 1H), 2.86 (d, *J* = 12.1 Hz, 1H), 7.37-7.48 (m, 3H), 7.60 (dd, *J* = 7.4 Hz, 1.0 Hz, 1H), 7.75 (d, *J* = 8.1 Hz, ¹H ), 7.82-7.90 (m, 1H), 8.50-8.57 (m, 1H). |
| 115 | 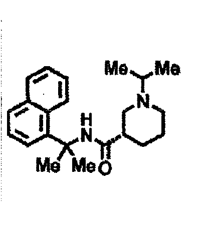 | ¹H NMR (300 MHz, CDCl₃)δ0.51 (d. *J* = 6.6 Hz, 3H), 0.70 (d, *J* = 6.4 Hz, 3H), 1.41-1.60 (m, 2H ), 1.64-1.80 (m, 1H), 1.87-2.13 (m, 2H), 1.98 (s, 3H), 2.04 (s, 3H), 2.27-2.41 (m ,3H), 2.59 (d, *J* = 11.2 Hz, 1H), 2.82 (d, *J* = 10.3 Hz, 1H), 7.40 -7.46 (m, 3H), 7.61 (d, *J* = 7.3 Hz, 1H), 7.76 (d , *J* = 8. 1 Hz, 1H), 7.83-7.88 (m, 1H), 8.54-8.57 (m, 1H). |
| 116 | 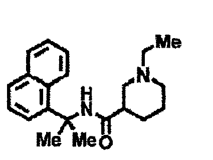 | ¹H NMR (300 MHz, CDCl₃)δ0.60 (t, *J* = 7. 2 Hz , 3H), 1.42-2.97 (m, 11H). 1.97 (s, 3H), 2.03 (s, 3H), 7.37-7.51 (m, 3H), 7.61 (d, *J* = 7.3 Hz, ¹H ), 7.76 (d, *J =* 8.1 Hz, 1H), 7.83-7.91 (m, 1H), 8.44-8.68 (m, 1H). (LC-MS : 325 / 0.538 / B) |

**[Table 23]**

| Example | Structural formula | ¹H-NMRδ : (LC-MS : [M+H]⁺ I Rt / Measuring condition) |
|---|---|---|
| 117 | 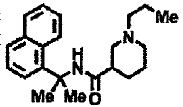 | ¹H NMR (300 MHz, CDCl₃)δ0.49 (t, *J* = 7.3 Hz, 3H), 0.73-0.93 (m, 1H), 0.93-1.12 (m, 1H), 1.41-1.58 (m, 2H), 1.65-2.10 (m. 5H), 1.97 (s, 3H), 2. 04 (s, 3H), 2.19 (d, *J* = 10.8 Hz, 1H), 2.31-2.4 4 (m, 1H), 2.66 (d, *J* = 9.5 Hz, 1H), 2.86 (d, *J* = 11.2 Hz, 1H), 7.40-7.49 (m, 3H), 7.61 (d, *J* = 7.3 Hz, 1H), 7.76 (d, *J* = 8.3 Hz, 1H), 7.83-7.91 (m, 1H), 8.49-8.60 (m, 1H). (LC-MS : 339 / 0.573 / B) |
| 118 | 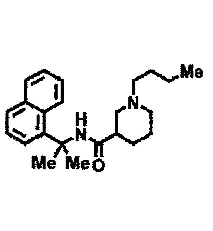 | ¹H NMR (300 MHz, CDCl₃)δ0.67 (t, *J* = 6.7 Hz, 3H), 0.69-1.07 (m, 4H), 1.39-1.62 (m, 2H), 1.62-2.11 (m, 5H), 1.98 (s, 3H), 2.04 (s, 3H), 2.17 (d, *J* = 11.6 Hz, 1H), 2.31-2.44 (m, 1H), 2.66 (d, *J* = 9.7 Hz, 1H), 2.86 (d, *J =* 11.0 Hz, 1H), 7 37-7.50 (m, 3H),7.61 (d, *J* = 7.3 Hz, 1H), 7.76 (d, *J* = 8.1 Hz, 1H), 7.82-7.93 (m, 1H), 8.50-8.6 2 (m, 1H). (LC-MS : 363 / 0.619 / B) |
| 119 | 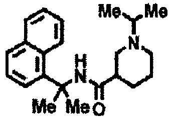 | ¹H NMR (300 MHz, CDClₐ)δ0.58 (t, *J* = 6.2 Hz, 3H), 0.75 (t, *J* = 6.4 Hz, 3H), 1.43-1.64 (m, 2 H), 1.64-1.80 (m, 1H), 1.80-1.94 (m, 1H), 1.97 (s, 3H), 2.03 (s, 3H), 2.11-2.30 (m, 1H), 2.30-2.52 (m, 3H), 2.52-2.68 (m, 1 H), 2.80 (d, *J* = 8.4 Hz , 1H), 7.36-7.50 (m, 3H), 7.60 (dd, *J* = 7.5 Hz, 1.1 Hz, 1H), 7.75 (d, *J* = 8.1 Hz, 1H), 7.81-7. 91 (m, 1H), 8.50-8.61 (m, 1 H). |
| 120 | 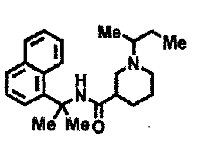 | ¹H NMR (300 MHz CDCl₃,)δ0.39 (t, *J* = 7.3 Hz, 2H), 0.56-1.13 (m, 4H), 1.42-2.38 (m, 6H), 1.97 (s, 3H), 2.04 (s, 3H), 2.36-2.68 (m, 3H), 2.68-2. 87 (m, 1H), 7.37-7.56 (m, 3H), 7.55-7.64 (m, 1H) , 7.75 d, *J* = 8.1 Hz, 1H), 7.82-7.90 (m, 1H), 8 .47-8.62 (m, 1H). |
| 121 | 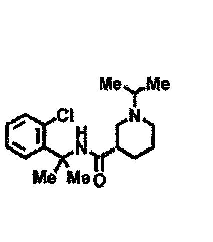 | ¹H NMR (400 MHz, CDCl₃) δ0.99 (d, *J* = 6.8 Hz , 3 H), 1.01 (d, *J* = 6.8 Hz, 3 H), 1.52-1.57 (m, 2 H), 1.74 (s, 3 H), 1.75-1.80 (m, 2H), 1.84 (s, 3 H), 2.24-2.35 (m, 2 H), 2.46-2.49 (m, 1 H), 2. 67-2.71 (m, 1 H), 2.76 (7, *J* = 2.8 Hz, 1 H), 2. 86-2.89 (1 H), 7.12 (td, *J* = 7.8, 1.2 Hz, 1 H), 7.20 (td, *J* = 7.8, 1.2 Hz, 1 H), 7.28 (dd, *J* = 7.8, 1.6 Hz, 1 H), 7.49 (dd, *J =* 7.8, 1.6 Hz, 1 H), 8.60 (br, 1 H). (LC-MS : 323 / 0.645 / C) |

**[Table 24]**

| Example | Structural formula | ¹H-NMRδ : (LC-MS : [M+H]⁺ / Rt / Measuring condition) |
|---|---|---|
| 122 | 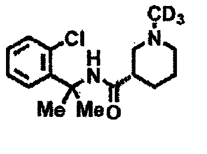 | ¹H NMR (400 MHz, CDCl₃) δ1.51-1.68 (m, 3 H), 1.73 (s, 3 H), 1.75-1.79 (m, 1H), 1.82 (s, 3 H), 2 .00 (br, 1 H), 2.27-2.60 (m, 4 H), 2.62-2.81 (m, 1 H), 7.12 (td, *J* = 7.6, 1.6 Hz, 1 H), 7.21 (td, *J* = 7.6, 1.6 Hz, 1 H), 7.28 (dd, *J* = 7.6, 1.6 Hz, 1 H), 7.49 (dd, *J* = 7.6, 1.6 Hz, 1 H), 7.99 (br, 1 H). (LC-MS : 298 / 0.702 / C) |

### Test Examples

Hereinafter, pharmacological test results of the representative compounds of the present invention will be indicated, and the pharmacological effects of the compounds will be explained, however, the present invention will not be limited by these Test Examples.

### Test Example 1

Evaluation of Agonist Activities Using Cells that stably expressing human-derived SSTR4 (human SSTR4-stably expressing cells)

### (1) Human SSTR4-stably expressing cells

Human SSTR4-stably expressing cells were prepared, and cultured. Specifically, HEK293 cells, human kidney-derived cells, were used as host cells. By introducing pcDNA3.1 (cat. No. V79020, invitrogen, Carlsbad, CA, USA) to which human SSTR gene was inserted into HEK293 cells, human SSTR4-stably expressing cell line was obtained. For selection, G418 (cat. No. 16513-84, Nacalai tesque, Japan) was used.

As the culture medium, D-MEM medium (cat. No. 11995, GIBCO, USA), containing 10% FBS and 200 µg/mL G418, was used, the culture was performed in a 225 cm² flask (cat. No. 11-006-010, Iwaki, Japan).

At about 80% confluence, the cells were harvested by treating with 20-fold diluted 0.25% Trypsin-EDTA (cat. No. 35554-64, Nacalai, tesque, Japan). The harvested cells were prepared at a reaction medium composed of Hanks Buffer (cat. No. 14065-056, invitrogen, Carlsbad, CA, USA)/20mM Hepes (cat. No. 15630-080, invitrogen, Carlsbad, CA, USA), and 0.1% BSA (cat. No. A7906, Sigma Aldrich, USA) to 70000 cells/mL. Gα16 and apoaequorin were transiently introduced to the harvested cells, and the cells were seeded to a 384 well plate (cat. No. 781090, Greiner, Germany) at about 2000 cells/30 µL/well. On the next day of cell seeding, Coelenterazine H (cat. No. S2011, Promega, USA) was added to each well (10 µL/well) to make a final concentration of 0.5 µmol/L, and the plate was centrifuged. Then, the plate was left at room temperature for 4 hours in shaded state.

### (2) Preparation of Test compounds

DMSO solution of each test compound having 1000-fold larger concentration than the final concentration was prepared. The DMSO solutions were made to have 10-fold concentration than the final concentration with Hanks/20 mmol/L Hepes/0.1% BSA.

### (3) Evaluation of Agonist Activity

For detection of luminescence signal due to SSTR4-stimulation, FDSS7000 (manufactured by Hamamatsu Photonics K.K.) was used. To plates having cells and the luminescence substrate, solutions of test compounds were added, then each luminescence signal was measured for 90 seconds, and each relative luminescence unit (RLU) was calculated. Based on luminescence signal from the wells to which a 1 µmol/L somatostatin solution was added taken as 100%, agonist activity of each test compound was evaluated. Evaluation results are shown in Tables 25 to 27.

**[Table 25]**

| Example | EC50 (nmol/L) | Example | EC50 (nmol/L) |
|---|---|---|---|
| 1 | 62.7 | 19 | 1.8 |
| 2 | 1.4 | 20 | 1.3 |
| 3 | 12.6 | 21 | 2.0 |
| 4 | 343 | 22 | 10.9 |
| 5 | 34.1 | 23 | 7.4 |
| 6 | 14.2 | 24 | 18.9 |
| 7 | 4.3 | 25 | 15.9 |
| 8 | 8.9 | 26 | 2.2 |
| 9 | 43.3 | 27 | 2.1 |
| 10 | 24.2 | 28 | 12.2 |
| 11 | 8.4 | 29 | 5.3 |
| 12 | 9.7 | 30 | 1.5 |
| 13 | 3.7 | 31 | 1.4 |
| 14 | 5.7 | 32 | 4.0 |
| 15 | 3.5 | 33 | >100 |
| 16 | 2.3 | 34 | >100 |
| 17 | 2.3 | 35 | 3.3 |
| 18 | 2.0 | 36 | >100 |

**[Table 26]**

| Example | EC50 (nmo l/L) | Example | EC50 (nmo l/L) |
|---|---|---|---|
| 37 | 2.5 | 67 | 1.5 |
| 38 | 5.0 | 68 | >100 |
| 39 | >100 | 69 | >100 |
| 40 | 4.8 | 70 | >100 |
| 41 | 12.8 | 71 | 3.3 |
| 42 | 45.6 | 72 | 2.8 |
| 43 | 13.0 | 73 | 7.1 |
| 44 | 28.2 | 74 | 20.9 |
| 45 | 84.9 | 75 | 73.3 |
| 46 | 21.6 | 76 | 1.9 |
| 47 | 8.0 | 77 | 3.0 |
| 48 | 10.0 | 78 | >100 |
| 49 | 12.0 | 79 | 43.5 |
| 50 | 4.3 | 80 | >100 |
| 51 | 5.0 | 81 | 3.4 |
| 52 | 1.2 | 82 | 1.1 |
| 53 | 8.8 | 83 | 7.4 |
| 54 | 8.2 | 84 | 19.8 |
| 55 | 8.4 | 85 | 1.5 |
| 56 | >100 | 86 | 16.2 |
| 57 | >100 | 87 | 9.4 |
| 58 | >100 | 88 | 7.5 |
| 59 | >100 | 89 | 11.2 |
| 60 | 4.8 | 90 | 3.1 |
| 61 | >100 | 91 | 2.6 |
| 62 | >100 | 92 | 3.2 |
| 63 | >100 | 93 | 6.0 |
| 64 | >100 | 94 | 28.8 |
| 65 | 6.2 | 95 | 7.8 |
| 66 | 20.5 | 96 | 11.7 |

**[Table 27]**

| Example | EC50 (nmol/L) | Example | EC50 (nmol/L) |
|---|---|---|---|
| 97 | 85.0 | 110 | 2.8 |
| 98 | >100 | 111 | 6.9 |
| 99 | >100 | 112 | >100 |
| 100 | 27.9 | 113 | 3.1 |
| 101 | 33.6 | 114 | 6.2 |
| 102 | 61.8 | 115 | 1.9 |
| 103 | 31.4 | 116 | 1.7 |
| 104 | 81.4 | 117 | 6.9 |
| 105 | >100 | 118 | 55.2 |
| 106 | 2.0 | 119 | 3.5 |
| 107 | >100 | 120 | 22.7 |
| 108 | 2.2 | 121 | 11.7 |
| 109 | 9.6 | 122 | 3.6 |

As shown in Tables 25 to 27, the compounds of the present invention indicated agonist activity in the evaluation test of agonist activity to SSTR4. Especially, the compounds obtained in Examples 2, 7, 13, 15, 16,17, 18, 19, 20, 21, 26, 27, 30, 31, 37, 52, 67, 72, 76, 77, 81, 82, 85, 90, 91, 92, 108, 110, 115, and 116 indicated stronger SSTR4 agonist activity compared with other compounds. The expression of "> 100" seen in Tables 25 to 27 shows that the EC₅₀ value is higher than 100 nmol/L, and the detailed value is omitted. It was verified that tested compounds having EC₅₀ value > 100 nmol/L also show agonist activity.

### Test Example 2

### Evaluation of amelioration in cognitive impairment using mouse Y-shaped maze test (hereinafter, may be called "Y-maze test")

In the Y-maze test using Slc:ddY mice (male, Japan SLC, Inc.) with body weight of 25-35 g or 27-30 g, when 0.6 mg/kg of Scopolamine HBr (cat. No. S0929, Sigma Aldrich, USA) is subcutaneously administered to mice, memory impairment is caused, and reducing in spontaneous alternation is observed. Accordingly, mice were pre-treated by orally receiving compounds involved with the present invention, and ameliorating action in memory impairment was evaluated. As the result, it was verified that the compound obtained in Example 2 shows, at 1 or 3 mg/kg (oral), significant ameliorating action in memory impairment. (hereinafter, spontaneous alternations in each group in Example 2 are shown: 0.5% MC receiving group, 69.9 ± 3.5; Scopolamine receiving group, 43.9 ± 1.9; 1 mg/kg receiving group, 59.6 ± 3.2; 3 mg/kg receiving group, 65.0 ± 1.8.) It was also verified that the compound obtained in Example 21 shows, at 1 or 3 mg/kg (oral), significant ameliorating action in memory impairment. (hereinafter, spontaneous alternations in each group in Example 21 are shown: 0.5% MC receiving group, 71.2 ± 3.5; Scopolamine receiving group, 47.5 ± 2.6; 1 mg/kg receiving group, 60.7 ± 2.6; 3 mg/kg receiving group, 64.6 ± 2.2.)

### Test Example 3

### Evaluation of cognitive function using mouse novel object recognition test (hereinafter, may be called "mORT")

In the mORT using Slc:ddY mice (male, Japan SLC, Inc.) with body weight of 25-45 g or 35-40 g, memory deterioration to familiar subject depending on the interval time between the first trial (training) and the second trial (test) is observed. If the second trial is performed 24 hours after performing the first trial, significant forgetting is observed. Accordingly, compounds involved in the present invention was administered before the first trial, and memory enhancing action in the second trial was evaluated. As the result, it was verified that the compound obtained in Example 2 has, at 3 mg/kg (oral), a tendency to enhance memory. (hereinafter, discrimination index as indexes for recognition function in Example 2 are shown: 0.5% MC receiving group, 0.050 ± 0.048; 3 mg/kg receiving group, 0.216 ± 0.053.) In the case of the compound obtained in Example 21, significant memory enhancing action was also verified at 3 mg/kg (oral). (hereinafter, discrimination index as indexes for cognitive function in Example 21 are shown: 0.5% MC receiving group, 0.014 ± 0.034; 3 mg/kg receiving group, 0.214 ± 0.072.) In the case of the compound in Example 21, even if it was given after the first trial, significant memory enhancing action was verified at 3 mg/kg (oral). (discrimination index as indexes for cognitive function are shown: 0.5% MC receiving group, 0.054 ± 0.026; 3 mg/kg receiving group, 0.245 ± 0.066.)

### Test Example 4

### Binding affinity evaluation using cell membrane from human SSTR4-expressing cells

From Chem-1 cells forcibly expressing human SSTR4, cell membrane was prepared by using Hepes Buffer. A reaction solution consisting of the prepared cell membrane, 0.1 nmol/L [¹²⁵I] Tyrll-Somatostatin 14, and a test compound was prepared, and incubated for 2 hours at 25°C. The cell membrane after incubation was filtered, and washed. After dried, the filter was immersed in a scintillator, its radioactivity was measured with a scintillation counter. By defining the difference in radioactivity between the substance not containing a test compound and the substance containing 1 µmol/L Somatostatin 14 as the specific binding amount, inhibitory rates of test compounds were calculated. Based on IC₅₀ of the test compound calculated from the inhibition curve, the concentration of [¹²⁵I] Tyr11-Somatostatin 14, and Kd of Somatostatin 14, Ki of the test compound was calculated. As the result, Ki to SSTR4 of the compound obtained in Example 21 was 21 nmol/L. Moreover, by using cell membrane from the cells expressing SSTR subtypes, binding affinity to SSTR1, SSTR2, SSTR3, and SSTR5 was similarly evaluated, and Ki of the compound obtained in Example 21 to them were as described in Table 28.

**[Table 28]**

| | Ki(nmol/L) |
|---|---|
| SSTR1 | 1570 |
| SSTR2 | >5000 |
| SSTR3 | >3000 |
| SSTR4 | 21 |
| SSTR5 | >5000 |

### Test Example 5

### Examination of acetylcholine release enhancing action with microdialysis test using rats

A guide cannula was placed into the hippocampus of Crlj: WI rats, and microdialysis was performed by inserting a probe after the animals were recovered. After administering the compounds involved with the present invention, the hippocampus was stimulated by changing the perfusate into the high potassium solution for 15 minutes. Acetylcholine levels in the perfusate were analyzed with a HPLC-ECD apparatus. As the result, the compound obtained in Example 21, at 10 mg/kg (oral), significantly enhanced acetylcholine level 60 minutes after administration compared with the Vehicle.

### Test Example 6

### Neprilysin membrane translocation test using human neprilysin/human SSTR4 coexpressing cells

SH-SY5Y cells stably expresing human neprilysin/human SSTR4 were seeded to culture plates, and incubated in a 37°C/5% CO₂ incubator overnight. On the next day, the compounds involved with the present invention were added, and cells were incubated in the 37°C /5% CO₂ incubator another night. On the next day, 4% paraformaldehyde was added, and cells were fixed. The cells were washed, and blocked with 3% BSA solution. Then, immunostaining with an anti-neprilysin antibody and Alexa Fluor 488 donkey anti-mouse IgG was performed, and analyzed with an IN CELL Analyzer. As the result, the compound obtained in Example 21 concentration-dependently induced the translocation of neprilysin to cell membrane, and its EC₅₀ was 9.3 nM.

### Test Example 7

### Evaluation of cognitive function improving action and Aβ reducing action using Tg 2576 mice

Tg 2576 mouse shows reduced cognition function due to excessive Aβ production in the brain. Four-month-old Tg 2576 mice received compounds involved with the present invention mixed in feed for 4 months. After completion of administration, the mORT test was performed to evaluate cognitive function, then the amount of brain Aβ was measured by ELISA. As the result, the compound obtained in Example 21 showed, at 30 mg/kg/day, significant cognitive function improving action. Discrimination index as indexes for evaluating cognitive function were as follows (vehicle, 0.064 ± 0.049, 30 mg/kg/day; 0.330 ± 0.062). The compound also showed significant hippocampus Aβ42 reducing action in 100 mg/kg/day compared with the Vehicle (Vehicle, 283.99 ± 27.89 pmol/g, 100 mg/kg/day; 198.11 ± 26.32 pmol/g).

Moreover, it was verified, as the results of a pharmacokinetic test (PK test) using rodents, that the compound of the present invention has more excellent in vivo kinetics, central nervous system penetration, and exposure level, compared with the pyrrolidine derivative described in Patent Literature 1. In particular, the following Test Example 8 was performed.

### Test Example 8

### Pharmacokinetic test (PK test) using rodents

Compounds of the present invention were orally administered to mice at a dose of 10 mg/kg, and an hour and 4 hours after, the plasma and the brain were collected, concentrations of the administered compounds were measured by LC/MS/MS. As well, protein binding rates in the plasma and the brain were measured with equilibrium dialysis method, and concentrations of non-binding compounds were calculated. The brain-plasma concentration ratio was about 5 in any cases of an hour and 4 hours after for compounds obtained in Examples 7, 14, 15, 16, 17, 18, 20, 21, 27, 40, and 50, which indicated excellent central nervous system penetration. Furthermore, non-binding levels of the compound in the brain 4 hours after administration exceeded EC₅₀ values in agonist activity evaluation using human SSTR4-stably expressing cells by 10-fold or more.

Thus, the compound of the present invention has more excellent in vivo kinetics, central nervous system penetration, and exposure level, compared with the pyrrolidine derivative described in Patent Literature 1.

### Industrial Applicability

As described above, derivatives represented by formula (I) or their pharmaceutically acceptable salts have strong SSTR4 agonist activity, and are useful for treatment of diseases involved with the central nerve system and/or the peripheral nerve system, endocrine diseases, neurodegenerative diseases, disorders such as inflammations, or pains, eye diseases, and tumor diseases.

## Claims

1. A compound represented by the following formula (I) or its pharmaceutically acceptable salt: 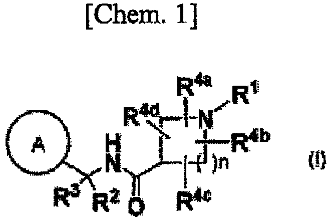
[wherein, R¹ represents C₁₋₄ alkyl which may be substituted by 1 to 5 fluorine atoms,
n is an integer of 1 to 4,
R² represents C₁₋₄ alkyl which may be substituted by the same or different 1 to 5 substituents selected from the group consisting of fluorine atom and hydroxy, or hydrogen atom,
R³ represents C₁₋₄ alkyl which may be substituted by the same or different 1 to 5 substituents selected from the group consisting of fluorine atom and hydroxy,
provided that n is 1, 3, or 4, R³ and R² may unitedly form a 3-6 membered saturated ring which may include any one of -O-, -NR⁵-, -SO-, or -SO₂-, and the saturated ring may be substituted by 1 to 5 substituents selected from the group consisting of C₁₋₄ alkyl which may be substituted by 1 to 5 fluorine atoms, C₁₋₄ alkoxy which may be substituted by 1 to 5 fluorine atoms, halogen, hydroxy, cyano, oxo, -CO₂R⁶, and -CONR⁷R⁸, provided that n is 2, R² and R³ do not unitedly form the saturated ring,
R^{4a}, R^{4b}, R^{4c}, and R^{4d}, similarly or differently, represent C₆₋₁₄ aryl which may be substituted by the same or different 1 to 5 substituents selected from the group consisting of C₁₋₄ alkyl (which may be substituted by 1 to 5 fluorine atoms), C₃₋₆ cycloalkyl, halogen, C₁₋₄ alkoxy (which may be substituted by 1 to 5 fluorine atoms), C₁₋₄ alkylthio, hydroxy, and cyano; C₁₋₄ alkyl which may be substituted by the same or different 1 to 5 substituents selected from the group consisting of fluorine atom, hydroxy, and C₁₋₃ alkoxy; C₁₋₃ alkoxy which may be substituted by 1 to 5 fluorine atoms; hydrogen atom; fluorine atom; hydroxy; or -CO₂R⁹, provided that n is 2, and R^{4a}, R^{4b}, R^{4c} and R^{4d} are hydrogen atoms, and provided that n is 1, 3, or 4, and two or more of R^{4a}, R^{4b}, R^{4c} and R^{4d} are optionally substituted C₁₋₄ alkyl, the two or more optionally substituted C₁₋₄ alkyl may unitedly form a 4-7 membered saturated ring,
A represents C₆₋₁₄ aryl or a 5-11 membered heteroaryl, the C₆₋₁₄ aryl and the 5-11 membered heteroaryl may be substituted by the same or different 1 to 5 substituents selected from the group consisting of C₁₋₄ alkyl, (which may be substituted by 1 to 5 fluorine atoms), C₃₋₆ cycloalkyl, halogen, C₆₋₁₄ aryl, C₆₋₁₄ aryloxy, C₁₋₄ alkoxy (which may be substituted by 1 to 5 fluorine atoms), C₁₋₄ alkylthio, hydroxy, cyano, nitro, -CO₂R¹⁰, -CONR¹¹R¹², -NR¹⁰OCOR¹¹, -NR¹⁰CONR¹¹R¹², -SOR¹³, -SO₂R¹⁴, -SO₂NR¹¹R¹², and -NR¹¹R¹², and provided that n is 3, R² is hydrogen atom, and R³ is tert-butyl, A is not unsubstituted phenyl,
R⁵ represents hydrogen atom, C₁₋₄ alkyl, C₃₋₆ cycloalkyl, -CONR¹⁵R¹⁶, -COR¹⁷, or -CO₂R¹⁸,
R⁶ and R⁹ similarly or differently represent hydrogen atom or C₁₋₄ alkyl,
R⁷ and R⁸ similarly or differently represent hydrogen atom, C₁₋₄ alkyl, and C₃₋₆ cycloalkyl, and provided that R⁷ and R⁸ are C₁₋₄ alkyl, R⁷ and R⁸ may unitedly form a 3-6 membered saturated heterocycle,
R¹⁰ represents hydrogen atom, C₁₋₄ alkyl, C₃₋₆ cycloalkyl, or C₆₋₁₄ aryl,
R¹¹ and R¹² similarly or differently represent hydrogen atom, C₁₋₄ alkyl, C₃₋₆ cycloalkyl, or C₆₋₁₄ aryl, and provided that R¹¹ and R¹² are C₁₋₄ alkyl, R¹¹ and R¹² may unitedly form a 3-6 membered saturated heterocycle,
R¹³ represents C₁₋₄ alkyl, C₃₋₆ cycloalkyl, or C₆₋₁₄ aryl,
R¹⁴ represents hydroxy, C₁₋₄ alkyl, C₃₋₆ cycloalkyl, C₁₋₄ alkoxy, C₆₋₁₄ aryloxy, or C₆₋₁₄ aryl,
R¹⁵ and R¹⁶ similarly or differently represent hydrogen atom or C₁₋₄ alkyl, and provided that R¹⁵ and R¹⁶ are C₁₋₄ alkyl, R¹⁵ and R¹⁶ may unitedly form a 3-6 membered saturated heterocycle,
R¹⁷ represents hydrogen atom, C₁₋₄ alkyl, or C₃₋₆ cycloalkyl,
R¹⁸ represents C₁₋₄ alkyl or C₃₋₆ cycloalkyl].

2. The compound or its pharmaceutically acceptable salt according to claim 1, wherein n is 1 or 3.

3. The compound or its pharmaceutically acceptable salt according to claim 1, wherein n is 3.

4. The compound or its pharmaceutically acceptable salt according to any one of claims 1 to 3, wherein R² and R³ are the same or different C₁₋₄ alkyl which may be substituted by the same or different 1-5 substituents selected from the group consisting of fluorine atoms and hydroxy, and R² and R³ may unitedly form a 3-6 membered saturated ring which may include any one of -O- or -SO₂-.

5. The compound or its pharmaceutically acceptable salt according to any one of claims 1 to 4, wherein A is C₆₋₁₄ aryl, and the C₆₋₁₄ aryl may be substituted by the same or different 1 to 5 substituents selected from the group consisting of C₁₋₄ alkyl (which may be substituted by 1 to 5 fluorine atoms), C₃₋₆ cycloalkyl, fluorine atom, chlorine atom, bromine atom, C₁₋₄ alkoxy (which may be substituted by 1 to 5 fluorine atoms), C₁₋₄ alkylthio, cyano, -CO₂R¹⁰, -CONR¹¹R¹², -SO₂NR¹¹R¹², and -NR¹¹R¹².

6. The compound or its pharmaceutically acceptable salt according to any one of claims 1 to 5, wherein R¹ is C₁₋₃ alkyl.

7. The compound or its pharmaceutically acceptable salt according to any one of claims 1 to 6, wherein R^{4a} and R^{4b}, similarly or differently, are C₆₋₁₄ aryl which may be substituted by the same or different 1 to 5 substituents selected from the group consisting of C₁₋₄ alkyl (which may be substituted by 1 to 5 fluorine atoms), C₃₋₆ cycloalkyl, halogen atom, C₁₋₄ alkoxy (which may be substituted by 1 to 5 fluorine atoms), C₁₋₄ alkylthio, hydroxy, and cyano; C₁₋₄ alkyl which may be substituted by the same or different 1 to 5 substituents selected from the group consisting of fluorine atom, hydroxy, and C₁₋₃ alkoxy; C₁₋₃ alkoxy which may be substituted by 1 to 5 fluorine atoms; hydrogen atom; fluorine atom; or -CO₂R⁹, provided that R^{4a} and R^{4b} are optionally substituted C₁₋₄ alkyl, the optionally substituted C₁₋₄ alkyl may unitedly form a 4-7 membered saturated ring, and
R^{4c} and R^{4d} are hydrogen atoms.

8. The compound or its pharmaceutically acceptable salt according to any one of claims 1 to 7, wherein R¹ is C₁₋₃ alkyl, and
R² and R³ are, similarly or differently, C₁₋₄ alkyl.

9. The compound or its pharmaceutically acceptable salt according to any one of claims 1 to 8, wherein A is C₆₋₁₄ aryl, and the C₆₋₁₄ aryl may be substituted by the same or different 1 to 3 substituents selected from the group consisting of C₁₋₄ alkyl (which may be substituted by 1 to 5 fluorine atoms), C₃₋₆ cycloalkyl, fluorine atom, chlorine atom, bromine atom, C₁₋₄ alkoxy (which may be substituted by 1 to 5 fluorine atoms), C₁₋₄ alkylthio, and cyano.

10. The compound or its pharmaceutically acceptable salt according to any one of claims 3 to 9, wherein, when n is 3,
R^{4a} and R^{4b} are, similarly or differently, substituents at the 2, 5, or 6-positions of the piperidine ring, and are C₁₋₄ alkyl which may be substituted by the same or different 1 to 5 substituents selected from the group consisting of hydroxy and C₁₋₃ alkoxy or hydrogen atom, provided that R^{4a} and R^{4b} are optionally substituted C₁₋₄ alkyl, the C₁₋₄ alkyl may unitedly form a 4-7 membered saturated ring, and
R^{4c} and R^{4d} are hydrogen atoms,
provided that R¹ is at the 1-position and the amido is at the 3-position of the piperidine ring.

11. The compound or its pharmaceutically acceptable salt according to any one of claims 3 to 9, wherein, when n is 3,
R^{4a} is the substituent at the 4-position of the piperidine ring and is C₆₋₁₄ aryl which may be substituted by the same or different 1 to 5 substituents selected from the group consisting of C₁₋₄ alkyl which may be substituted by 1 to 5 fluorine atoms, C₃₋₆ cycloalkyl, halogen, C₁₋₄ alkoxy which may be substituted by 1 to 5 fluorine atoms, C₁₋₄ alkylthio, hydroxy, and cyano; or hydrogen atom, and
R^{4b}, R^{4c} and R^{4d} are hydrogen atoms,
provided that R¹ is at the 1-position and the amido is the 3-position of the piperidine ring.

12. The compound or its pharmaceutically acceptable salt according to any one of claims 3 to 9, wherein, when n is 3, R^{4a} is the substituent at the 2, or 6-position of the piperidine ring and is C₁₋₄ alkyl which may be substituted by the same or different 1 to 5 substituents selected from the group consisting of fluorine atom, hydroxy, and C₁₋₃ alkoxy; or hydrogen atom, and R^{4b}, R^{4c}, and R^{4d} are hydrogen atoms,
provided that R¹ is at the 1-position and the amido is the 3-position of the piperidine ring.

13. The compound or its pharmaceutically acceptable salt according to any one of claims 3 to 9, wherein, when n is 3,
R^{4a} is the substituent at the 5-position of the piperidine ring and is C₁₋₄ alkyl which may be substituted by the same or different 1 to 5 substituents selected from the group consisting of fluorine atom, hydroxy, and C₁₋₃ alkoxy; C₁₋₃ alkoxy which may be substituted by 1 to 5 fluorine atoms; fluorine atom; or hydrogen atom, and
R^{4b}, R^{4c}, and R^{4d} are hydrogen atoms,
provided that R¹ is at the 1-position and the amido group is the 3-position of the piperidine ring.

14. The compound or its pharmaceutically acceptable salt according to any one of claims 1 to 13, wherein R^{4a}, R^{4b}, R^{4c}, and R^{4d} are hydrogen atoms.

15. The compound or its pharmaceutically acceptable salt according to claim 1, wherein the compound is selected from the group consisting of:
(3S)-1-methyl-N-[2-(naphthalene-1-yl)propane-2-yl]pyperidine-3-carboxamide;
N-[1-(2,3-dimethylphenyl)cyclopropyl]-1-methylpiperidine-3-ca rboxamide;
N-[4-(3,5-dichlorophenyl)tetrahydro-2H-pyran-4-yl]-1-methylpi peridine-3-carboxamide;
(3S)-N-[2-(2,3-dichlorophenyl)propane-2-yl]-1-methylpiperidin e-3-carboxamide;
(3S)-N-[2-(2,4-dichlorophenyl)propane-2-yl]-1-methylpiperidin e-3-carboxamide;
(3S)-N-[2-(2,5-dichlorophenyl)propane-2-yl]-1-methylpiperidin e-3-carboxamide;
(3S)-N-[2-(2,3-dimethylphenyl)propane-2-yl]-1-methylpiperidin e-3-carboxamide;
(3S)-N-[2-(2-chloro-3-methylphenyl)propane-2-yl]-1-methylpip eridine-3-carboxamide;
(3S)-N-[2-(3-chloro-2-methylphenyl)propane-2-yl]-1-methylpip eridine-3-carboxamide;
(3S)-N-[2-(3-chloro-5-methylphenyl)propane-2-yl]-1-methylpip eridine-3-carboxamide;
(3S)-N-[2-(4-chloro-2-methoxylphenyl)propane-2-yl]-1-methylp iperidine-3-carboxamide;
(3S)-N-[2-(5-chloro-2-methoxylphenyl)propane-2-yl]-1-methylp iperidine-3-carboxamide;
(3S)-N-[2-(4-chloro-2-ethylphenyl)propane-2-yl]-1-methylpiper idine-3-carboxamide;
(3S)-N-[2-(2,4-diethylphenyl)propane-2-yl]-1-methylpiperidine-3-carboxamide;
(3S)-N-[2-(2,4-dimethylphenyl)propane-2-yl]-1-methylpiperidin e-3-carboxamide;
(3S)-N-[2-(2-chloro-4-methylphenyl)propane-2-yl]-1-methylpip eridine-3-carboxamide;
(3S)-N-[2-(2-chloro-5-methylphenyl)propane-2-yl]-1-methylpip eridine-3-carboxamide;
(3S)-N-[2-(2-chlorophenyl)propane-2-yl]-1-methylpiperidine-3-carboxamide;
(3S)-N[2-(2-methylphenyl)propane-2-yl]-1-methylpiperidine-3-carboxamide;
(3S)-N-[2-(3-chloro-4-methylphenyl)propane-2-yl]-1-methylpip eridine-3-carboxamide;
N-[2-(4-chloro-2-methoxylphenyl)propane-2-yl]-1-methylpiperi dine-3-carboxamide;
(3S)-N-[2-(5-fluoro-2-methoxylphenyl)propane-2-yl]-1-methylp iperidine-3-carboxamide;
(3S)-N-[2-(2,6-dichlorophenyl)propane-2-yl]-1-methylpiperidin e-3-carboxamide;
(3S)-N-{2-[2-(trifluoromethoxy)phenyl]propane-2-yl}-1-methyl piperidine-3-carboxamide;
(3S)-N-{2-[2-(methylsulfanyl)phenyl]propane-2-yl}-1-methylpi peridine-3-carboxamide;
(3S)-N-[2-(2-bromophenyl)propane-2-yl]-1-methylpiperidine-3-carboxamide;
(3S)-N-[2-(2-chloro-3-fluorophenyl)propane-2-yl]-1-methylpipe ridine-3-carboxamide;
(3S)-N-[2-(2-chloro-4-fluorohenyl)propane-2-yl]-1-methylpiperi dine-3-carboxamide;
(3S)-N-[2-(2-chloro-5-fluorohenyl)propane-2-yl]-1-methylpiperi dine-3-carboxamide;
(3S)-N-[2-(2-ethylphenyl)propane-2-yl]-1-methylpiperidine-3-c arboxamide;
(3S)-N-[2-(2-chlorophenyl)propane-2-yl]-1-ethylpiperidine-3-ca rboxamide; or
(3S)-N-[2-(naphthalene-1-yl)propane-2-yl]-1-(propane-2-yl)pipe ridine-3-carboxamide; and
(3S)-N-[2-(5-chloro-2-methylphenyl)propane-2-yl]-1-methylpip eridine-3-carboxamide: or its pharmaceutically acceptable salts.

16. A pharmaceutical composition, comprising the compound or its pharmaceutically acceptable salt according to any one of claims 1 to 15.

17. An agent for activating somatostatin receptor subtype 4, comprising the compound or its pharmaceutically acceptable salt according to any one of claims 1 to 15.

18. An agent for treating or preventing a disease that can be treated or prevented by activating somatostatin receptor subtype 4, comprising the compound or its pharmaceutically acceptable salt according to any one of claims 1 to 15, as an active ingredient.

19. The agent according to claim 18, wherein the disease is epilepsy, depression, behavior disorder, memory impairment, learning disabilities, attention deficit disorder, pain, neurodegenerative diseases, or neurogenic bladder.

20. The agent according to claim 18, wherein the disease is hyperproliferative disorder, acromegaly, melanoma, breast cancer, prostatic adenoma, prostate cancer, lung cancer, intestinal and colorectal cancer, or skin cancer.

21. The agent according to claim 18, wherein the disease is arthritis, rheumatoid arthritis, or rheumatoid spondylitis.

22. The agent according to claim 18, wherein the disease is psoriasis, atopic dermatitis, or asthma.

23. The agent according to claim 18, wherein the disease is Graves' disease, inflammatory bowel disease, nephropathy, diabetic angiopathy, ischemic disease, or benign prostatic hypertrophy.

24. The agent according to claim 18, wherein the disease is age-related macular degeneration, glaucoma, or diabetic retinopathy.

25. Use of the compound or its pharmaceutically acceptable salt according to any one of claims 1 to 15 for treating or preventing a disease that can be treated or prevented by activating somatostatin receptor subtype 4.
